# EUROPEAN PATENT SPECIFICATION

(11) **EP 2 425 837 B1**
(45) Date of publication and mention of the grant of the patent: **04.03.2015**
(21) Application number: 10769307.9
(22) Date of filing: 27.04.2010
(51) Int. Cl.: A61K 31/52, A61K 31/4439, A61K 31/5377, A61K 31/366, A61P 31/16, A61P 11/00

(54) **USE OF CELL AUTOPHAGY (TYPE II CELL APOPTOSIS) INHIBITORS**
VERWENDUNG VON ZELLAUTOPHAGEN (TYP-II-ZELLAPOPTOSE)-HEMMERN
UTILISATION D'INHIBITEURS D'AUTOPHAGIE CELLULAIRE (APOPTOSE CELLULAIRE DE TYPE II)

(30) Priority: 27.04.2009 CN 200910082966
(43) Date of publication of application: 07.03.2012
(73) Proprietor: Institute of Basic Medical Sciences Chinese Academy of Medical Sciences, Beijing 100005 (CN)
(72) Inventor: JIANG, Chengyu, Beijing 100005 (CN); RAO, Shuan, Beijing 100005 (CN); LIU, Haolin, Beijing 100005 (CN); GUO, Feng, Beijing 100005 (CN); WANG, Hongliang, Beijing 100005 (CN); SUN, Yang, Beijing 100005 (CN); LI, Chenggang, Beijing 100005 (CN)
(74) Representative: Pistolesi, Roberto
(86) International application number: PCT/CN2010/072231
(87) International publication number: WO 2010/124618

(56) References cited:
- WO-A2-2009/023059
- US-A1- 2006 276 438
- DATABASE WPI Week 200849 Thomson Scientific, London, GB; AN 2008-H74031 XP002682031, & WO 2007/139150 A1 (ONO PHARM CO LTD) 6 December 2007 (2007-12-06)
- LEVINE B ET AL: "Autophagy in cell death: an innocent convict?", THE JOURNAL OF CLINICAL INVESTIGATION,, vol. 115, no. 10, 1 October 2005 (2005-10-01), pages 2679-2688, XP008116133, DOI: 10.1172/JCI26390
- WANG HONGLIANG.: 'CHINESE DOCTORAL DISSERTATIONS FULL-TEXT DATABASE' AGRICULTURE SCIENCE AND TECHNOLOGY. 15 October 2009,
- RAO SHUAN.: 'CHINESE DOCTORAL DISSERTATIONS FULL-TEXT DATABASE' MEDICINE AND HEALTH SCIENCES. 15 December 2009,

## Description

### Technical field of the invention

The present invention is related to use of inhibitors of autophagy. In particularly, the present invention is related to the use of inhibitor of autophagy, such as 3-methyladenine, SB203580, LY294002 or wortmannin and the like, for preparing medicaments for preventing and/or treating the avian influenza of mammals. The present invention is also related to the use of inhibitor of autophagy for preparing medicaments for preventing and/or treating lung injury of mammals induced by nanometer materials.

### Background of the Invention

Influenza virus is pathogenic virus which induces influenza, belongs to RNA virus, can cause acute respiratory infectious disease and results in many complications such as myocarditis, pneumonia, bronchitis, and so on. Because of the high contagiousness, the influenza virus is extremely easily prevailing, even in the world-wide range. Influenza which has broken out in Europe during 1917-1919 is the most serious influenza outbreak in history, resulting in the death of 20 million people. Recently, H5N1 avian influenza virus is widely spreading in the worldwide range via domestic fowls and migrant birds. According to reports, the mortality of patients infected by H5N1 virus is approximately 50%, which means that H5N1 avian flu may prevail in the whole worldwide range (Peter S. Tang, Marco Mura, Rashmi Seth et al. Acute lung injury and cell death: how many ways can cells die? Am J Physiol Lung Cell Mol Physiol 294: L632-L641 (2008)).

Acute respiratory disorders induced by influenza virus are the primary cause of death, the main characterization of which is acute lung injury. The "storm" of cytokines induced by inflammatory factors constitutes the main pathopoiesis mechanism of influenza virus. Recently, cell death induced by pathogene is considered to be another important mechanism underlying the lung injury (Peter S. Tang, Marco Mura, Rashmi Seth et al. Acute lung injury and cell death: how many ways can cells die? Am J Physiol Lung Cell Mol Physiol 294: L632-L641 (2008)).

Avian flu is abbreviation of avian influenza, which is a contagious disease caused by a subtype (also called as avian influenza virus) of influenza A virus. According to various types of pathogene, avian influenza can be divided into three types: highly pathogenic, low pathogenic and non pathogenic avian influenza. So far, all of the outbreaks of the highly pathogenic avian influenza are caused by H5 and H7 subtype virus. Avian influenza can infect many kinds of animals, including bird, pig, horse, seal, whale and human, and so on. In the prior art, the pathopoiesis mechanism of avian influenza is not clear, and there is not effective medicaments for the prevention and treatment. Therefore, it is important to study and to explore efficient medicaments to prevent and treat avian influenza.

The form of cell death is mainly divided into two types: Programmed Cell Death (PCD) and Necrosis. Programmed Cell Death is a cell suicide mechanism developed during the long period of evolution of organism, and plays an important role in the aspects of eliminating worn-out, redundant or cancerous cells and maintaining the homeostasis of the internal environment of organism. In recent years, a new form of programmed cell death, i.e. autophagic programmed cell death, has drawn more and more cell biologists' attention. Autophagy is named as type II programmed cell death, the cell death of which is mainly characterized in the appearance of abundant vacuole enveloping cytoplasm and organelles, and the degradation of components inside the vacuole via lysosome (Beth Levine and Junying Yuan, Autophagy in cell death: an innocent convict? J. Clin. Invest. 115:2679-2688 (2005).).

The mechanism underlying autophagy involves many signal transmitting systems, in which mTOR (Mammalian Target of Rapamycin) signal transduction pathway has been generally demonstrated. TOR kinase is a sensor of amino acids, ATP and hormone, and plays an important role in regulating the growth of cells. TOR kinase inhibits the occurrence of autophagy, functions as a negative regulator, and plays a role of "gatekeeper". Ribosomal protein S6 (p70S6) positioned in the mammal cells inhibits the occurrence of autophagy. It is located downstream of TOR signal pathway, and the activity of which is regulated by mTOR (Klionsky DJ, Meijer AJ, Codogno P et al. Autophagy and P70S6 kinase. Autophagy 1(1): 059-061. (2005)). In the prior art, Rapamycin exerts an effect on inhibiting the activity of p70S6 and inducing the occurrence of autophagy through inhibiting the activity of mTOR.

As a new material, nanometer materials are widely used for scientific research, cosmetology, clothing and manufacture, and so on. The research on nanometer is becoming hot topic gradually, and it has been reported that some nanometer materials can induce cell autophagy (Zabirnyk O, Yezhelyev M, Seleverstov O. Nanoparticles as a novel class of autophagy activators. Autophagy. 2007 May-Jun; 3(3):278-81.). Some nanometer materials may induce lung injury (Byrne JD, Baµgh JA. The significance of nanoparticles in particle-induced pulmonary fibrosis. Mcgill J Med. 2008 Jan; 11(1):43-50.). However, the specific mechanism underlying the interaction between nanometer materials and cells is not clear, and the mechanism underlying the lung injury induced by nanometer materials via cell autophagy has not been reported. Therefore, there is the need to provide efficient medicaments for preventing and/or treating lung injury induced by nanometer materials.

### Summary of the Invention

The present invention provides, in one aspect, the use of inhibitor of autophagy in preparing medicaments for preventing and/or treating the influenza of mammals, preferably, the avian influenza of mammals. Preferably, the said inhibitor is 3-methyladenine. Preferably, the present invention provides the use of 3-methyladenine in preparation of drugs for preventing influenza, preferably, the avian influenza.

In one embodiment of the invention, the mammal is human.

In another aspect, the present invention provides the use of inhibitor of autophagy for preventing and/or treating lung injury of mammals induced by influenza virus, preferably, the avian influenza virus.

In one embodiment of the invention, the lung injury is acute respiratory distress syndrome.

In one embodiment of the invention, the avian influenza is caused by H5N1, H5N2 and H9N2 types of avian influenza virus.

In one embodiment of the invention, the 3-methyladenine is an inhibitor of autophagy, and it is an inhibitor of signal transduction pathway of PI3K Class III.

In another aspect, the invention provides the use of inhibitor of autophagy in preparation of medicaments for preventing and/or treating lung injury of mammals induced by nanometer materials. And the said inhibitor is 3-methyladenine.

In one embodiment of the invention, the mammal is human.

The nanometer materials of the invention include PAMAM G3, G4, G5, G5.5, G6, G7 and G8, and so on.

In one embodiment of the invention, the said 3-methyladenine is an inhibitor of autophagy, and it is an inhibitor of signal transduction pathway of PI3K Class III.

In one embodiment of the invention, the lung injury is acute respiratory distress syndrome.

In this invention, the said inhibitor of autophagy may be the inhibitor of signal transduction pathway of cells, and the said signal transduction pathway of cells includes the signal transduction pathways of TSC1/2, LC3, Atg5-Atg12, P38, TSC1/2 and PI3K. Preferably, the said signal transduction pathway is signal transduction pathway of Atg5-Atg12-LC3 cell.

In this invention, the inhibitor of cell autophagy (type II cell apoptosis) can be used as the agonist of signal transduction pathway of cells, wherein the said signal transduction pathway of cells includes signal transduction pathways of AKT and mTOR. Preferably, the signal transduction pathway is signal transduction pathway of mTOR-TSC1/2-AKT.

The inventor discovered that the inhibitor of autophagy has significant effect on preventing and/or treating avian influenza, particularly, the prevention effect thereof is surprising.

The avian influenza includes, but not limited to, avian influenza induced by H5N1, H9N2, H5N2 avian influenza virus or the inactivated virus thereof or surface protein thereof.

The inventor also discovered that the inhibitor of autophagy has significant effect on preventing and/or treating lung injury induced or aggravated by nanometer materials, particularly, the prevention effect thereof is surprising.

The said inhibitor of cell autophagy (type II cell apoptosis) includes, but not limited to, 3-methyladenine, SB203580, LY294002 or wortmannin.

This invention demonstrated that the prevention and/or treatment effect of the inhibitor of autophagy is significant, which can obviously prevent the occurrence of the autophagy so as to greatly reduce the death of cells, especially the death of lung epithelial cells of mammals; Experimental results proved that there is a significant difference between the survival rate of cells treated with the inhibitor of autophagy firstly and then treated with inactivated H5N1 virus and the survival rate of cells treated only with inactivated H5N1 virus. The prevention and treatment effect of the inhibitor of autophagy can be further proven via the *in vivo* experiments such as pathological photographs, inflammatory cell counting, lung wet/dry ratio, alteration of lung elasticity and mortality of mice, and so on. It has also been proven that the inhibitor of autophagy obviously decreases the injury of lung tissue so as to prevent and treat avian influenza. Furthermore, it has been also proven that the inhibitor of cell autophagy prevents and treats the lung injury induced or aggravated by nanometer materials through the same *in vivo* and *in vitro* experiments.

### Brief Description of the Drawings

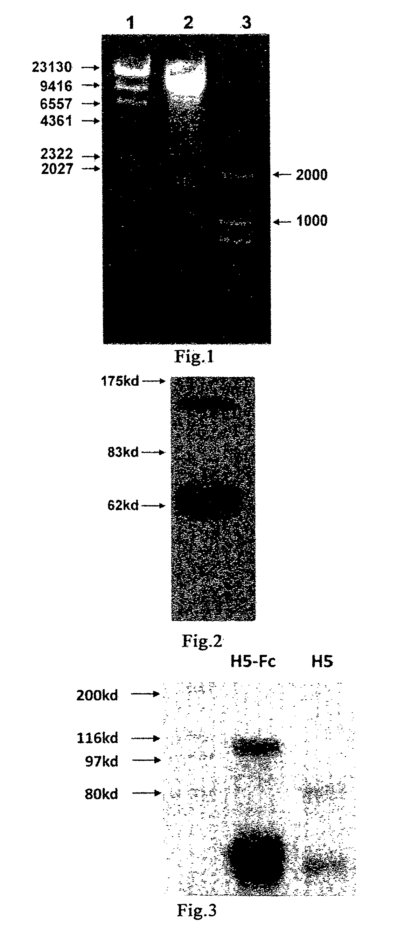

Figure 1 displays the results that the expression vector Peak13 CD5L TEV human IgG inserted with H5 gene was digested with restriction enzyme digestion and separated by agarose gel electrophoresis. Lanes 1, 2 and 3 indicates λ-HindIII Marker, Peak 13 CD5L H5 TEV human IgG, D2000 Marker, respectively. The size of Marker is 564bp (difficult to be distinguished from the figure), 2027bp, 2322bp, 4361 bp, 6557bp, 9416bp and 23130bp, in turn, from small to big (from bottom to top); The size of D2000 Marker is 100bp, 250bp, 500bp, 750bp, 1000bp and 2000bp, in turn, from small to big (from bottom to top). Plasmid was restricted by Nhe I/BamH I enzyme to obtain a 1.56Kb fragment, demonstrating that H5 gene has been inserted into the expression vector.
Figure 2 displays the expression result of fusion protein H5Fc expressed in 293ET cell detected by Western Blotting. It demonstrates that fusion protein H5Fc was well expressed in host cell, and the molecular weight of the expressed protein was approximately 110KD and 60KD. H5 protein was cleaved by the enzyme inside the host so as to form two bands.
Figure 3 displays the result of polyacrylamide gel electrophoresis of the purified H5Fc fusion protein and H5 protein, colored by coomassie brilliant blue (CBB). It demonstrated that the well purified fusion protein H5Fc and H5 protein can be obtained. The purified H5Fc protein was restricted by TEV enzyme, and then preceded by affinity chromatography so as to obtain purified H5 protein with the molecular weight of approximately 80KD.

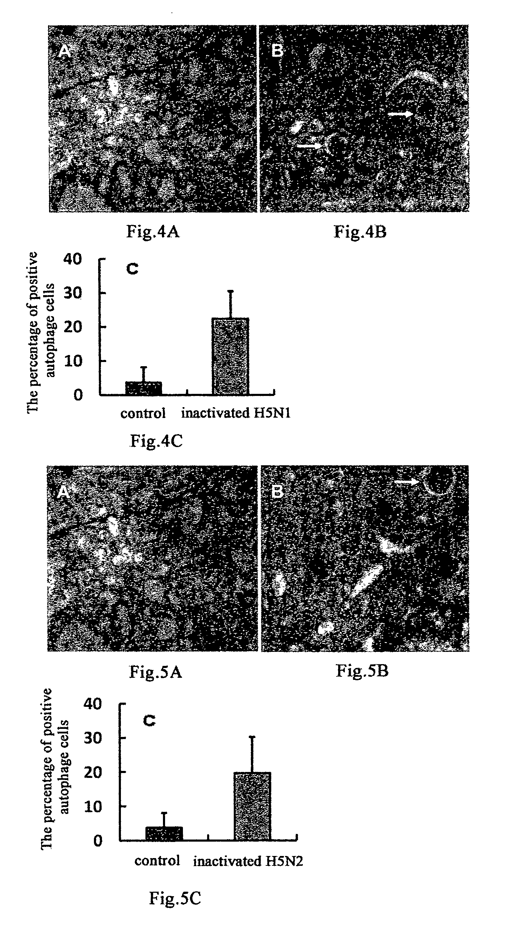

Figure 4 displays the electron micrograph (×20000) of Hela cells treated in different ways and the percentage of autophagy cells (type II cell apoptosis) observed under electron microscope in bar graph. Fig. A indicates the electron micrograph of Hela cells treated with adjuvant for 4 hours. Fig. B indicates the electron micrograph of Hela cells treated with H5N1 avian influenza inactivated virus for 4 hours, the arrow indicates autophagosome. Fig. C indicates the percentage of autophagy cells (type II PCD) of Hela cells under the above two conditions in bar graph. It demonstrated that autophagy of Hela cells occurs under the effect of the inactivated H5N1 avian influenza virus.
Figure 5 displays the electron micrograph (×20000) of Hela cells treated in different ways and the percentage of autophagy cells (type II PCD) observed under electron microscope in bar graph. Fig. A indicates the electron micrograph of Hela cells treated with adjuvant for 4 hours. Fig. B indicates the electron micrograph of Hela cells treated with inactivated H5N2 avian influenza virus for 4 hours, the arrow indicates autophagosome. Fig. C indicates the percentage of autophagy cells (type II PCD) of Hela cells under the above two conditions in bar graph. It demonstrates that autophagy of Hela cells occurs under the effect of the inactivated H5N2 avian influenza virus.

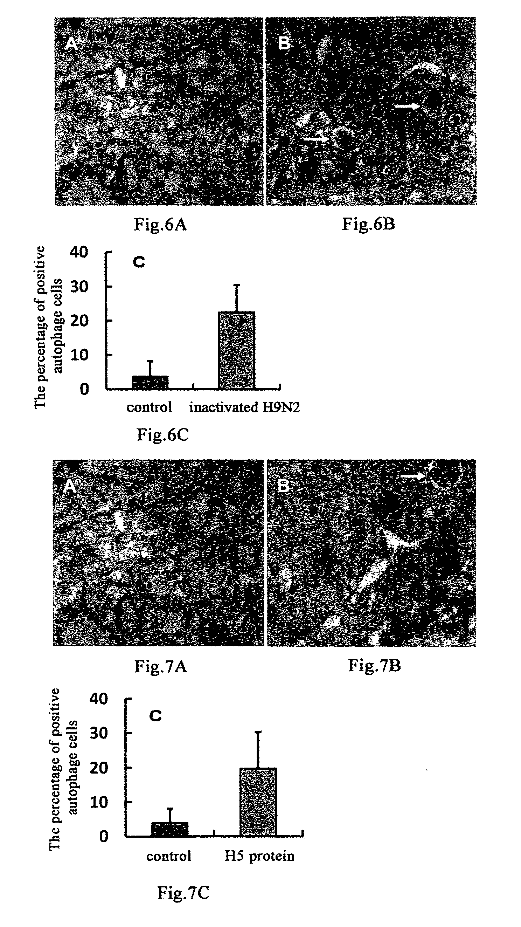

Figure 6 displays the electron micrograph (×20000) of Hela cells treated in different ways and the percentage of autophagy cells (type II PCD) observed under electron microscope in bar graph. Fig. A indicates the electron micrograph of Hela cells treated with adjuvant for 4 hours. Fig. B indicates the electron micrograph of Hela cells treated with inactivated H9N2 avian influenza virus for 4 hours, the arrow indicates autophagosome. Fig. C indicates the percentage of autophagy cells (type II PCD) of Hela cells under the above two conditions in bar graph. It demonstrates that autophagy of Hela cells occurs under the effect of the inactivated H9N2 avian influenza virus.
Figure 7 displays the electron micrograph (×20000) of Hela cells treated in different ways and the percentage of autophagy cells (type II PCD) observed under electron microscope in bar graph. Fig. A indicates the electron micrograph of Hela cells treated with BSA protein for 4 hours. Fig. B indicates the electron micrograph of Hela cells treated with H5 protein for 4 hours, the arrow indicates autophagosome. Fig. C indicates the percentage of autophagy cells (type II PCD) of Hela cells under the above two conditions in bar graph. It demonstrates that autophagy of Hela cells occurs under the effect of the H5 protein. Meanwhile it also demonstrated that the expressed H5 protein has biological activity.

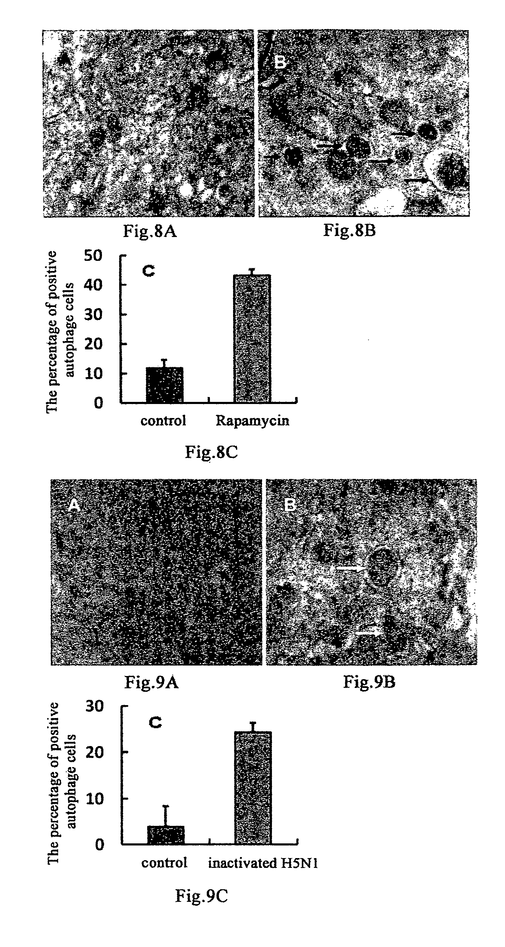

Figure 8 displays the electron micrograph (×20000) of Hela cells treated in different ways and the percentage of autophagy cells (type II PCD) observed under electron microscope in bar graph. Fig. A indicates the electron micrograph of Hela cells treated with DMSO for 4 hours. Fig. B indicates the electron micrograph of Hela cells treated with 5µM Rapamycin for 4 hours, the arrow indicates autophagosome. Fig. C indicates the percentage of autophagy cells (type II PCD) of Hela cells under the above two conditions in bar graph. It can be known from literatures that Rapamycin results in the occurrence of cell autophagy. Thus, the method of the experiment is proven to be accurate and effective, considering Rapamycin as a positive control of the experiment.
Figure 9 displays the electron micrograph (×20000) of A549 cells treated in different ways and the percentage of autophagy cells (type II PCD) observed under electron microscope in bar graph. Fig. A indicates the electron micrograph of A549 cells treated with chicken chorioallantoic fluid for 4 hours. Fig. B indicates the electron micrograph of A549 cells treated with inactivated H5N1 avian influenza virus for 4 hours, the arrow indicates autophagosome. Fig. C indicates the percentage of cell autophagy (type II PCD) of A549 cells under the above two conditions in the bar graph. It demonstrated that autophagy of A549 cells occurred under the effect of inactivated H5N1 avian influenza virus.

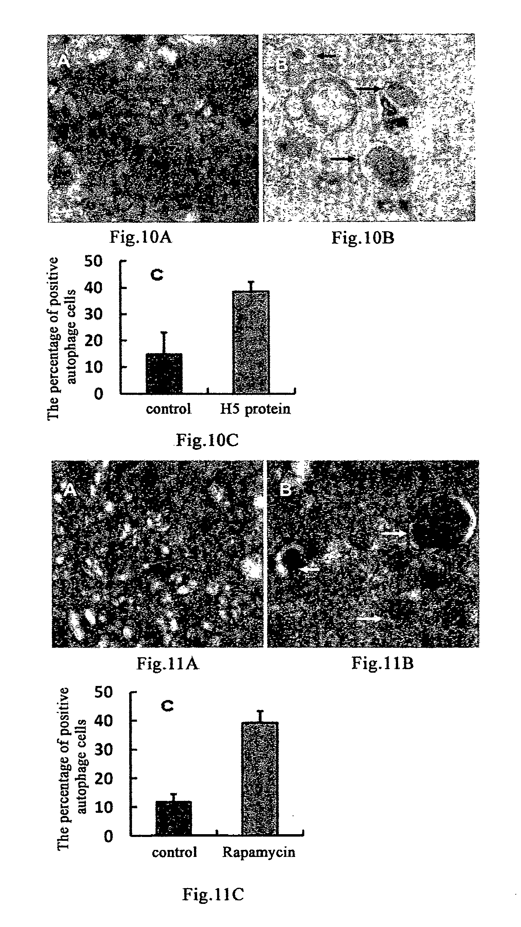

Figure 10 displays the electron micrograph (x20000) of A549 cells treated in different ways and the percentage of autophagy cells (type II PCD) observed under electron microscope in bar graph. Fig. A indicates the electron micrograph of A549 cells treated with BSA for 4 hours. Fig. B indicates the electron micrograph of A549 cells treated with H5 protein for 4 hours, the arrow indicates autophagosome. Fig. C indicates the percentage of cell autophagy (type II PCD) of A549 cells under the above two conditions in the bar graph. It demonstrates that autophagy of A549 cells occurred under the effect of H5 protein, and it also demonstrated that the expressed H5 protein has biological activity.
Figure 11 displays the electron micrograph (×20000) of A549 cells treated in different ways and the percentage of autophagy cells (type II PCD) observed under electron microscope in bar graph. Fig. A indicates the electron micrograph of A549 cells treated with DMSO for 4 hours. Fig. B indicates the electron micrograph of A549 cells treated with 5uM Rapamycin for 4 hours, the arrow indicates autophagosome. Fig. C indicates the percentage of cell autophagy (type II PCD) of A549 cells under the above two conditions in the bar graph. It can be known from literatures that Rapamycin results in the occurrence of cell autophagy. Thus, the method of the experiment is proven to be accurate and effective, considering Rapamycin as a positive control of the experiment.

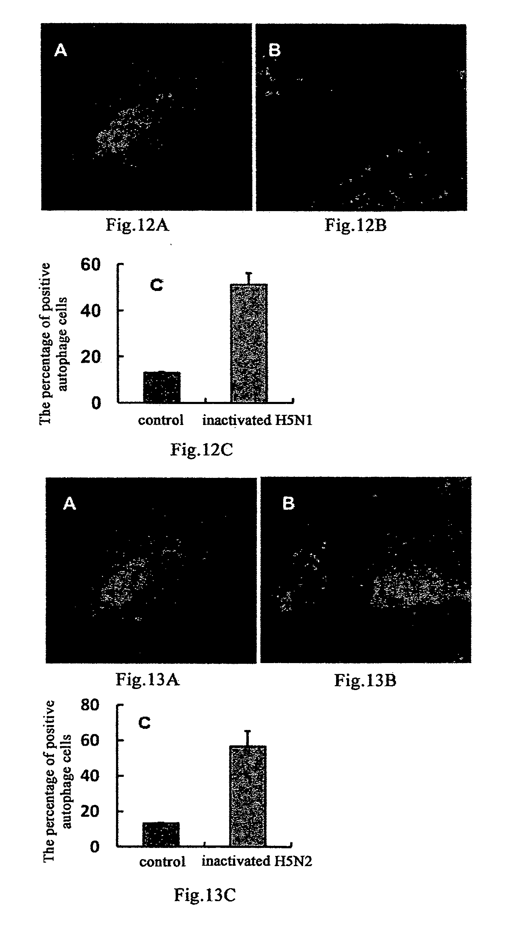

Figure 12 displays photographs of Hela cells transfected with EGFP-LC3 plasmid and treated in different ways under laser scanning confocal microscope (×1000) and the percentage of cells with EGFP-LC3 agglutination in the bar graph. Fig. A indicates the photograph of Hela cells treated with adjuvant for 4 hours under the laser scanning confocal microscope. Fig. B indicates the photograph of Hela cells treated with inactivated H5N1 avian influenza virus for 4 hours under laser scanning confocal microscope. Fig. C indicates the percentage of Hela cells with EGFP-LC3 agglutination under the above two conditions in the bar graph. Typical characterization after the occurrence of cell autophagy is the agglutination of LC3 (ATG8) molecular so as to form autophagysome. If the autophagy happens in cells, LC3 molecular labeled with EGFP will aggregate, and strongly emitted green fluorescence can be observed under confocal microscope; While for the cells without autophagy, the green fluorescence dispersed or only a little agglutinated. It demonstrates that autophagy of Hela cells occurred under the effect of inactivated H5N1 avian influenza virus.
Figure 13 displays photographs of Hela cells transfected with EGFP-LC3 plasmid and treated in different ways under laser scanning confocal microscope (×1000) and the percentage of cells with EGFP-LC3 agglutination in bar graph. Fig. A indicates photograph of Hela cells treated with adjuvant for 4 hours under the laser scanning confocal microscope. Fig. B indicates the photographs of Hela cells treated with inactivated H5N2 avian influenza virus for 4 hours under laser scanning confocal microscope, and Fig. C indicates the percentage of Hela cells with EGFP-LC3 agglutination under the above two conditions in the bar graph. It demonstrated that autophagy of Hela cells occurred under the effect of inactivated H5N2 avian influenza virus.

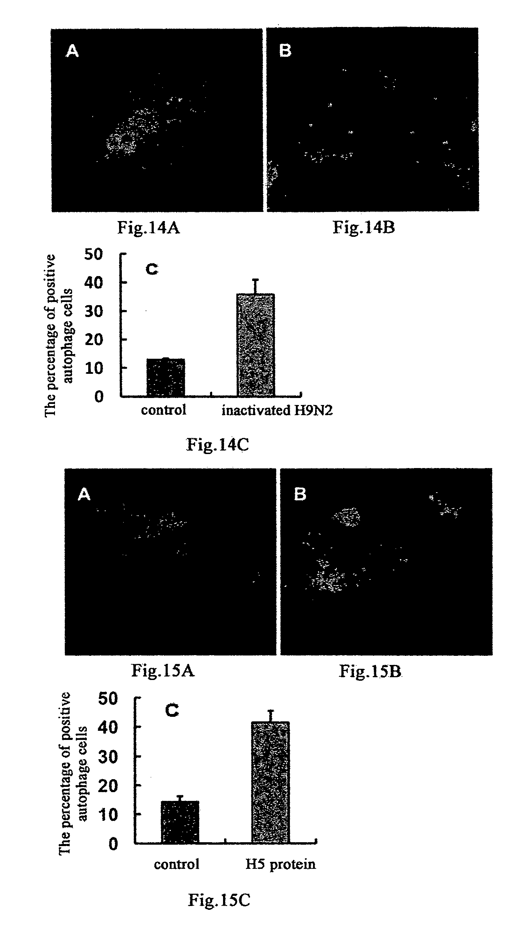

Figure 14 displays the photographs (×1000) of Hela cells transfected with EGFP-LC3 plasmid and treated in different ways under laser scanning confocal microscope and the percentage of cells with EGFP-LC3 agglutination in bar graph. Fig. A indicates photograph of Hela cells treated with adjuvant for 4 hours under the laser scanning confocal microscope. Fig.B indicates photographs of Hela cells treated with inactivated H9N2 avian influenza virus for 4 hours under laser scanning confocal microscope. Fig. C indicates the percentage of cells with EGFP-LC3 agglutination under the above two conditions in the bar graph. It demonstrates that autophagy of Hela cells occurred under the effect of inactivated H9N2 avian influenza virus.
Figure 15 displays photographs (×1000) of Hela cells transfected with EGFP-LC3 plasmid and then treated in different ways under laser scanning confocal microscope and the percentage of cells with EGFP-LC3 agglutination in bar graph. Fig. A indicates photograph of Hela cells treated with BSA protein for 4 hours under laser confocal microscope. Fig.B indicates photograph of Hela cells treated with H5 protein for 4 hours provided by laser confocal microscope. Fig. C indicates the percentage of Hela cells with EGFP-LC3 agglutination under the above two conditions in the bar graph. It demonstrates that autophagy of Hela cells occurred under the effect of H5 protein, and it also demonstrated that the expressed H5 protein has biological activity.

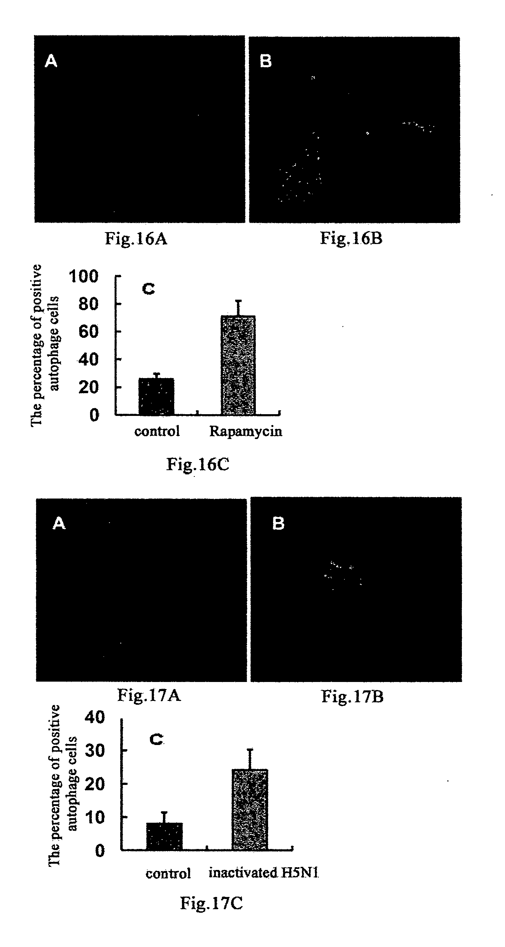

Figure 16 displays photographs (×1000) of Hela cells transfected with EGFP-LC3 plasmid and then treated in different ways under laser scanning confocal microscope and the percentage of the cells with EGFP-LC3 agglutination in bar graph. Fig. A indicates photograph of Hela cells treated with DMSO for 4 hours provided by laser confocal microscope. Fig.B indicates photographs of Hela cells treated with Rapamycin for 4 hours provided by laser confocal microscope. Fig. C indicates the percentage of Hela cells with EGFP-LC3 agglutination under the above two conditions in bar graph. It is known from literatures that Rapamycin induces the occurrence of cell autophagy. Thus, the method of the experiment is proven to be accurate and effective, considering Rapamycin as a positive control of the experiment.
Figure 17 displays photographs (×1000) of A549 cells transfected with EGFP-LC3 plasmid and then treated in different ways under laser scanning confocal microscope and the percentage of the Hela cells with EGFP-LC3 agglutination in bar graph. Fig. A indicates photograph of A549 cells treated with negative control, chicken chorioallantoic fluid for 4 hours provided by laser confocal microscope. Fig. B indicates photographs of A549 cells treated with inactivated H5N1 avian influenza virus for 4 hours provided by laser confocal microscope. Fig. C indicates the percentage of A549 cells with EGFP-LC3 agglutination under the above two conditions in bar graph. It demonstrates that autophagy of A549 cells occurred under the effect of inactivated H5N1 avian influenza virus.

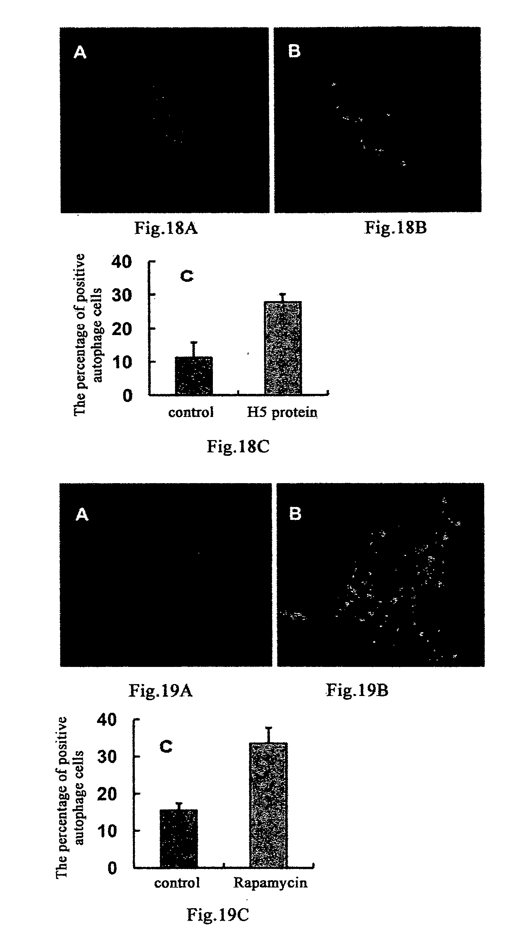

Figure 18 displays photographs (×1000) of A549 cells transfected with EGFP-LC3 plasmid and then treated in different ways under laser scanning confocal microscope and the percentage of Hela cells with EGFP-LC3 agglutination in bar graph. Fig. A indicates photograph of A549 cells treated with BSA for 4 hours provided by laser confocal microscope. Fig. B indicates photograph of A549 cells treated with H5 protein for 4 hours provided by laser confocal microscope. Fig. C indicates the percentage of A549 cells with EGFP-LC3 agglutination under the above two conditions in bar graph. It demonstrates that autophagy of A549 cells occurred under the effect of H5 protein, and it also demonstrated that the expressed H5 protein has biological activity.
Figure 19 displays photographs (×1000) of A549 cells transfected with EGFP-LC3 plasmid and then treated in different ways under laser scanning confocal microscope and the percentage of the Hela cells with EGFP-LC3 agglutination in bar graph. Fig. A indicates photograph of A549 cells treated with DMSO for 4 hours provided by laser confocal microscope. Fig.B indicates photographs of A549 cells treated with Rapamycin for 4 hours provided by laser confocal microscope. Fig. C indicates the percentage of A549 cells with EGFP-LC3 agglutination under the above two conditions in bar graph. It is known from literatures that Rapamycin induces the occurrence of cell autophagy. Thus, the method of the experiment is proven to be accurate and effective, considering Rapamycin as a positive control of the experiment.

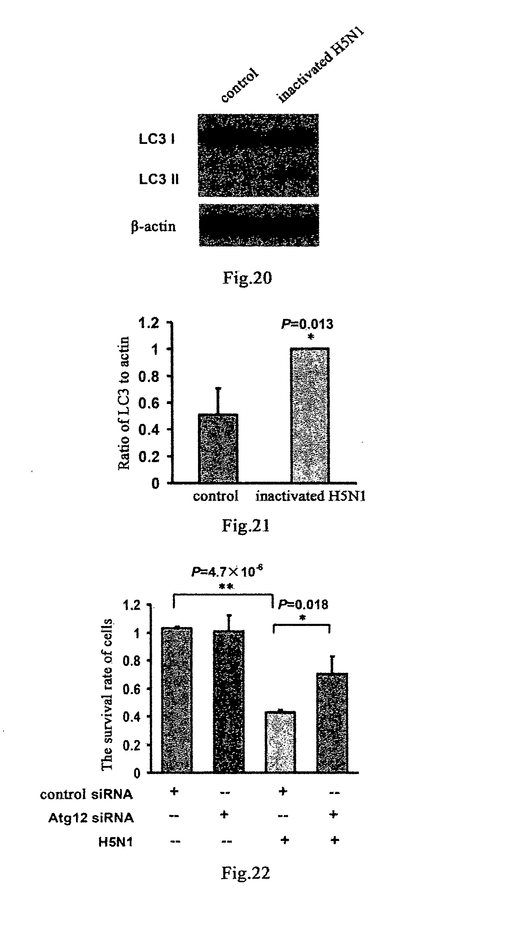

Figure 20 displays the results of Western Blot experiment of A549 cells treated in different ways. The first lane on the left was a lysis sample of A549 cells treated with negative control for 1.5 hours, and the second lane was a lysis sample of A549 cells treated with inactivated H5N1 avian influenza virus for 1.5 hours; The antibody was the antibody agonist LC3 and actin, respectively (from top to bottom). The relative expression level of LC3II was increased, demonstrating that the inactivated H5N1 avian influenza virus activates LC3 signal pathway and induces cell autophagy (type II PCD).
Figure 21 displays the relative ratio by quantifying the densities of the bands obtained from the repeated Western Blotting experiments shown in Figure 22 by using the software of Quantity one-4.6.3. The left one indicates the relative ratio of LC3II to actin of A549 cells treated with negative control in the Western Blotting experiments, and the right one indicates the relative ratio of LC3II compared with actin of A549 cells treated with inactivated H5N1 avian influenza virus in the Western Blotting experiments. The value of the ratio was adjusted to 1. It demonstrates that inactivated H5N1 avian influenza virus activates LC3 signal pathway and induces cell autophagy (type II PCD).
Figure 22 displays the survival percentages of A549 cells treated in different ways in bar graph. A549 cells were transfected with control siRNA and Atg12 siRNA respectively, then treated with control agent or inactivated H5N1 avian influenza virus. The results of cell survival rate were detected through MTT kit. It demonstrates that inactivated H5N1 avian influenza virus greatly reduces of survival rate of the cells, whereas Atg12 siRNA attenuates the effects of inactivated H5N1 avian influenza virus. Namely, inhibitory effect on cell autophagy (type II PCD) relieves the cell death induced by inactivated H5N 1 avian influenza virus.

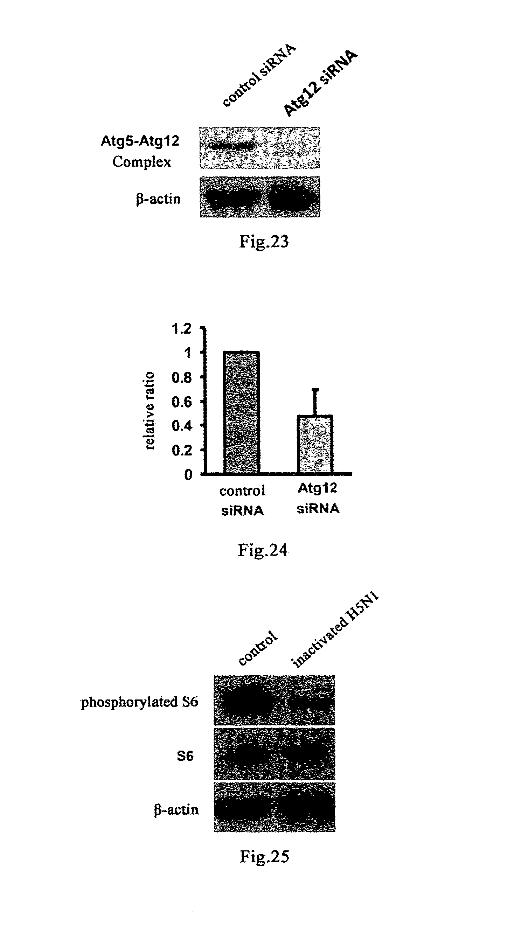

Figure 23 displays the result of Western Blotting experiments of A549 cells treated in different ways. The left lane was a lysis sample of A549 cells transfected with control siRNA and then treated with inactivated H5N1 avian influenza virus, and the right lane was a lysis sample of A549 cells transfected with control Atg12 siRNA and then treated with inactivated H5N1 avian influenza virus. The antibody used was the antibody agonist Atg12 and actin, respectively (from top to bottom). As Atg5 and Atg12 form complex inside the cell so as to induce cell autophagy (type II PCD), the detecting result was the relative amount of the complex of Atg5 and Atg12 to actin. It demonstrates that Atg12 siRNA effectively reduces the relative amount of complex of Atg5 and Atg 12.
Figure 24 displays the relative ratio in bar graph by quantifying the densities of the bands obtained from the repeated Western Blotting experiments shown in Figure 25 by using the software of Quantity one-4.6.3. The left one indicates the relative ratio of the complex of Atg5 and Atg12 to actin in A549 cells transfected with control siRNA, and the value was adjusted to 1. The right one indicates relative ratio of complex of Atg5 and Atg12 to actin in A549 cells transfected with control Atg12 siRNA. It demonstrates that Atg12 siRNA effectively reduces the relative amount of complex of Atg5 and Atg 12.
Figure 25 displays the result of Western Blotting experiments of A549 cells treated in different ways. The left lane was a lysis sample of A549 cells treated with negative control, and the right lane was a lysis sample of A549 cells treated with inactivated H5N1 avian influenza virus. The antibody used was the antibody agonist phosphorylated S6, S6 and actin, respectively (from top to bottom). S6 is the substrate of mTOR. The relative reduction of phosphorylated S6 indicated that the activity of mTOR pathway was inhibited, which demonstrates that inactivated H5N1 avian influenza virus inhibits mTOR signal pathway and further demonstrates that inactivated H5N1 avian influenza virus induces cell autophagy (type II PCD) via mTOR signal pathway.

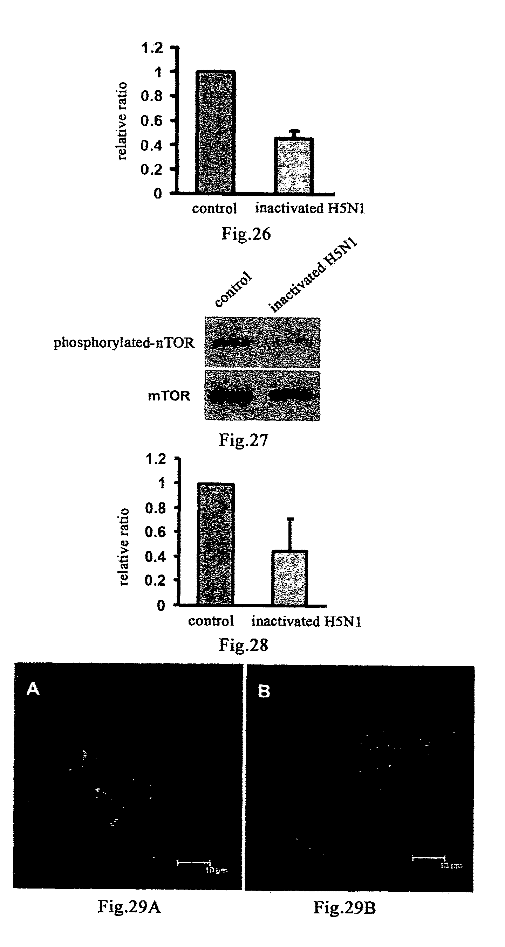

Figure 26 displays the relative ratio in bar graph by quantifying the densities of the bands obtained from the repeated Western Blotting experiments by using the software of Quantity one-4.6.3. The left one indicates the relative ratio of phosphorylated S6 to S6 in A549 cells treated with negative control in the Western Blotting experiments, and the value was adjusted to 1. The right one indicates the relative ratio of phosphatized S6 to S6 in A549 cells treated with inactivated H5N1 avian influenza virus in the Western Blotting experiments. It demonstrates that inactivated H5N1 avian influenza virus inhibits mTOR signal pathway and further demonstrates that inactivated H5N1 avian influenza virus induces cell autophagy (type II PCD) via inhibiting mTOR signal pathway.
Figure 27 displays the result of Western Blotting experiments of A549 cells treated in different ways. The left lane was a lysis sample of A549 cells treated with negative control, and the right lane was a lysis sample of A549 cells treated with inactivated H5N1 avian influenza virus. The antibodies used were antibodies agonist phosphorylated mTOR and mTOR, respectively (from top to bottom). The amount of phosphorylated mTOR was decreased relatively, suggesting that inactivated H5N1 avian influenza virus inhibits mTOR signal pathway and that inactivated H5N1 avian influenza virus induces cell autophagy (type II PCD) via inhibiting mTOR signal pathway.
Figure 28 displays the relative ratio in bar graph by quantifying the densities of the bands obtained from the repeated Western Blotting experiments by using the software of Quantity one-4.6.3. The left one indicates the relative ratio of phosphorylated mTOR to mTOR of A549 cells treated with negative control in the Western Blotting experiments, and the value was adjusted to 1. The right one indicates the relative ratio of phosphorylated mTOR and to mTOR of A549 cells treated with inactivated H5N1 avian influenza virus in the Western Blotting experiments. It demonstrates that inactivated H5N1 avian influenza virus inhibits mTOR signal pathway and that inactivated H5N1 avian influenza virus induces cell autophagy (type II PCD) via inhibiting mTOR signal pathway.
Figure 29 displays photographs (×1000) of A549 cells transfected with EGFP-LC3 plasmid and then treated in different ways under laser scanning confocal microscope. Fig. A indicates photograph of A549 cells transfected with negative control siRNA and then treated with inactivated H5N1 avian influenza virus provided by laser confocal microscope. Fig. B indicates photographs of A549 cells transfected with TSC2 siRNA and then treated with inactivated H5N1 avian influenza virus provided by laser confocal microscope. After transfection of TSC2 siRNA, cells with EGFP-LC3 agglutination was decreased, suggesting that TSC2 siRNA inhibits cell autophagy (type II PCD) induced by inactivated H5N1 avian influenza virus.

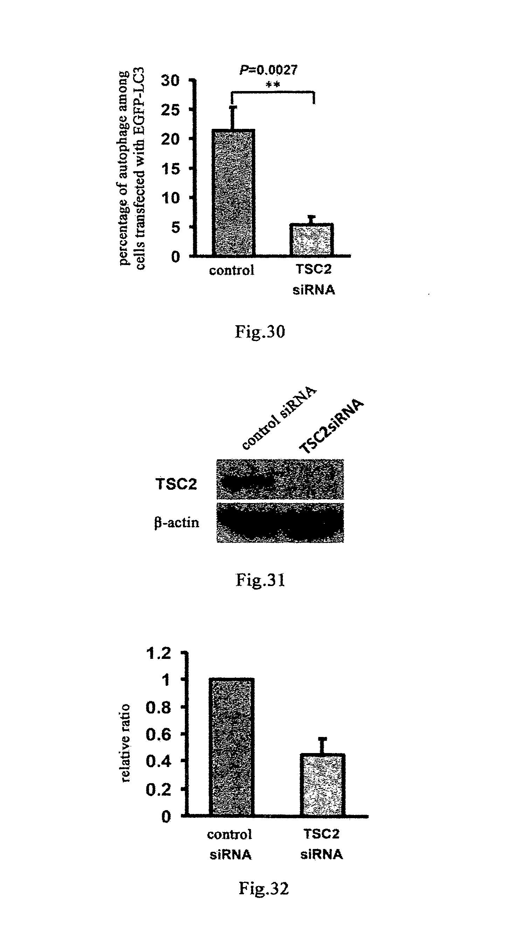

Figure 30 displays the percentage of cells with EGFP-LC3 agglutination among the A549 cells transfected with EGFP-LC3 plasmid and then treated in different ways in bar graph. The left one indicates percentage of the cells with EGFP-LC3 agglutination among the A549 cells transfected with negative control siRNA and then treated with inactivated H5N1 avian influenza virus, and the right one indicates the percentage of cells with EGFP-LC3 agglutination among A549 cells transfected with TSC2 siRNA and then treated with inactivated H5N1 avian influenza virus. It demonstrates that TSC2 siRNA inhibits cell autophagy (type II PCD) induced by inactivated H5N1 avian influenza virus.
Figure 31 displays the result of A549 cells treated in different ways in Western Blotting experiments. The samples of A549 cells transfected with control siRNA and TSC2 siRNA , respectively, were lysed 48h after the transfection; The antibodies used are antibodies agonist TSC2 and actin, respectively (from top to bottom). It demonstrates that TSC2 siRNA can significantly reduce the expression level of TSC2.
Figure 32 displays the relative ratio in bar graph by quantifying the densities of the bands obtained from the repeated Western Blotting experiments shown in Figure 25 by using the software of Quantity one-4.6.3. The left one indicates relative ratio of TSC2 to actin in A549 cells transfected with control siRNA, and the value of the ratio was adjusted to 1. The right one indicates relative ratio of TSC2 to actin in A549 cells transfected with TSC2 siRNA. It demonstrates that TSC2 siRNA can significantly reduce the expression level of TSC2.

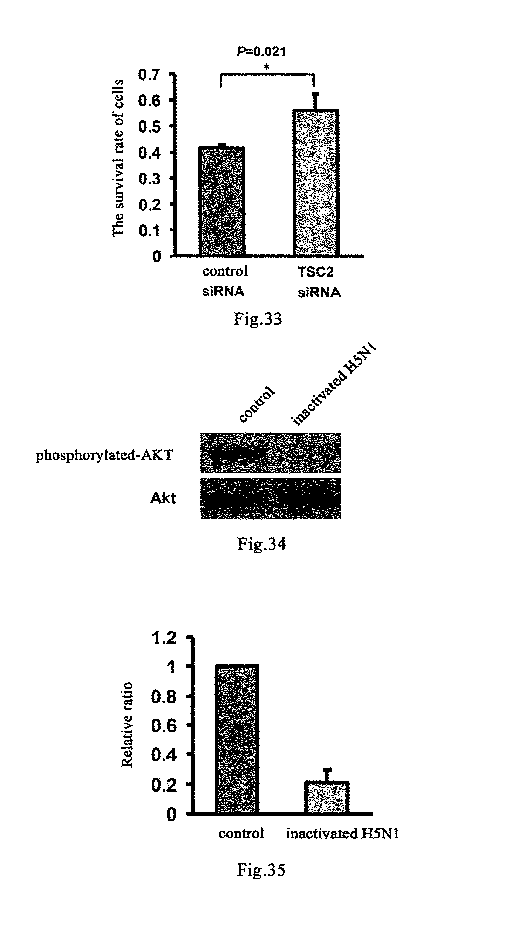

Figure 33 displays survival percentage of A549 cells treated in different ways in bar graph. A549 cells were transfected with control siRNA and TSC2 siRNA, respectively, and then treated with inactivated H5N1 avian influenza virus. The the result of the survival rate of the cells was detected by MTT kit. The survival rates of the cells were increased via the transfection of TSC2 siRNA. It demonstrates that TSC2 siRNA can delay the cell death induced by inactivated H5N1 avian influenza virus.
Figure 34 displays the result of Western Blotting experiments of A549 cells treated in different ways. The left lane was lysis sample of A549 cells treated with negative control, and the right lane was lysis sample of A549 cells treated with inactivated H5N1 avian influenza virus. The antibodies used were antibodies agonist phosphorylated Akt and Akt, respectively (from top to bottom). The reduction of the relative amount of phosphorylated Akt indicates that inactivated H5N1 avian influenza virus can inhibit Akt signal pathway.
Figure 35 displays the relative ratio in bar graph by quantifying the densities of the bands obtained from the repeated Western Blotting experiments by using the software of Quantity one-4.6.3. The left one indicates the relative ratio of phosphorylated Akt to Akt in A549 cells treated with negative control in the Western Blotting experiments, and the value of the ratio was adjusted to 1. The right one indicates the relative ratio of phosphorylated Akt to Akt in A549 cells treated with inactivated H5N1 avian influenza virus in the Western Blotting results. It demonstrates that inactivated H5N1 avian influenza virus can inhibit Akt signal pathway.

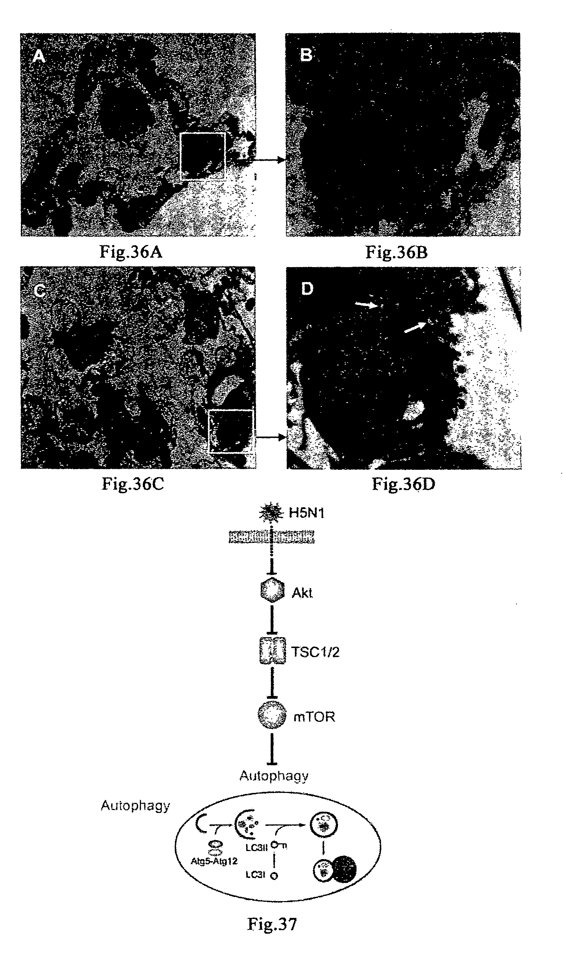

Figure 36 displays the electron micrographs of lung tissue of mice perfused with chicken chorioallantoic fluid or inactivated H5N1 avian influenza virus. Fig. A indicates electron micrograph of lung tissue perfused with chicken chorioallantoic fluid; Fig. B indicates partially enlarged view of the position shown in white box of A. A more complete cell was observed, however, no autophagosome was observed; Fig. C indicates electron micrograph of lung tissue perfused with inactivated H5N1 avian influenza virus; Figure D indicates partially enlarged view of the position shown in white box of A. A more complete cell was observed. An autophagosome in the cell was observed (represented by arrow). It demonstrates that inactivated H5N1 avian influenza virus can induce cell autophagy (type II PCD) of lung tissue.
Figure 37 displays diagram of cell signal pathway. From the above experimental result, we can get the conclusion as shown in the diagram: avian influenza virus can induce cell autophagy (type II PCD) via the pathway of from AKT to TSC1/2 to mTOR and autophagy. Avian influenza virus inhibits AKT, AKT inhibits TSC1/2, TSC1/2 inhibits mTOR pathway, mTOR pathway inhibits cell autophagy (type II PCD); The pathway of autophagyworks through Atg5-Atg12 to LC3 pathway so as to induce cell autophagy (type II PCD).

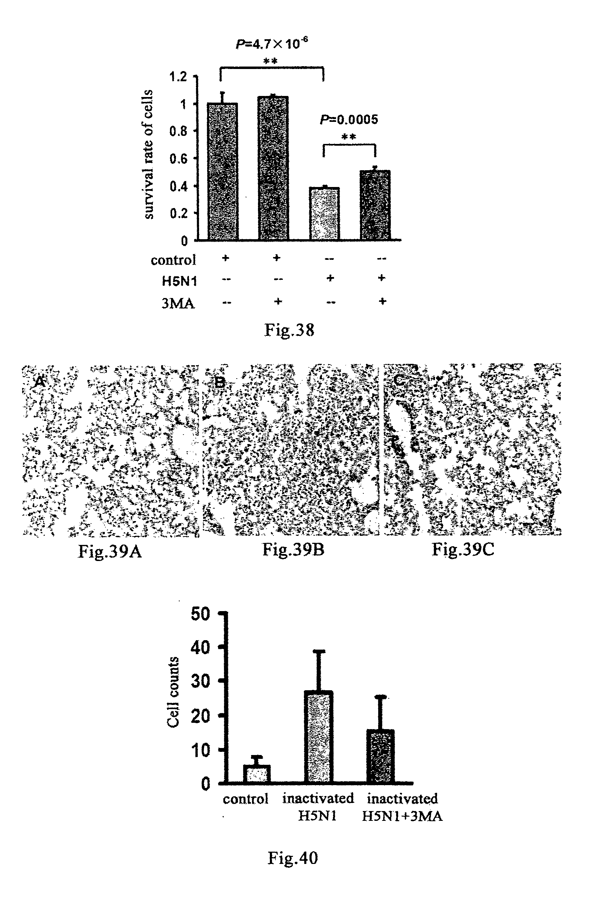

Figure 38 displays survival percentage of A549 cells treated in different ways in bar graph. After being treated with negative control, 3MA, or inactivated H5N1 avian influenza virus, the survival rates of A549 cells were detected through MTT kit. It demonstrates that inactivated H5N1 avian influenza virus greatly reduces the survival rate of the cells, whereas 3MA decreases the cell death induced by inactivated H5N1 avian influenza virus.
Figure 39 displays the photographs (×200) of pathological sections from lung of Balb/c mice. Fig. A indicates the photograph of mice injected with control (chicken chorioallantoic fluid) via trachea. 6 hours after injection, the lung was dissected and sliced to obtain pathological sections. Fig. B indicates the photographs of mice injected with inactivated H5N1 avian influenza virus via trachea. 6 hours after the injection, the lung was dissected and sliced to obtain pathological sections. Fig. C indicates the photograph of mice injected with 3-MA (30mg/kg) and inactivated H5N1 avian influenza virus. 30 minutes after the 3-MA injection, the mice were injected with inactivated H5N1 avian influenza virus via trachea. 6 hours later, the lung was dissected and sliced to obtained pathological sections. It demonstrates that inactivated H5N1 avian influenza virus can lead to severe lung injury, whereas 3-MA has some effect on ameliorating the lung injury induced by inactivated H5N1 avian influenza virus.
Figure 40 displays the counts of inflammatory cells located in pathological section of lung tissue under oil immersion lens (×1000) in bar graph. From left to right are the counting results in lung pathological sections from the mice injected with control (chicken chorioallantoic fluid) via trachea 6 hours before the dissection, from the mice injected with inactivated H5N1 avian influenza virus via trachea 6 hours before the dissection, from the mice firstly injected with 3-MA (30mg/kg) via intraperitoneal injection, and 30 min later, injected with inactivated H5N1 avian influenza virus via trachea 6 hours before the dissection, respectively. Inflammatory cell infiltration is one of the most important indicators. The injection of inactivated H5N1 avian influenza virus leaded to abundantly infiltrated inflammatory cells, whereas 3-MA reduced the counts of inflammatory cells induced by inactivated H5N1 avian influenza virus. The results indicate that inactivated H5N1 avian influenza virus leads to severe lung injury, whereas 3-MA has some effect on ameliorating the lung injury induced by inactivated H5N1 avian influenza virus.

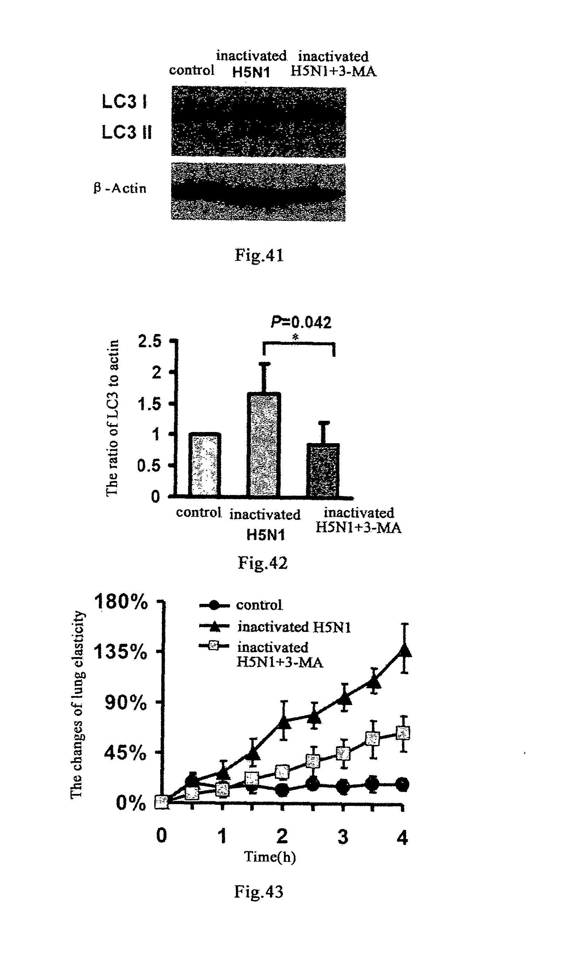

Figure 41 displays the results of Western Blot of Balb/c mice lung tissue LC3. The first lane indicates the sample of total protein extracted from homogenized lung tissue from the mice injected with control (chicken chorioallantoic fluid) via trachea for 2 hours; the second lane indicates the sample of total protein extracted from homogenized lung tissue from the mice injected with inactivated H5N1 avian influenza virus via trachea for 2 hours, and the third lane indicates the sample of total protein extracted from homogenized lung tissue from the mice injected with 3-MA (30mg/kg) via intraperitoneal injection firstly, and 30 min later injected with inactivated H5N1 avian influenza virus via trachea for 2 hours. The antibodies used are antibodies agonist LC3 and β-actin. It demonstrates that the content of LC3 II in mice lung tissue was increased by the stimulation of the inactivated H5N1 avian influenza virus and the autophagy occurred, whereas 3-MA relieved the occurrence of autophagy.
Figure 42 displays the relative ratio in bar graph by quantifying the densities of the bands obtained from the repeated Western Blotting experiments by using the software of Quantity one-4.6.3. The relative ratio of LC3 II to β-actin corresponds to the densities of bands shown in figure 42. It demonstrates that the content of LC3 II in mice lung tissue was increased by the stimulation of inactivated H5N1 avian influenza virus increases and autophagy occurred, whereas 3-MA relieved the occurrence of autophagy.
Figure 43 displays the result of elasticity of Balb/c mice lung tissue. It shows the changes of elasticity of Balb/c mice lung tissue from the mice group injected with control (chicken chorioallantoic fluid) via trachea, from the group injected with inactivated H5N1 avian influenza virus via trachea, and from the group injected with 3-MA (30mg/kg) via intraperitoneal injection firstly, and 30 min later injected with inactivated H5N1 avian influenza virus via trachea, respectively. The changes of lung elasticity of mice with spontaneous breath were detected every 30min for 4 hours. Lung elasticity is an important indicator to measure lung function. The injection of inactivated avian influenza virus via trachea greatly decreased the compliance of mice lung, whereas 3-MA has some effect on relieving the induced injury and on protecting function of lung.

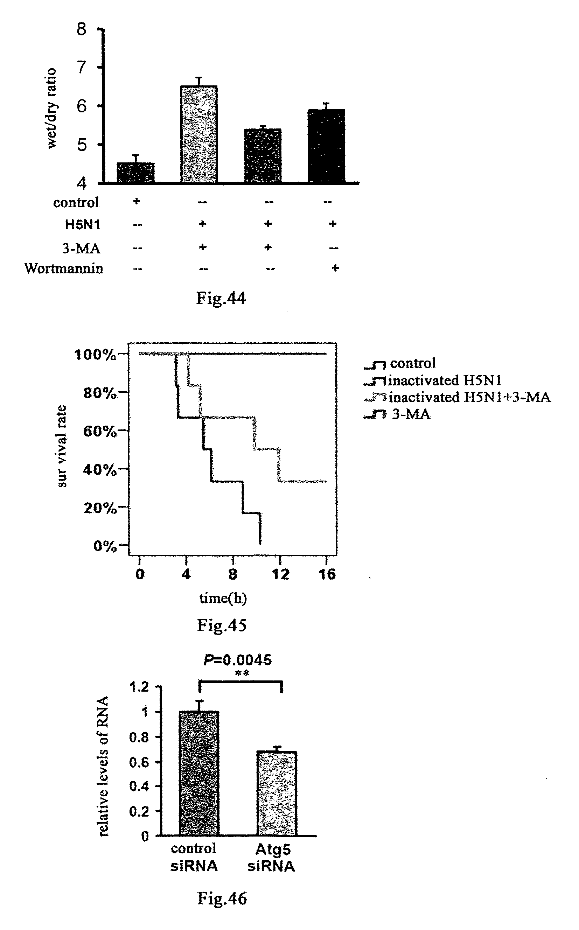

Figure 44 displays wet/dry ratio of Balb/c mice lung of in bar graph. It shows, from left to right, the wet/dry ratio of mice lung from the mice injected with control (chicken chorioallantoic fluid) 6 hours before the dissection via trachea, from the mice injected with inactivated H5N1 avian influenza virus 6 hours before the dissection via trachea, and from the mice injected with 3-MA (30mg/kg) via intraperitoneal injection firstly, and 30 min later injected with inactivated H5N1 avian influenza virus via trachea, from the mice injected with Wortmannin (1.5mg/kg) via intraperitoneal injection firstly, and 30 min later injected with inactivated H5N1 avian influenza virus via trachea. Lung wet/dry ratio is one of important indicators of lung injury. Injection of inactivated H5N1 avian influenza virus greatly increased the wet/dry ratio, whereas 3-MA and Wortmannin decreased the augmentation of wet/dry ratio induced by inactivated H5N1 avian influenza virus. The result indicates that inactivated H5N1 avian influenza virus leads to the severe lung injury, whereas 3-MA and Wortmannin have some effect on ameliorating the lung injury induced by inactivated H5N1 avian influenza virus.
Figure 45 displays the survival curve of Balb/c mice. The mice were injected with 3-MA (15mg/kg) via intraperitoneal injection. 30 min, 2h and 8h after the injection, the mice were injected with inactivated H5N1 avian influenza virus via trachea. The survival situations were observed every 15 minutes. It demonstrates that 3-MA has effect on delaying the death of mice.
Figure 46 displays the real-time PCR results of lung tissue of mice. The mice were injected with control siRNA and Atg5 siRNA, respectively, 24 hours later, lung tissues were homogenized, RNAs were extracted, and real-time PCRs were performed. It demonstrates that the injection of Atg5 siRNA via trachea effectively leads to the decrease of Atg5 mRNA level.

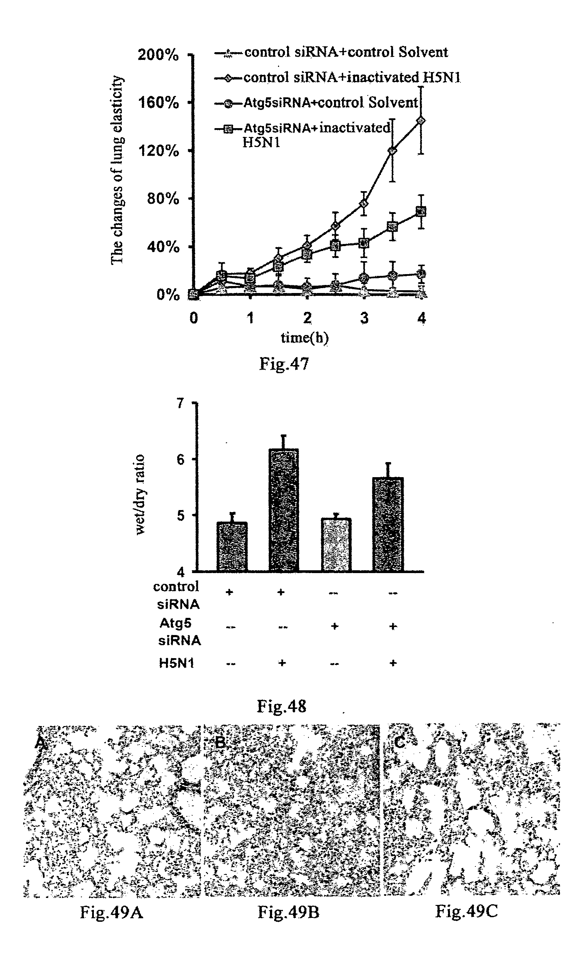

Figure 47 displays the result of elasticity of Balb/c mice lung tissue. The mice were injected with control siRNA and Atg5 siRNA, respectively. 24 hours later, the mice were injected with control (chicken chorioallantoic fluid) and inactivated H5N1 avian influenza virus, respectively. The elasticity of mice lung tissue was detected every 30min. Detections of the changed lung elasticity of the mice with spontaneous breath were performed within 4 hours. The injection of inactivated avian influenza virus via trachea greatly decreased the compliance of mice lung, whereas Atg5 siRNA has some effect on relieving the lung injury via inhibiting the expression of Atg5 protein, suggesting that inactivated H5N1 avian influenza virus induces the occurrence of lung injury via activating cell autophagy (type II PCD).
Figure 48 displays lung wet/dry ratio of Balb/c mice in bar graph. Mice were injected with control siRNA and Atg5 siRNA, respectively. 24 hours later, mice were injected with control (chicken chorioallantoic fluid) and inactivated H5N1 avian influenza virus, respectively. The wet/dry ratio of mice lung tissue of was shown 4 hours later. Lung wet/dry ratio is one of important indicators of lung injury. Injection of inactivated H5N1 avian influenza virus greatly increased wet/dry ratio, whereas Atg5 siRNA has some effect on relieving lung injury through inhibiting the expression of Atg5 protein, suggesting that inactivated H5N1 avian influenza virus induces the occurrence of lung injury through activating cell autophagy (type II PCD).
Figure 49 displays photographs (×200) of Balb/c mice lung in pathological sections. Fig. A indicates the photographs of mice injected with Fc protein via trachea after acid aspiration. The lung was dissected 6h later to obtain pathological sections. Fig.B indicates photographs of mice injected with H5Fc protein via trachea after acid aspiration. The lung was dissected 6h later to obtain pathological sections. Fig. C indicates photographs of mice injected with 3-MA (30mg/kg) via intraperitoneal injection firstly, and 30 min later injected with H5Fc protein via trachea after acid aspiration. The lung was dissected 6h later to obtain pathological sections. It indicates that H5Fc protein after acid aspiration aggravates the lung injury, whereas 3-MA has effect on ameliorating the lung injury induced by H5Fc protein after acid aspiration.

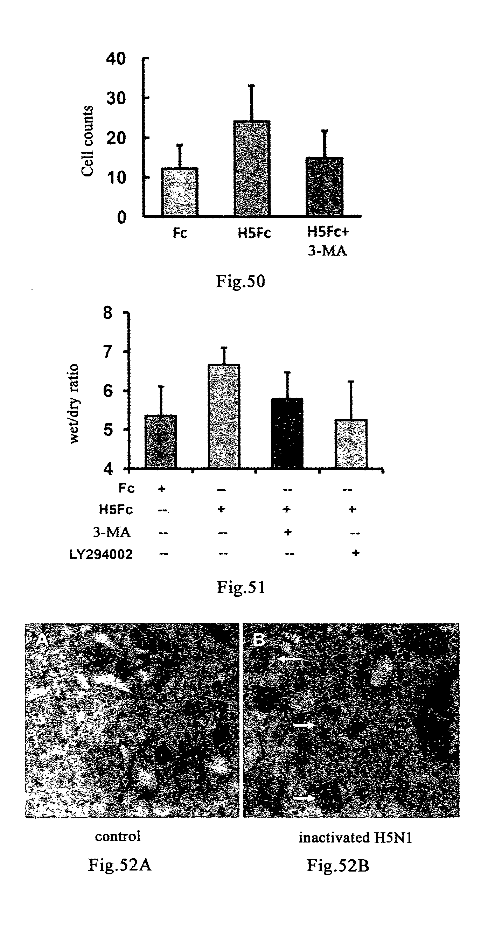

Figure 50 displays the counts of inflammatory cell of lung tissue in pathological sections under oil immersion lens (×1000) in bar graph. From left to right, indicates the counting results in lung pathological sections from the mice injected with Fc protein via trachea after acid aspiration, from the mice injected with H5Fc protein via trachea after acid aspiration, from the mice injected with 3-MA (30mg/kg) via intraperitoneal injection firstly, and 3min later injected with H5Fc protein via trachea after acid aspiration, respectively. The lung was dissected 6h later. Inflammatory cell infiltration is one of important indicators. The injection of H5Fc protein increased the counts of infiltrated inflammatory cells, whereas 3-MA reduced the augmentation of the counts inflammatory cells induced by H5Fc protein. The result proves that H5Fc protein after acid aspiration can aggravate lung injury, whereas 3-MA has some effect on ameliorating the lung injury induced by H5Fc protein after acid aspiration.
Figure 51 displays wet/dry ratio of Balb/c mice lung in bar graph. From left to right are lung wet/dry ratio of mice injected with Fc protein via trachea after acid aspiration, lung wet/dry ratio of mice injected with H5Fc protein via trachea after acid aspiration, lung wet/dry ratio of mice injected with 3-MA (30mg/kg) via intraperitoneal injection firstly, and 30 min later injected with H5Fc protein via trachea after acid aspiration, respectively, lung wet/dry ratio of mice injected with LY294002 (0.25mg/kg) via intraperitoneal injection firstly, and 30 min later injected with H5Fc protein via trachea for 6 hours after acid aspiration. Lung wet/dry ratio is one of important indicators of lung injury. The injection of H5Fc protein via trachea after acid aspiration greatly increased the wet/dry ratio, whereas 3-MA and LY294002 decreased the augmentation of wet/dry ratio induced by H5Fc protein after acid aspiration. The result proves that H5Fc protein after acid aspiration leads to the severe lung injury, whereas 3-MA and LY294002 have some effect on ameliorating the lung injury induced by H5Fc protein after acid aspiration.

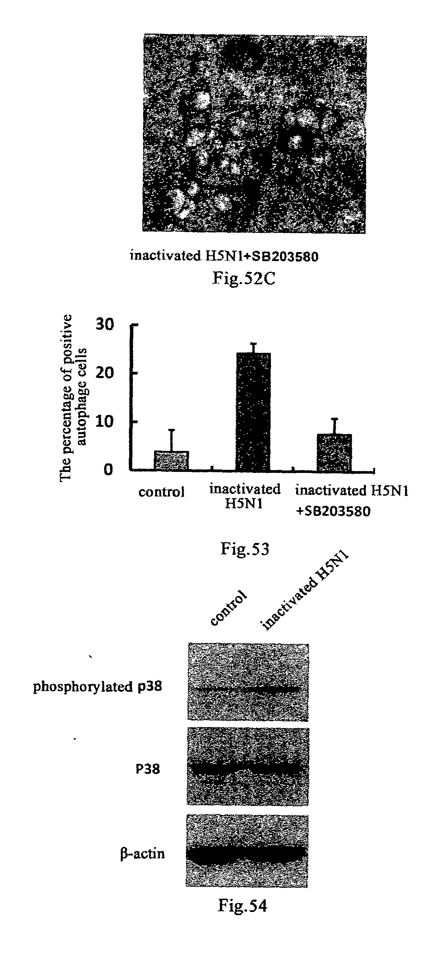

Figure 52 displays electron micrograph (×20000) of A549 cells treated in different ways. The first photograph of Fig. A indicates the electron micrograph of A549 cells treated with adjuvant for 4 hours. Fig.B indicates the electron micrograph of A549 cells treated with Inactivated H5N1 avian influenza virus for 4 hours, and Fig. C indicates the electron micrograph of A549 cells pretreated with SB203580, a specific inhibitor of P38 pathway for 1 hour firstly, and then treated with inactivated H5N1 avian influenza virus for 4 hours. It demonstrates that autophagy of A549 cells occurred under the effect of inactivated H5N1 avian influenza virus, whereas SB203580, a specific inhibitor of P38 pathway reduced cell autophagy.
Figure 53 displays percentage of A549 cells with autophagy (type II PCD) after various treatments in bar graph under electron microscope. The first graph on the left indicates percentage of A549 cells with autophagy (type II PCD) after treatment with adjuvant for 4 hours, the second graph indicates the percentage of A549 cells with autophagy (type II PCD) after treatment with inactivated H5N1 avian influenza virus for 4 hours, the third graph indicates the percentage of A549 cells with autophagy (type II PCD) after pretreatment with SB203580, a specific inhibitor of P38 pathway for 1 hour, and then with inactivated H5N1 avian influenza virus for 4 hours. Results show that the ratio of A549 cells with autophagy after the treatment only with inactivated H5N1 avian influenza virus is 24.3%. By pretreated with SB203580, an inhibitor of P38 pathway prior to the treatment with inactivated H5N1 avian influenza virus, the ratio of A549 cells with autophagy is 7.73%. There is significant difference between the two cases. The result proves that SB203580, the inhibitor of P38 pathway can effectively inhibit the occurrence of autophagy induced by inactivated H5N1 avian influenza virus.
Figure 54 displays the results of A549 cells treated in different ways in Western Blotting experiments. The first lane on the left indicates a lysis sample of A549 cells treated with adjuvant for 4 hours. The second lane indicates a lysis sample of A549 cells treated with inactivated H5N1 avian influenza virus for 4 hours. The antibodies used are antibodies agonist phosphorylated P38, P38 and actin, respectively (from top to bottom). It demonstrates that in the case that when the amount of samples was roughly the same (actin was used as internal parameter to ensure the same amount of samples), the expression level of phosphorylated P38 was significantly increased. Namely, inactivated H5N1 avian influenza virus can activate P38 signal pathway.

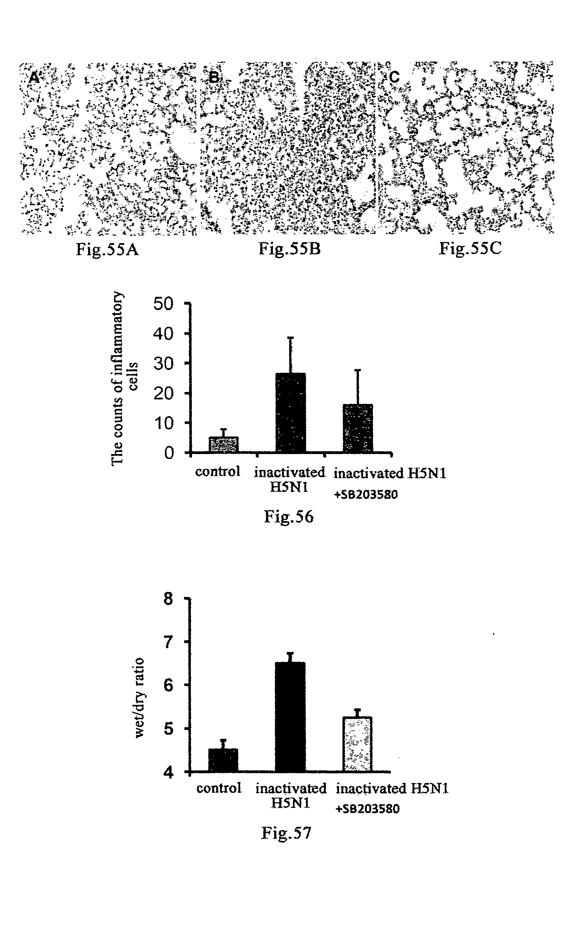

Figure 55 displays photograph (×200) of pathological sections of Balb/c mice lung. Fig. A indicates the photograph of pathological sections from mice injected with control (chicken chorioallantoic fluid) via trachea. The lung was dissected 6 hours after the injection. Fig. B indicates the photograph of pathological sections from mice injected with inactivated H5N1 avian influenza virus via trachea. The lung was dissected 6 hours after the injection. Fig. C indicates the photograph of pathological sections from mice injected with SB203580 (16mg/kg) via intraperitoneal injection firstly, and 30 min later injected with inactivated H5N1 avian influenza virus via trachea. The lung was dissected 6 hours after the injection. It demonstrates that inactivated H5N1 avian influenza virus leaded to severe lung injury, whereas SB203580 has some effect on ameliorating the lung injury induced by inactivated H5N1 avian influenza virus. It shows that inactivated H5N1 avian influenza virus induces lung injury through p38 signal pathway.
Figure 56 displays the counts of inflammatory cells in lung tissue pathological sections under oil immersion lens (×1000). From left to right were the counting results in lung pathological sections of mice injected with control (chicken chorioallantoic fluid) via trachea, the counting results in lung pathological sections of mice injected with inactivated H5N1 avian influenza virus via trachea, the counting results in lung pathological sections of mice injected with SB203580 (16mg/kg) via intraperitoneal injection firstly, and 30min later injected with inactivated H5N1 avian influenza virus via trachea, respectively. The lung was dissected 6 hours after the injection. Inflammatory cell infiltration is one of important indicators of lung injury. Injection of inactivated H5N1 avian influenza virus leaded to abundantly infiltrated inflammatory cells, whereas SB203580 reduced the counts of infiltrated inflammatory cells induced by inactivated H5N1 avian influenza virus. The results prove that inactivated H5N1 avian influenza virus leads to severe lung injury, whereas SB203580 has some effect on ameliorating the lung injury induced by inactivated H5N1 avian influenza virus.
Figure 57 displays wet/dry ratio of Balb/c mice lung in bar graph. They indicate, from left to right, lung wet/dry ratio of mice 6 hours after the injection of control (chicken chorioallantoic fluid) via trachea, lung wet/dry ratio of mice 6 hours after injection of inactivated H5N1 avian influenza virus via trachea, lung wet/dry ratio of mice 6 hours after the injection with SB203580 (16mg/kg) via intraperitoneal injection firstly, and 1h later injected with inactivated H5N1 avian influenza virus via trachea. Lung wet/dry ratio is one of important indicators of lung injury. The injection of inactivated H5N1 avian influenza virus greatly increased wet/dry ratio, whereas SB203580 decreased augmentation of wet/dry ratio induced by inactivated H5N1 avian influenza virus. The result proves that inactivated H5N1 avian influenza virus leads to the severe lung injury, whereas SB203580 has some effect on ameliorating the lung injury induced by inactivated H5N1 avian influenza virus.

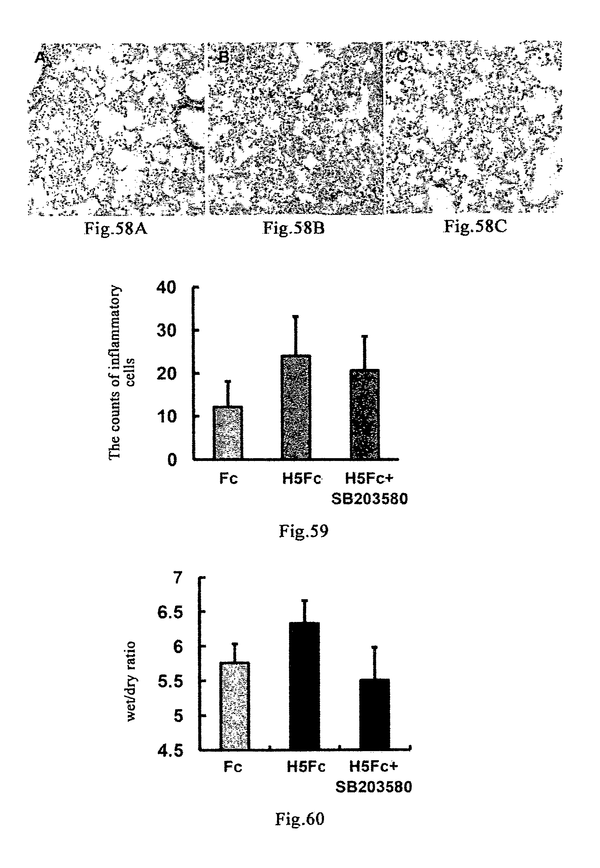

Figure 58 displays photograph (×200) of lung pathological sections of Balb/c mice. The left one is the photograph of pathological sections from mice injected with Fc protein via trachea after acid aspiration. The lung was dissected 6h after the injection. The middle one is the photograph of pathological sections from mice injected with H5Fc protein via trachea after acid aspiration. The lung was dissected 6h after the injection. The right one is the photograph of pathological sections from mice injected with SB203580 (16mg/kg) via intraperitoneal injection firstly, and 30min later injected with H5Fc protein via trachea after acid aspiration. The lung was dissected 6h after the last injection. It proves that injection of H5Fc protein after acid aspiration aggravated the lung injury, whereas SB203580 has some effect on amelioratiing the lung injury induced by H5Fc protein after acid aspiration.
Figure 59 displays the counts of inflammatory cells in lung tissue pathological sections under oil immersion lens (×1000). Fig. A indicates the counting results in lung pathological sections from mice injected with Fc protein via trachea after acid aspiration. Fig.B indicates counting results in lung pathological sections from mice injected with H5Fc protein via trachea after acid aspiration. Fig. C indicates counting results in lung pathological sections from mice injected with SB203580 (16mg/kg) via intraperitoneal injection firstly, and 30 min later injected with H5Fc protein via trachea after acid aspiration, respectively. The lung was dissected 6h after the last injection. Inflammatory cell infiltration is one of important indicators of lung injury. The injection of H5Fc protein increased the counts of infiltrated inflammatory cells, whereas SB203580 reduced the augmentation of infiltrated inflammatory cells induced by H5Fc protein. The result proves that H5Fc protein after acid aspiration aggravates lung injury, whereas SB203580 has some effect on ameliorating the lung injury induced by H5Fc protein after acid aspiration.
Figure 60 displays lung wet/dry ratio of Balb/c mice in bar graph. From left to right, they indicate lung wet/dry ratio of mice injected with Fc protein via trachea after acid aspiration, lung wet/dry ratio of mice injected with H5Fc protein via trachea after acid aspiration, lungwet/dry ratio of mice injected with SB203580 (16mg/kg) via intraperitoneal injection firstly, and then 30min later injected with H5Fc protein via trachea after acid aspiration, respectively. The lung was dissected 6h after the last injection. Lung wet/dry ratio is one of important indicators of lung injury, the injection of H5Fc protein via trachea after acid aspiration greatly increased wet/dry ratio, whereas SB203580 decreased the augmentation of wet/dry ratio induced by H5Fc protein after acid aspiration. The result proves that H5Fc protein after acid aspiration leads to the severe lung injury, whereas SB203580 has some effect on ameliorating the lung injury induced by H5Fc protein after acid aspiration.

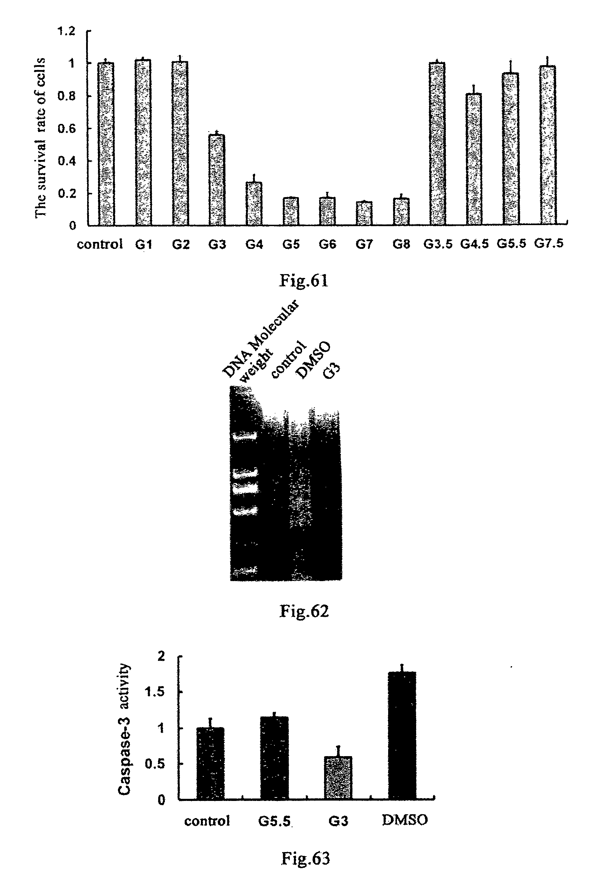

Figure 61 displays percentage of survival A549 cells after various treatments. A549 cells were treated with various generations of nanometer materials (PAMAM). 24h after the treatment, percentage of survival A549 cells was detected through MTT agent.
Figure 62 displays genomic electrophoretogram of A549 cells treated in different ways. The A549 cells were collected after treated with control, dimethyl sulfoxide (DMSO, 6% v/v) and nanometer material PAMAM G3 (100µg/mL). The genomic DNA of cells was isolated with Genome Extraction Kit and agarose gel electrophoresis was performed. Dimethyl sulfoxide was serving as inducer for apoptosis.
Figure 63 displays the result of Caspase-3 activity in A549 cells treated in different ways. A549 cells were treated with control, dimethyl sulfoxide (DMSO, 6% v/v), nanometer material PAMAM G5.5 and PAMAM G3 generation for 24 hours, respetively. The Caspase-3 activity of cells was detected with Caspase-3 activity test kit.

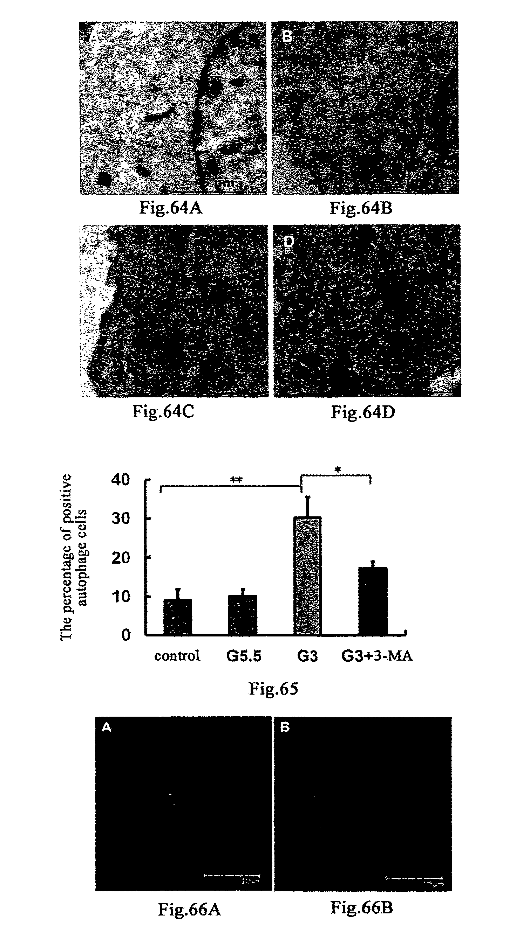

Figure 64 displays electron micrograph (×20000) of A549 cells treated in different ways. Fig. A indicates the electron micrograph of A549 cells treated with control for 24 hours. Fig .B indicates the electron micrograph of A549 cells treated with nanometer material PAMAM G5.5 (100µg/mL) for 24 hours. Fig. C indicates the electron micrograph of A549 cells treated with nanometer material PAMAM G3 (100µg/mL) for 24 hours. Fig. D indicates the electron micrograph of A549 cells firstly treated with 3-MA (10mM) for 1 hour, and then treated with nanometer material PAMAM G3 (100µg/mL) for 24 hours. It demonstrates that autophagy of A549 cells occurred under the effect of nanometer material PAMAM G5, and the cell death induced by cell autophagy can be relieved by 3-MA.
Figure 65 displays the percentage of A549 cells with autophagy (type II cell apoptosis) in bar graph. Typical characterization after occurrence of cell autophagy is the appearance of autophagosome. The percentage of cells having more than or equal to 2 autophagosomes per hundred random cells was calculated. It is obvious that 3-MA reduced the percentage of cells with autophagy (type II cell apoptosis) induced by nanometer material PAMAM G3. Namely 3-MA can treat or relieve cell autophagy (type II cell apoptosis).

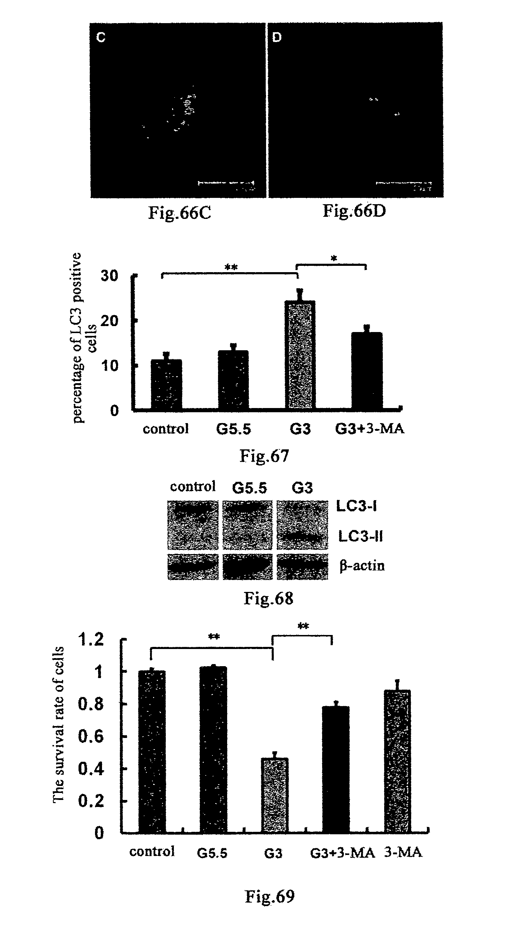

Figure 66 displays photographs (×1000) of A549 cells transfected with EGFP-LC3 plasmid and then treated in different ways under laser scanning confocal microscope. Fig. A indicates photographs of A549 cells transfected with EGFP-LC3 plasmid and then treated with control for 24 hours provided by laser confocal microscope. Fig.B indicates photograph of A549 cells transfected with EGFP-LC3 plasmid and then treated with nanometer material PAMAM G5.5 (100µg/mL) for 24 hours provided by laser confocal microscope. Fig. C indicates photograph of A549 cells transfected with EGFP-LC3 plasmid and then treated with nanometer material PAMAM G3 (100µg/mL) for 24 hours provided by laser confocal microscope. Fig. D indicates photograph of A549 cells transfected with EGFP-LC3 plasmid and treated with 3-MA (10mM) firstly for 1 hour, and then treated with nanometer material PAMAM G3 (100µg/mL) for 24 hours provided by laser confocal microscope.
Figure 67 displays the percentage of cells with EGFP-LC3 agglutination under the above conditions in bar graph. Typical characterization for the occurrence of cell autophagy is the agglutination of LC3 (ATG8) molecular so as to form autophagysome. LC3 molecular was labeled with EGFP. If the cell autophagy occurs, LC3 molecular will aggregate, and strongly emitting green fluorescence can be observed under confocal microscope. However, if the cells autophagy does not occur, green fluorescence disperses or only a little agglutinates. It demonstrates that of the autophagy of A549 cells occurred under the effect of nanometer material PAMAM G3, and such cell death induced by cell autophagy can be relieved by 3-MA.
Figure 68 displays the results of Western Blotting experiments of A549 cells treated in different ways. The first lane on the left indicates a lysis sample of A549 cells treated with control for 4 hours, the second lane indicates a lysis sample of A549 cells treated with nanometer material PAMAM G5.5 (100µg/mL) for 4 hours, and the third lane indicates a lysis sample of A549 cells treated with nanometer material PAMAM G3 (100µg/mL) for 4 hours; The antibodies used were antibodies agonist LC3B and actin, respectively (from top to bottom). It demonstrates that the expression level of LC3B-II protein was significantly increased. Namely, nanometer material PAMAM G3 can induce cell autophagy.
Figure 69 displays the percentage of survival A549 cells after different treatments. A549 cells were pretreated with control, nanometer material PAMAM G5.5, PAMAM G3, 3-MA for 1 hour, respectively, and then treated with nanometer material PAMAM G3 and drug control 3-MA for 24 hours. The result of cell survival rate was detected by MTT method. It shows that 3-MA can significantly reduce cell death induced by nanometer material PAMAM G3.

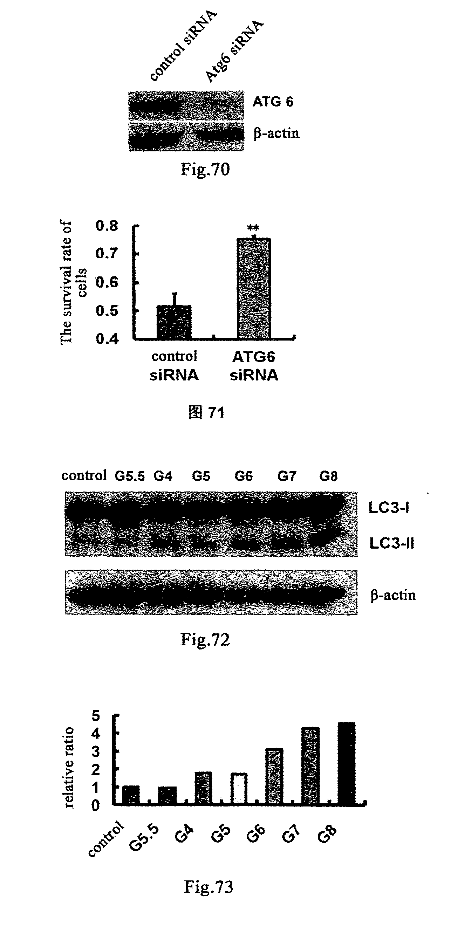

Figure 70 displays the results of Western Blot experiments of A549 cells after different treatments. The samples were the lysis samples of A549 cells transfected with Control siRNA or with ATG6 siRNA, respectively, for 48 hours. The antibodies were antibodies agonist ATG6 and actin, respectively (from top to bottom). The results of Western Blotting experiments show that ATG6 siRNA effectively inhibits the expression of ATG6 gene.
Figure 71 displays the percentage of survival A549 cells after various treatments. A549 cells were transfected with Control siRNA and ATG6 siRNA , respectively. Then treated with nanometer material PAMAM G3 (100µg/mL) for 24 hours. The cell survival rate was detected by MTT method. It demonstrates that nanometer material PAMAM G3 induces the cell death by regulating expression of ATG6 gene so as to form autophagy.
Figure 72 displays the result of Western Blotting experiments of A549 cells treated in different ways. A549 cells were treated with different generations of nanometer materials (PAMAM) for 4 hours. Then the expression level of LC3-II in cells was detected through Western Blotting. It shows that nanometer materials PAMAM G4, G5, G6, G7, G8 all significantly increase the expression level of LC3-II in cells, suggesting that all of these nanometer materials can induce autophagy to cause cell death.

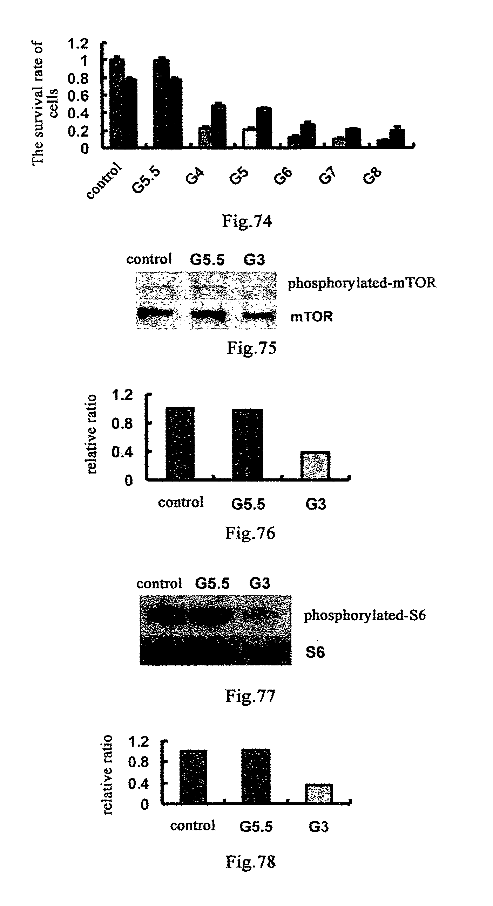

Figure 73 displays the relative ratio in bar graph by quantifying the densities of the bands obtained from the Western Blotting experiments shown in Figure 75 by using the software of Quantity one-4.6.3. They successively indicates the relative ratio of LC3II to actin in the Western Blotting experiment of A549 cells treated with negative control, G5.5, G4, G6, G7 and G8, and the Control value was adjusted to 1. The values of other groups were divided by the Control value. It demonstrates that G4, G5, G6, G7 and G8 can activate LC3 signal pathway and induce cell autophagy (type II cell apoptosis).
Figure 74 displays the result of Western Blotting of A549 cells treated in different ways. A549 cells were treated with different generations of nanometer materials (PAMAM) for 24 hours, and the result of cell survival rate were detected by MTT method. It demonstrates that nanometer materials PAMAM G4, G5, G6, G7 and G8 all can induce cell death.
Figure 75 displays the result of Western Blotting experiments of A549 cells treated in different ways. The first lane indicates a lysis sample of A549 cells treated with control for 24 hours, the second lane indicates a lysis sample of A549 cells treated with nanometer material PAMAM G5.5 for 24 hours, and the third lane indicates a lysis sample of A549 cells treated with nanometer material PAMAM G3 for 24 hours; The antibodies used were antibodies agonist phosphorylated mTOR on position 2481 and total mTOR, respectively (from top to bottom). It demonstrates that the expression level of phosphorylated mTOR protein was significantly decreased. Namely, mTOR pathway was inhibited.
Figure 76 displays the relative ratio in bar graph by quantifying the densities of the bands obtained from the Western Blotting experiments shown in Figure 78 by using the software of Quantity one-4.6.3. They indicates successively the relative ratio of phosphorylated mTOR to mTOR protein in the Western Blotting experiments of A549 cells treated with negative control, G5.5 (100µg/mL), and G3 (100µg/mL). The Control value was adjusted to 1. Values of other groups were divided by the Control value. It demonstrates that G3 can inhibit mTOR signal pathway and induce cell autophagy (type II cell apoptosis).
Figure 77 indicates the results of Western Blotting experiments of A549 cells treated in different ways. The first lane indicates a lysis sample of A549 cells treated with control for 24 hours, the second lane indicates a lysis sample of A549 cells treated with nanometer material PAMAM G5.5 (100µg/mL) for 24 hours, and the third lane indicates a lysis sample of A549 cells treated with nanometer material PAMAM G3 (100µg/mL) for 24 hours; The antibodies used were antibodies agonist phosphorylated S6 and total S6, respectively (from top to bottom). It demonstrates that the expression level of phosphorylated S6 protein was significantly decreased, and S6 pathway was inhibited. Namely, mTOR pathway was inhibited.

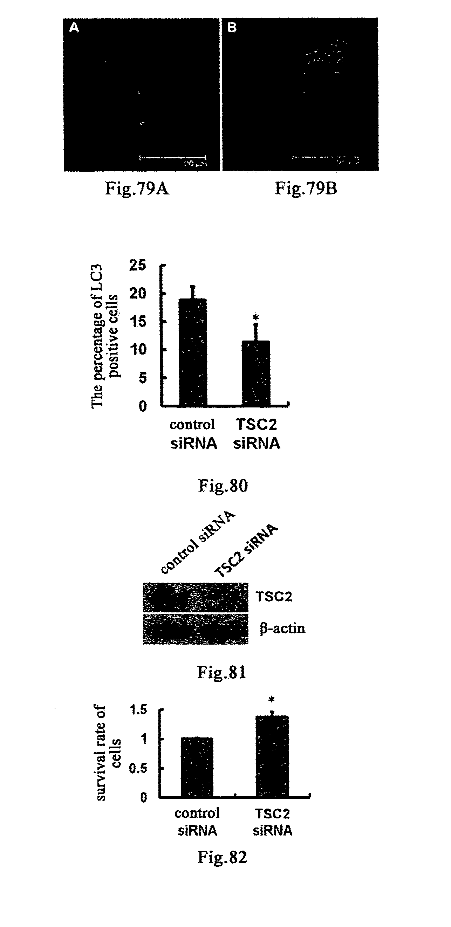

Figure 78 displays the relative ratio in bar graph by quantifying the densities of the bands obtained from the Western Blotting experiments shown in Figure 80 by using the software of Quantity one-4.6.3. They successively indicate the relative ratio of phosphorylated S6 to S6 protein in the Western Blotting experiments of A549 cells treated with negative control, G5.5 (100µg/mL), and G3 (100µg/mL), and the Control value was adjusted to 1. Values of other groups were divided by the Control value. It demonstrates that G3 can inhibit the activation of S6, namely G3 can inhibit mTOR signal path, and induce cell autophagy (type II cell apoptosis).
Figure 79 displays photograph (×1000) of A549 cells transfected with EGFP-LC3 plasmid and then treated in different ways provided by laser scanning confocal microscope. Fig. A indicates photograph of cells treated with Control siRNA for 24 hours, and then treated with nanometer material PAMAM G3 (100µg/mL) provided by laser confocal microscope. Fig.B indicates photograph of cells treated with TSC2 siRNA for 24 hours, and then treated with nanometer material PAMAM G3 (1 00µg/mL) provided by laser confocal microscope.
Figure 80 displays the percentage of cells with EGFP-LC3 agglutination after the A549 cells transfected with EGFP-LC3 plasmid were treated in different ways. Fig. A indicates the percentage of cells with EGFP-LC3 agglutination after A549 cells transfected with negative control siRNA and then treated with PAMAM G3 (100µg/mL), and Fig.B indicates the percentage of cells with EGFP-LC3 agglutination after A549 cells transfected with TSC2 siRNA and then treated with PAMAM G3 (100µg/mL). It demonstrates that TSC2 siRNA can inhibit cell autophagy (type II cell apoptosis) induced by PAMAM G3 (100µg/mL).
Figure 81 displays the results of Western Blotting experiments of A549 cells treated in different ways. A549 cells were transfected with control siRNA and TSC 2 siRNA firstly, and then treated with nanometer material PAMAM G3 (100µg/mL) for 24 hours, and the survival rate of cells were detected by MTT method. It shows that TSC2 siRNA effectively inhibits the expression of TSC2 gene.
Figure 82 displays percentage of survival A549 cells after various treatments in bar graph. A549 cells were transfected with control siRNA and TSC 2 siRNA, respectively, then treated with nanometer material PAMAM G3 (100µg/mL) for 24 hours. The survival rates of the cells were detected by MTT method. It demonstrates that nanometer material PAMAM G3 induces the cell death by regulating the expression of TSC 2 gene to form autophagy.

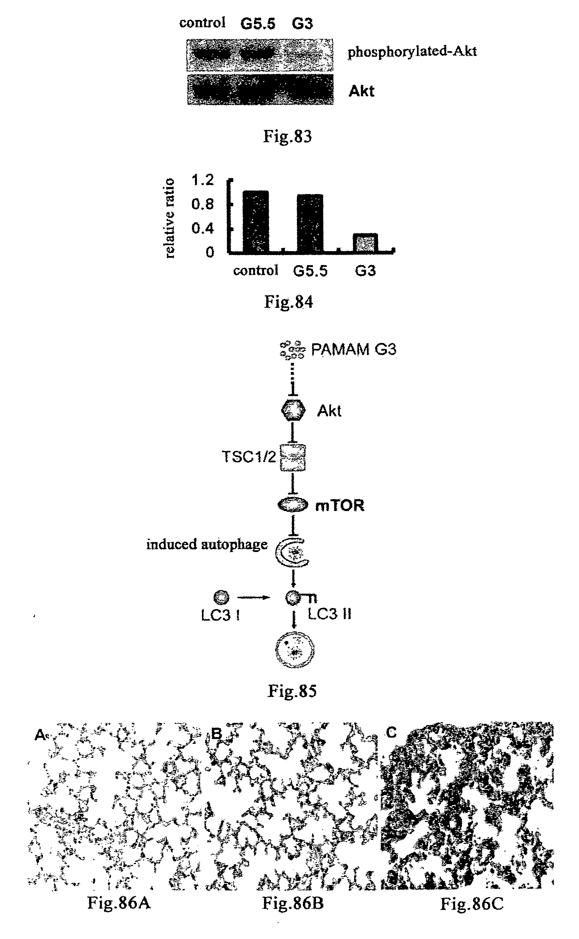

Figure 83 displays the results of Western Blotting and quantitative analysis of A549 cells treated in different ways. The first lane indicates a lysis sample of A549 cells treated with control for 24 hours, the second lane indicates a lysis sample of A549 cells treated with nanometer material PAMAM G5.5 (100µg/mL) for 24 hours, and the third lane indicates a lysis sample of A549 cells treated with nanometer material PAMAM G3 (100µg/mL) for 24 hours; the antibodies used were antibodies agonist phosphorylated Akt and total Akt, respectively (from top to bottom). It demonstrates that the expression level of phosphorylated Akt protein was significantly decreased, and Akt pathway was inhibited.
Figure 84 displays the relative ratio in bar graph by quantifying the densities of the bands obtained from the Western Blotting experiments shown in Figure 86 by using the software of Quantity one-4.6.3. They successively indicate the relative ratio of phosphorylated akt to akt protein in the Western Blotting experiments of A549 cells treated with negative control, G5.5 (100µg/mL), and G3 (100µg/mL), and the Control value was adjusted to 1. Values of other groups were divided by the Control value. It demonstrates that G3 can inhibit the activation of akt signal pathway.
Figure 85 displays diagram of cell signal pathway. From the above experimental results, we conclude as shown in the diagram: nanometer material PAMAM G3 can activate the pathway of Akt-TSC1/2-mTOR-autophagy. The activation of mTOR can inhibit the occurrence of Autophagy; and the activation of TSC1/2 can inhibit the activation of mTOR so as to enhance cell autophagy (type II cell apoptosis).
Figure 86 displays photograph (×200) of lung pathological sections of Balb/c mice. Fig. A indicates the photograph of pathological sections. The mice were injected with control via trachea, and 4 hours later, the lung was dissected and sliced into pathological sections. Fig.B indicates the photograph of pathological sections. The mice were injected with nanometer material PAMAM G5.5 (100µg/mL) via trachea, and 4 hours later, the lung was dissected and sliced. Fig. C indicates the photograph of pathological sections. The mice were injected with nanometer material PAMAM G3 (100µg/mL) via trachea, 4 hours later, the lung was dissected and sliced. It demonstrates that nanometer material PAMAM G3 leaded to the severe lung injury.

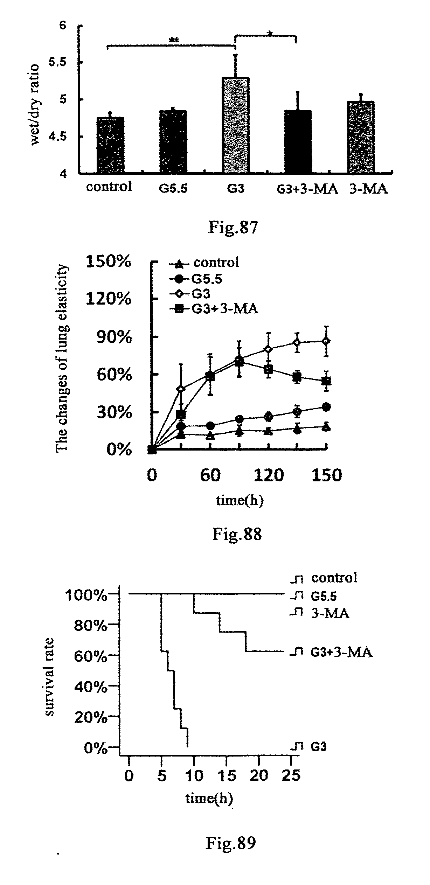

Figure 87 displays lung wet/dry ratio of Balb/c mice in bar graph. They indicate, from left to right, lung wet/dry ratio of mice 16 hours after the injection of control via trachea for, lung wet/dry ratio of mice 16 hours after the injection of nanometer material PAMAM G5.5 (100µg/mL) via trachea, lung wet/dry ratio of mice 16 hour after the injection of nanometer material PAMAM G3 (100µg/mL) via trachea, lung wet/dry ratio of mice 16 hour after the injection of nanometer material PAMAM G3 via trachea, and 1 hour before the injection of PAMAM G3 via trachea, mice were injected with 3-MA via intraperitoneal, and lung wet/dry ratio of mice 17 hours after the injection of 3-MA (15mg/kg) via intraperitoneal. Lung wet/dry ratio is one of important indicators of lung injury. The injection of nanometer material PAMAM G3 via trachea increased wet/dry ratio of lung tissue, whereas 3-MA relieved the the increased lung wet/dry ratio of mice induced by nanometer material PAMAM G3. The result proves that nanometer material PAMAM G3 leads to the severe lung injury, whereas 3-MA has some effect on ameliorating the lung injury induced by nanometer material PAMAM G3.
Figure 88 displays diagram of the changed lung elasticity of Balb/c mice. From left to right, it indicates successively the changed lung elasticity of Balb/c mice injected with control via trachea, the changed lung elasticity of mice injected with nanometer material PAMAM G5.5 (50mg/kg) via trachea, lung wet/dry ratio of mice injected with nanometer material PAMAM G3 (50mg/kg), the changed lung elasticity of mice injected with 3-MA (15mg/kg) via intraperitoneal, and 1h later injected with nanometer material PAMAM G3. Lung elasticity is one of important indicators of lung injury. The nanometer material PAMAM G3 leads to the changes of lung elasticity of mice, whereas 3-MA relieves the changes of lung elasticity of mice induced by nanometer material PAMAM G3. The result proves that nanometer material PAMAM G3 leads to the severe lung injury, whereas 3-MA has some effects on ameliorating the lung injury induced by nanometer material PAMAM G3.
Figure 89 displays survival curve of Balb/c mice. Balb/c mice were treated as follows: injected with control via trachea; injected with nanometer material PAMAM G5.5 (50mg/kg) via trachea; injected with nanometer material PAMAM G3 (50mg/kg) via trachea; injected with 3-MA (15mg/kg) via intraperitoneal injection firstly, and 1h later injected with nanometer material PAMAM G3 (50mg/kg) via trachea; and injected with 3-MA (15mg/kg) via intraperitoneal injection. The mice were observed for 24 hours continuously. Counted up the surviving conditions of mice every other hour, and conducted statistical analysis. Results: nanometer material PAMAM G3 increased the death rate of mice, whereas 3-MA can significantly relieve the death of mice induced by nanometer material PAMAM G3.

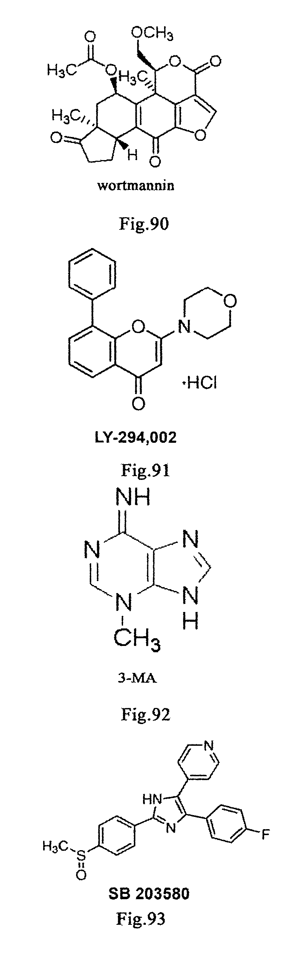

Figure 90 displays the structural formula of wortmannin. CAS No. is 19545-26-7, molecular formula is C23H24O8, and molecular weight is 428.43.
Figure 91 displays structural formula of LY-294,002. CAS No. is 934389-88-5, molecular formula is C19H17NO3 · HCl, and molecular weight is 343.80.
Figure 92 displays structure of 3-Methyladenine. CAS No. is 15142-23-4, molecular formula is C6H7N5, and molecular weight is 149.15.
Figure 93 displays the structure of SB 203580. CAS No. is 152121-47-6, molecular formula is C21H16FN3OS, and molecular weight is 377.43.

### Detailed Descriptions of the Embodiments

### Illustrations of the terms

Term "autophagic programmed cell death" in this invention is also called as type II programmed cell death (Autophagy). Such type of cell death is mainly characterized in the appearance of abundant vacuole enveloping cytoplasm and organelles in cytoplasm, and the degradation of components inside the vacuole via lysosome. Autophagy plays important roles in the growth, development and diseases of cells. Autophagy is mainly to eliminate damaged cell structure, aging organelles and unnecessary biomacromolecules and the like inside the degraded cells. Autophagy also simultaneously provides raw materials for the construction of organelles in the cells while digestion, namely the recycling of cell structure.

Term "SB203580" is also called as SB 203580 or SB-203580 by those skills in the art, which is a kind of common p38 MAPK inhibitor. SB203580 can pass through cells, inhibit p38 MAPK (p38 MAP kinase), and inhibit the activations of follow-up MAPKAP Kinase-2 and MAPKAP Kinase-3. SB203580 can effectively inhibit part of signal transduction induced by some inflammatory factor (such as IL-1β and TNF-α) through inhibiting p38 MAPK. SB203580 selectively inhibits p38 MAPK, IC50 is 600 nM; and it has no prominent inhibitory effect on JNK/SAPK and p44/42 MAPK (i.e. Erk1/2), IC50 is only 100 µM. Molecular weight of SB203580 is 377.43, molecular formula is C21H16FN3OS, and CAS Number is 152121-47-6. The purity of the product is generally more than 99%.

Term "3-MA" or "3MA" is the abbreviation of 3-methyladenine. 3-MA is the inhibitor of phosphotidylinositol 3 kinase, and it can specifically block the fusion of autophagic vacuoles and lysosome in Autophagy, and is widely used as the inhibitor of Autophagy (Per O Seglen and Paul B Gordon. (1982) 3-Methyladenine: specific inhibitor of autophagic/lysosomal protein degradation in isolated rat hepatocytes. Proc Natl Acad Sci USA 79(6): 1889-1892).

Term "LY294002", also called as LY-294,002, LY-294002 or LY 294002 by those skills in the art, is a kind of common protein kinase inhibitor which can block phosphotidylinsitol-3-Kinase cell signal transduction pathways. LY294002 can pass through cells, specifically inhibit PI3K, inhibit signaling pathway of PI3K/Akt, including commonly inhibiting phosphorylation of Akt. IC50 of LY294002 to purified PI3K is 1.4 µM. Molecular weight of LY294002 is 307.3, molecular formula is C19H17NO3, and CAS Number is 154447-36-6. The purity of the produce is generally more than 98%.

Bafilomycin A 1 may also be used as an inhibitor of Autophagy.

Acute respiratory distress syndrome (ARDS) is a clinical syndrome mainly characterized by alveolar capillary damage induced by the attack of intrapulmonary and extrapulmonary diseases such as severe infection, trauma, shock and the like, belongs to severe stage or type of acute lung injury (ALI). The clinical features of ARDS include breathing frequently and respiratory distress, progressive hypoxia, diffuse alveolar infiltration presented in X-ray.

### Example 1

Codons encoding H15 protein which are rarely employed in the expression host were substituted with condons which are frequently employed in the expression host by the method of gene optimization methods of codon optimization and humanization. The amino acid sequence corresponding to H5 protein of the wild type H5N1 virus (A/Thailand/4(SP-528)/2004(H5N1)) was searched via NCBI. The codons encoding the said amino acid sequence were substituted with those frequently employed in expression host, so as to obtain a number of optimized gene sequences encoding H5 protein. The sequences which may form complex secondary structure were removed by using the software of DNAMAN and the optimized H5 genes without the sequences encoding transmembrane segments were picked out. The genes were synthesized in Qingke Biological, Beijing, Co., LTD to be used as target genes for constructing plasmids.

Synthetic sequence is shown in SEQ ID NO: 1.

### Example 2

Construction and detection of plasmids (the experimental method refers to "Molecular Cloning", the third edition).

Vector: Peak13 CD5L TEV human IgG (stored in the inventor's laboratory).

Insertion of the fragment of optimized H5 gene without the transmembrane sequence.

After the processes of enzyme digestion, electrophoresis, purification, ligation and transformation as indicated in the Molecular Cloning, the inserted fragment was identified. The plasmids tested to be correct by the enzyme (Nhe I /BamH I) digestions were sequenced (Qingke Biological, Beijing, Co., LTD) for further confirmation.

The experimental results are shown in Figure 1. Lanes 1, 2 and 3 indicates λ-Hind III Marker, Peak13 CD5L H5 TEV human IgG, D2000 Marker, respectively. The size of λ-HindIII Marker is 564bp (difficult to be distinguished from the figure), 2027bp, 2322bp, 4361bp, 6557bp, 9416bp and 23130bp, in turn, from small to big (from bottom to top); The size of D2000 Marker is 100bp, 250bp, 500bp, 750bp, 1000bp and 2000bp, in turn, from small to big (from bottom to top). Plasmid was restricted by Nhe I/BamH I enzyme to obtain a 1.56Kb fragment, demonstrating that H5 gene has been inserted into the expression vector.

### Example 3 Maximal extraction of plasmid

A large amount of the recombined plasmids were extracted by the method of CsCl density gradient centrifugation (refers to "Molecular Cloning", the third edition).

### Example 4 Transfection

2×10⁵ cells were dispersed to each well of 6-well cell culture plate, and 24 hours later, the constructed recombinant plasmid containing H5 gene was transfected by using liposome (lipofectamine^{™} 2000 available from Invitrogen^{™}). The cultured cells were collected after incubated for a period of time. The molecular weight of fusion protein was determined by Western Blotting method.

### Example 5 Detection of the clanged expression levels of protein in A549 cells affected by inactivated H5N1 avian influenza virus via Western Blotting method

1) A549 cells in logarithmic growth phase were separated into a 6-well plate.
2) 24 hours later, the inactivated H5N1 avian influenza virus, with multiplicity of infection (M.O.I.) of 2, or chicken chorioallantoic fluid of same volume was added.
3) 1.5 hours later, lysed cells were collected. The change of LC3 was detected by Western Blotting method, and actin was used as an internal parameter of loading quantities. Or 4 hours later, lysed cells were collected, the changed expression levels of phosphorylated mTOR, phosphorylated S6, and phosphorylated Akt were detected by Western Blotting method, and corresponding total proteins were used as internal parameters of loading quantities.

The molecular weight of proteins was detected by the known Western Blotting method for those skills in the art. The semi-quantitative detection was also performed to determine the up- or down-regulations of some proteins (the specific experimental method of Western Blotting refers to "Molecular Cloning", the third edition).

Primary antibodies used in the experiments, anti-mTOR, anti-phospho-mTOR (Ser2481), anti-AKT, anti-phospho-AKT (Ser473) were all purchased from Cell Signaling Technology; The primary antibody, Anti-LC3B was purchased from Abcam; The primary antibody of Anti-TSC2, anti-ATG5 and anti-ATG12 were purchased from Santa Cruz Biotechnology; The primary antibody of Anti-β-actin was purchased from Sigma-Aldrich; Both the second antibody labeled with horse radish peroxidase (HRP) and the Western blotting Kit were puchased from Santa Cruz Biotechnology; 3-Methyladenine and rapamycin were purchased from Sigma-Aldrich.

Experimental results are shown in Figures 2, 20, 23, 25, 27, 31, 34, 41, 54, 68, 70, 72, 75, 77, 81 and 83.

Figure 2 displays the expression result of fusion protein H5Fc expressed in 293ET cell detected by Western Blotting. It demonstrates that fusion protein H5Fc was well expressed in host cell, and the molecular weight of the expressed protein was approximately 110KD and 60KD. H5 protein was cleaved by the enzyme inside the host so as to form two bands.

Figure 20 displays the results of Western Blot experiment of A549 cells treated in different ways. The first lane on the left was a lysis sample of A549 cells treated with negative control for 1.5 hours, and the second lane was a lysis sample of A549 cells treated with inactivated H5N1 avian influenza virus for 1.5 hours; The antibody was the antibody agonist LC3 and actin, respectively (from top to bottom). The relative expression level of LC3II was increased, demonstrating that the inactivated H5N1 avian influenza virus activates LC3 signal pathway and induces cell autophagy (type II cell apoptosis).

Figure 23 displays the result of Western Blotting experiments of A549 cells treated in different ways. The left lane was a lysis sample of A549 cells transfected with control siRNA and then treated with inactivated H5N1 avian influenza virus, and the right lane was a lysis sample of A549 cells transfected with control Atg12 siRNA and then treated with inactivated H5N1 avian influenza virus. The used antibody was the antibody agonist Atg12 and actin, respectively (from top to bottom). As Atg5 and Atg12 form complex inside the cell so as to induce cell autophagy (type II cell apoptosis), the detecting result was the relative amount of the complex of Atg5 and Atg12 to actin. It demonstrates that Atg12 siRNA effectively reduces the relative amount of complex of Atg5 and Atg12.

Figure 25 displays the result of Western Blotting experiments of A549 cells treated in different ways. The left lane was a lysis sample of A549 cells treated with negative control, and the right lane was a lysis sample of A549 cells treated with inactivated H5N1 avian influenza virus. The used antibody was the antibody agonist phosphorylated S6, S6 and actin, respectively (from top to bottom). S6 is the substrate of mTOR. The relative reduction of phosphorylated S6 indicated that the activity of mTOR pathway was inhibited, which demonstrates that inactivated H5N1 avian influenza virus inhibits mTOR signal pathway and further demonstrates that inactivated H5N1 avian influenza virus induces cell autophagy (type II cell apoptosis) via mTOR signal pathway.

Figure 27 displays the result of Western Blotting experiments of A549 cells treated in different ways. The left lane was a lysis sample of A549 cells treated with negative control, and the right lane was a lysis sample of A549 cells treated with inactivated H5N1 avian influenza virus. The used antibodies were antibodies agonist phosphorylated mTOR and mTOR, respectively (from top to bottom). The amount of phosphorylated mTOR was decreased relatively, suggesting that inactivated H5N1 avian influenza virus inhibits mTOR signal pathway and that inactivated H5N1 avian influenza virus induces cell autophagy (type II cell apoptosis) via inhibiting mTOR signal pathway.

Figure 31 displays the result of A549 cells treated in different ways in Western Blotting experiments. The samples of A549 cells transfected with control siRNA and TSC2 siRNA , respectively, were lysed 48h after the transfection; The antibodies used are antibodies agonist TSC2 and actin, respectively (from top to bottom). It demonstrates that TSC2 siRNA can significantly reduce the expression level of TSC2.

Figure 34 displays the result of Western Blotting experiments of A549 cells treated in different ways. The left lane was lysis sample of A549 cells treated with negative control, and the right lane was lysis sample of A549 cells treated with inactivated H5N1 avian influenza virus. The antibodies used were antibodies agonist phosphorylated Akt and Akt, respectively (from top to bottom). The reduction of the relative amount of phosphorylated Akt indicates that inactivated H5N1 avian influenza virus can inhibit Akt signal pathway.

Figure 41 displays the results of Western Blot of Balb/c mice lung tissue LC3. The first lane indicates the sample of total protein extracted from homogenized lung tissue from the mice injected with control (chicken chorioallantoic fluid) via trachea for 2 hours; the second lane indicates the sample of total protein extracted from homogenized lung tissue from the mice injected with inactivated H5N1 avian influenza virus via trachea for 2 hours, and the third lane indicates the sample of total protein extracted from homogenized lung tissue from the mice injected with 3-MA (30mg/kg) via intraperitoneal injection firstly, and 30 min later injected with inactivated H5N1 avian influenza virus via trachea for 2 hours. The antibodies used are antibodies agonist LC3 and β-actin. It demonstrates that the content of LC3 II in mice lung tissue was increased by the stimulation of the inactivated H5N1 avian influenza virus and the autophagy occurred, whereas 3-MA relieved the occurrence of autophagy.

Figure 54 displays the results of A549 cells treated in different ways in Western Blotting experiments. The first lane on the left indicates a lysis sample of A549 cells treated with adjuvant for 4 hours. The second lane indicates a lysis sample of A549 cells treated with inactivated H5N1 avian influenza virus for 4 hours. The antibodies used are antibodies agonist phosphorylated P38, P38 and actin, respectively (from top to bottom). It demonstrates that in the case that when the amount of samples was roughly the same (actin was used as internal parameter to ensure the same amount of samples), the expression level of phosphorylated P38 was significantly increased. Namely, inactivated H5N1 avian influenza virus can activate P38 signal pathway.

Figure 68 displays the results of Western Blotting experiments of A549 cells treated in different ways. The first lane on the left indicates a lysis sample of A549 cells treated with control for 4 hours, the second lane indicates a lysis sample of A549 cells treated with nanometer material PAMAM G5.5 (100µg/mL) for 4 hours, and the third lane indicates a lysis sample of A549 cells treated with nanometer material PAMAM G3 (100µg/mL) for 4 hours; The antibodies used were antibodies agonist LC3B and actin, respectively (from top to bottom). It demonstrates that the expression level of LC3B-II protein was significantly increased. Namely, nanometer material PAMAM G3 can induce cell autophagy.

Figure 70 displays the results of Western Blot experiments of A549 cells after different treatments. The samples were the lysis samples of A549 cells transfected with Control siRNA or with ATG6 siRNA, respectively, for 48 hours. The antibodies were antibodies agonist ATG6 and actin, respectively (from top to bottom). The results of Western Blotting experiments show that ATG6 siRNA effectively inhibits the expression of ATG6 gene.

Figure 72 displays the result of Western Blotting experiments of A549 cells treated in different ways. A549 cells were treated with different generations of nanometer materials (PAMAM) for 4 hours. Then the expression level of LC3-II in cells was detected through Western Blotting. It shows that nanometer materials PAMAM G4, G5, G6, G7, G8 all significantly increase the expression level of LC3-II in cells, suggesting that all of these nanometer materials can induce autophagy to cause cell death.

Figure 75 displays the result of Western Blotting experiments of A549 cells treated in different ways. The first lane indicates a lysis sample of A549 cells treated with control for 24 hours, the second lane indicates a lysis sample of A549 cells treated with nanometer material PAMAM G5.5 for 24 hours, and the third lane indicates a lysis sample of A549 cells treated with nanometer material PAMAM G3 for 24 hours; The antibodies used were antibodies agonist phosphorylated mTOR on position 2481 and total mTOR, respectively (from top to bottom). It demonstrates that the expression level of phosphorylated mTOR protein was significantly decreased. Namely, mTOR pathway was inhibited.

Figure 77 indicates the results of Western Blotting experiments of A549 cells treated in different ways. The first lane indicates a lysis sample of A549 cells treated with control for 24 hours, the second lane indicates a lysis sample of A549 cells treated with nanometer material PAMAM G5.5 (100µg/mL) for 24 hours, and the third lane indicates a lysis sample of A549 cells treated with nanometer material PAMAM G3 (100µg/mL) for 24 hours; The antibodies used were antibodies agonist phosphorylated S6 and total S6, respectively (from top to bottom). It demonstrates that the expression level of phosphorylated S6 protein was significantly decreased, and S6 pathway was inhibited. Namely, mTOR pathway was inhibited.

Figure 81 displays the results of Western Blotting experiments of A549 cells treated in different ways. A549 cells were transfected with control siRNA and TSC 2 siRNA firstly, and then treated with nanometer material PAMAM G3 (100µg/mL) for 24 hours, and the survival rate of cells were detected by MTT method. It shows that TSC2 siRNA effectively inhibits the expression of TSC2 gene.

Figure 83 displays the results of Western Blotting and quantitative analysis of A549 cells treated in different ways. The first lane indicates a lysis sample of A549 cells treated with control for 24 hours, the second lane indicates a lysis sample of A549 cells treated with nanometer material PAMAM G5.5 (100µg/mL) for 24 hours, and the third lane indicates a lysis sample of A549 cells treated with nanometer material PAMAM G3 (100µg/mL)for 24 hours; the antibodies used were antibodies agonist phosphorylated Akt and total Akt, respectively (from top to bottom). It demonstrates that the expression level of phosphorylated Akt protein was significantly decreased, and Akt pathway was inhibited.

### Example 6 Quantitative analysis of Western Blotting

1) Scanned results of Western Blotting.
2) Quantified the density of bands which need to be read in Western Blotting experiments by the software of Quantity one-4.6.3.
3) Calculated the ratio of bands which needs to be compared.
4) Adjusted the control value to 1 to obtain other relative value.

Experimental results are shown in Figures 21, 24, 26, 28, 32, 35, 42, 73, 76, 78 and 84.

Figure 21 displays the relative ratio by quantifying the densities of the bands obtained from the repeated Western Blotting experiments shown in Figure 22 by using the software of Quantity one-4.6.3. The left one indicates the relative ratio of LC3II to actin of A549 cells treated with negative control in the Western Blotting experiments, and the right one indicates the relative ratio of LC3II compared with actin of A549 cells treated with inactivated H5N1 avian influenza virus in the Western Blotting experiments. The value of the ratio was adjusted to 1. It demonstrates that inactivated H5N1 avian influenza virus activates LC3 signal pathway and induces cell autophagy (type II cell apoptosis).

Figure 24 displays the relative ratio in bar graph by quantifying the densities of the bands obtained from the repeated Western Blotting experiments shown in Figure 25 by using the software of Quantity one-4.6.3. The left one indicates the relative ratio of the complex of Atg5 and Atg12 to actin in A549 cells transfected with control siRNA, and the value was adjusted to 1. The right one indicates relative ratio of complex of Atg5 and Atg12 to actin in A549 cells transfected with control Atg12 siRNA. It demonstrates that Atg12 siRNA effectively reduces the relative amount of complex of Atg5 and Atg12.

Figure 26 displays the relative ratio in bar graph by quantifying the densities of the bands obtained from the repeated Western Blotting experiments by using the software of Quantity one-4.6.3. The left one indicates the relative ratio of phosphorylated S6 to S6 in A549 cells treated with negative control in the Western Blotting experiments, and the value was adjusted to 1. The right one indicates the relative ratio of phosphatized S6 to S6 in A549 cells treated with inactivated H5N1 avian influenza virus in the Western Blotting experiments. It demonstrates that inactivated H5N1 avian influenza virus inhibits mTOR signal pathway and further demonstrates that inactivated H5N1 avian influenza virus induces cell autophagy (type II cell apoptosis) via inhibiting mTOR signal pathway.

Figure 28 displays the relative ratio in bar graph by quantifying the densities of the bands obtained from the repeated Western Blotting experiments by using the software of Quantity one-4.6.3. The left one indicates the relative ratio of phosphorylated mTOR to mTOR of A549 cells treated with negative control in the Western Blotting experiments, and the value was adjusted to 1. The right one indicates the relative ratio of phosphorylated mTOR and to mTOR of A549 cells treated with inactivated H5N1 avian influenza virus in the Western Blotting experiments. It demonstrates that inactivated H5N1 avian influenza virus inhibits mTOR signal pathway and that inactivated H5N1 avian influenza virus induces cell autophagy (type II cell apoptosis) via inhibiting mTOR signal pathway.

Figure 32 displays the relative ratio in bar graph by quantifying the densities of the bands obtained from the repeated Western Blotting experiments shown in Figure 25 by using the software of Quantity one-4.6.3. The left one indicates relative ratio of TSC2 to actin in A549 cells transfected with control siRNA, and the value of the ratio was adjusted to 1. The right one indicates relative ratio of TSC2 to actin in A549 cells transfected with TSC2 siRNA. It demonstrates that TSC2 siRNA can significantly reduce the expression level of TSC2.

Figure 35 displays the relative ratio in bar graph by quantifying the densities of the bands obtained from the repeated Western Blotting experiments by using the software of Quantity one-4.6.3. The left one indicates the relative ratio of phosphorylated Akt to Akt in A549 cells treated with negative control in the Western Blotting experiments, and the value of the ratio was adjusted to 1. The right one indicaets the relative ratio of phosphorylated Akt to Akt in A549 cells treated with inactivated H5N1 avian influenza virus in the Western Blotting results. It demonstrates that inactivated H5N1 avian influenza virus can inhibit Akt signal pathway.

Figure 42 displays the relative ratio in bar graph by quantifying the densities of the bands obtained from the repeated Western Blotting experiments by using the software of Quantity one-4.6.3. The relative ratio of LC3 II to β-actin corresponds to the densities of bands shown in figure 42. It demonstrates that the content of LC3 II in mice lung tissue was increased by the stimulation of inactivated H5N1 avian influenza virus increases and autophagy occurred, whereas 3-MA relieved the occurrence of autophagy.

Figure 73 displays the relative ratio in bar graph by quantifying the densities of the bands obtained from the Western Blotting experiments shown in Figure 75 by using the software of Quantity one-4.6.3. They successively indicates the relative ratio of LC3II to actin in the Western Blotting experiment of A549 cells treated with negative control, G5.5, G4, G6, G7 and G8, and the Control value was adjusted to 1. The values of other groups were divided by the Control value. It demonstrates that G4, G5, G6, G7 and G8 can activate LC3 signal pathway and induce cell autophagy (type II cell apoptosis).

Figure 76 displays the relative ratio in bar graph by quantifying the densities of the bands obtained from the Western Blotting experiments shown in Figure 78 by using the software of Quantity one-4.6.3. They indicates successively the relative ratio of phosphorylated mTOR to mTOR protein in the Western Blotting experiments of A549 cells treated with negative control, G5.5 (100µg/mL), and G3 (100µg/mL). The Control value was adjusted to 1. Values of other groups were divided by the Control value. It demonstrates that G3 can inhibit mTOR signal pathway and induce cell autophagy (type II cell apoptosis).

Figure 78 displays the relative ratio in bar graph by quantifying the densities of the bands obtained from the Western Blotting experiments shown in Figure 80 by using the software of Quantity one-4.6.3. They successively indicate the relative ratio of phosphorylated S6 to S6 protein in the Western Blotting experiments of A549 cells treated with negative control, G5.5 (100µg/mL), and G3 (100µg/mL), and the Control value was adjusted to 1. Values of other groups were divided by the Control value. It demonstrates that G3 can inhibit the activation of S6, namely G3 can inhibit mTOR signal pathway, and induce cell autophagy (type II cell apoptosis).

Figure 84 displays the relative ratio in bar graph by quantifying the densities of the bands obtained from the Western Blotting experiments shown in Figure 86 by using the software of Quantity one-4.6.3. They successively indicate the relative ratio of phosphorylated akt to akt protein in the Western Blotting experiments of A549 cells treated with negative control, G5.5 (100µg/mL), and G3 (100µg/mL), and the Control value was adjusted to 1. Values of other groups were divided by the Control value. It demonstrates that G3 can inhibit the activation of akt signal pathway.

### Example 7 Establishment of constant expression cell strain

(1) Approximately 10µg recombinant plasmids were digested with a restriction endonuclease AvrII (purshased from NEW ENGLAND BioLabs® Inc,USA). Applied a small amount of enzyme-digested products to electrophoresis to determine the plasmid was completely digested. The remained enzyme-digested products were purified by Purification Kit (purchased from V-Gene), recovering the DNA and removing the enzyme and protein and the like therein.
(2) Cells were digested with trypsin, transferred into culture medium, and dispersed to single cell. 2×10⁵ cells were dispersed into to each well of 6-well cell culture plate.
(3) 24 hours later, water (negative control) and 0.5µg purified DNA were transfected into plasmid, respectively, by using the liposome (lipofectamineTM 2000, purchased from Invitrogen TM) according to the Manual provided by the kit.
(4) 48 hours later, cells were dispersed into 12-well cell culture plate (cells in each well of 6-well cell culture plate were dispersed equally to four wells of 12-well cell culture plate), drug (puromysin) with different concentrations were gradiently added for screening (purchased from CALBIOCHEM® CLONTECH), and cells without inserted DNAs were killed.
(5) 72 hours later, some wells of cells (corresponding to an appropriate concentration of drug), among which all negative control cells were killed and some cells transfected with DNA were still viable, were selected. Individual cell in wells was dispersed to 96-well cell culture plate by limited dilution method.
(6) About 10 days later, cell clones were picked up and was detected by ELISA and Western Blotting method.

The molecular weight was detected by Western Blotting method, so as to determine the correct expression of genes. The concentration of proteins was detected by ELISA kit, and the cell strains which highly expressed were selected.

### Example 8 Detection of the expression level of fusion proteins by ELISA method

The concentration of protein was detected by ELISA method. The agent used in ELISA was BD Pharmingen^{™} ELISA kit purchased from BD Biosciences.

### Example 9 Purification of proteins

H5Fc proteins were purified by Protein A protein column of Amersham. (Amersham Biosciences AB, Sweden; CAT NO: 17-04020-03)

Collected and cultured the constant expression cell strains.

Dialysis: dialyzed the collected culture medium. Components of the dislysate: 11.54mM/L Na2HPO4, 8.46mM/L NaH2PO4 (Beijing Chemical Factory, China), 1mM EDTA (Promega U.S.A), pH7.0. Time of dialysis was generally no less than 8 hours, and volume of dislysate was at least 20 times of that of supernatant.

Filtration: filtered the dialyzed liquid. The filter membrane used was 0.45µm Durapore membrane filters produced by Millipore (Millipore, Ireland; CAT NO.: HVLP04700).

Purification: steps of purification were performed according to the protocol in Product Instruction provided by Amersham, and the device used was Econo Gradient Pump Kits produced by Bio-Rad (Bio-Rad, U.S.A).

The purified protein sample was identified through the Western Blotting and SDS-polyacrylamide gel staining with Coomassie brilliant blue. The concentration of protein was determined by Lowry method (Lowry kit is available from Tianxiang Bonding Company CAT NO: TB090-1).

The H5Fc proteins resulted from purification were digested with TEV enzyme, and were passed through Protein A column again to get purified H5 protein.

The experimental result is shown in Figure 3.

Fig. 3 demonstrated that the well purified fusion protein H5Fc and H5 protein can be obtained. The purified H5Fc protein was restricted by TEV enzyme, and then preceded by affinity chromatography so as to obtain purified H5 protein with the molecular weight of approximately 80KD.

### Example 10 Preparation and observation of electron microscope specimens

### 1. The effect of inactivated avian influenza virus on inducing cell autophagy (type II cell apoptosis) of Hela cells and A549 cell.

1) Dispersed Hela cells and A549 cells in logarithmic growth phase onto 6 cm-plate, separately, the number of cells being no less than 2×10⁵.
2) 24 hours later, added inactivated avian influenza virus with Virus titer (MOI) of 10. The immunologic adjuvant with same volume was used as negative control.
3) 4 hours after affected by virus, digested and collected the cells into EP tube. Rested the cells for a moment and then centrifuged for 5 minutes at 800g.
4) Removed supernatant at best, and then added slowly along the wall of tube the new prepared 2.5% glutaraldehyde, and stored at 4 degree.
5) Repeatedly washed 3 times with phosphate buffer.
6) Fixed 1 hour with 1% osmic acid (OsO₄) solution.
7) Washed 3 times with ddH₂O.
8) Dehydrated with gradient acetone (50%, 70%, 80%, 90% and 100% once, 10 min for each time, and then treated with 100% acetone twice, 1 hour for each time).
9) Embedded and soaked for 2 hours with 812 resin and acetone embedding agent at 1:1, and then soaked for 4 hours with 100% 812 resin.
10) Polymerized for 12 hours at 37□, for 12 hours at 45°C, and for 24 hours at 60°C.
11) Repaired the block, located and then sliced sections.
12) Stained: stained for 5-10min with sodium ethoxide acetate, and stained for 10min with lead citrate,
13) Observed and photographed.
14) Randomly selected at least 100 cells for observing and counting. If there was no or only one typical autophgysome in cells, it was defined as autophagy negative; if there were more than two autophgysome in cells, it was defined as autophagy positive.

Experimental results are shown in Figures 4A, 4B, 4C, 5A, 5B, 5C, 6A, 6B, 6C, 9A, 9B and 9C.

Figure 4 displays the electron micrograph (×20000) of Hela cells treated in different ways and the percentage of autophagy cells (type II cell apoptosis) observed under electron microscope in bar graph. Fig. A indicates the electron micrograph of Hela cells treated with adjuvant for 4 hours. Fig. B indicates the electron micrograph of Hela cells treated with H5N1 avian influenza inactivated virus for 4 hours, the arrow indicates autophagosome. Fig. C indicates the percentage of autophagy cells (type II cell apoptosis) of Hela cells under the above two conditions in bar graph. It demonstrated that autophagy of Hela cells occurs under the effect of the inactivated H5N1 avian influenza virus.

Figure 5 displays the electron micrograph (×20000) of Hela cells treated in different ways and the percentage of autophagy cells (type II cell apoptosis) observed under electron microscope in bar graph. Fig. A indicates the electron micrograph of Hela cells treated with adjuvant for 4 hours. Fig. B indicates the electron micrograph of Hela cells treated with inactivated H5N2 avian influenza virus for 4 hours, the arrow indicates autophagosome. Fig. C indicates the percentage of autophagy cells (type II cell apoptosis) of Hela cells under the above two conditions in bar graph. It demonstrates that autophagy of Hela cells occurs under the effect of the inactivated H5N2 avian influenza virus.

Figure 6 displays the electron micrograph (×20000) of Hela cells treated in different ways and the percentage of autophagy cells (type II cell apoptosis) observed under electron microscope in bar graph. Fig. A indicates the electron micrograph of Hela cells treated with adjuvant for 4 hours. Fig. B indicates the electron micrograph of Hela cells treated with inactivated H9N2 avian influenza virus for 4 hours, the arrow indicates autophagosome. Fig. C indicates the percentage of autophagy cells (type II cell apoptosis) of Hela cells under the above two conditions in bar graph. It demonstrates that autophagy of Hela cells occurs under the effect of the inactivated H9N2 avian influenza virus.

Figure 9 displays the electron micrograph (×20000) of A549 cells treated in different ways and the percentage of autophagy cells (type II cell apoptosis) observed under electron microscope in bar graph. Fig. A indicates the electron micrograph of A549 cells treated with chicken chorioallantoic fluid for 4 hours. Fig. B indicates the electron micrograph of A549 cells treated with inactivated H5N1 avian influenza virus for 4 hours, the arrow indicates autophagosome. Fig. C indicates the percentage of cell autophagy (type II cell apoptosis) of A549 cells under the above two conditions in the bar graph. It demonstrated that autophagy of A549 cells occurred under the effect of inactivated H5N1 avian influenza virus.

### 2. Effect of recombinant H5 protein on inducing cell autophagy (type II cell apoptosis) of Hela cells and A549 cells.

1) Dispersed Hela cells and A549 cells in logarithmic growth phase onto 6cm-plate, separately, the number of cells being no less than 2×10⁵.
2) 24 hours later, added 0.5mM H5 proteins. BSA of the same amount was used as negative control.
3) After 4h treatment, digested and collected the cells into EP tube. Rested the cells for a moment and then centrifuged for 5 minutes at 800g.
4) Removed supernatant at best, and then added slowly along the wall of tube the new prepared 2.5% glutaraldehyde and stored at 4 degree.
5) Repeatedly washed 3 times with phosphate buffer.
6) Fixed for 1 hour with 1% osmic acid (OsO₄) solution.
7) Washed 3 times with ddH₂O.
8) Dehydrated with gradient acetone (50%, 70%, 80%, 90% and 100% once, 10min for each time, and treated with 100% acetone twice, 1 hour for each time).
9) Embedded and soaked for 2 hours with 812 resin and acetone embedding agent at 1:1, and then soaked for 4 hours with 100% 812 resin.
10) Polymerized for 12 hours at 370, for 12 hours at 45°C, and for 24 hours at 60°C.
11) Repaired the block, located and then sliced sections.
12) Stained: Stained for 5-10 min with sodium ethoxide acetate, and stained for 10min with lead citrate,
13) Observed and photographed.
14) Randomly selected at least 100 cells for observing and counting, if there was no or only one typical autophgysome in cells, it was defined as autophagy negative; if there were more than two autophgysome in cells, it was defined as autophagy positive.

Experimental results are shown in Figures 7A, 7B, 7C, 10A, 10B and 10C.

Figure 7 displays the electron micrograph (×20000) of Hela cells treated in different ways and the percentage of autophagy cells (type II cell apoptosis) observed under electron microscope in bar graph. Fig. A indicates the electron micrograph of Hela cells treated with BSA protein for 4 hours. Fig. B indicates the electron micrograph of Hela cells treated with H5 protein for 4 hours, the arrow indicates autophagosome. Fig. C indicates the percentage of autophagy cells (type II cell apoptosis) of Hela cells under the above two conditions in bar graph. It demonstrates that autophagy of Hela cells occurs under the effect of the H5 protein. Meanwhile it also demonstrated that the expressed H5 protein has biological activity.

Figure 10 displays the electron micrograph (×20000) of A549 cells treated in different ways and the percentage of autophagy cells (type II cell apoptosis) observed under electron microscope in bar graph. Fig. A indicates the electron micrograph of A549 cells treated with BSA for 4 hours. Fig. B indicates the electron micrograph of A549 cells treated with H5 protein for 4 hours , the arrow indicates autophagosome. Fig. C indicates the percentage of cell autophagy (type II cell apoptosis) of A549 cells under the above two conditions in the bar graph. It demonstrates that autophagy of A549 cells occurred under the effect of H5 protein, and it also demonstrated that the expressed H5 protein has biological activity.

### 3. Effect of positive control drug Rapamycin on inducing cell autophagy (type II cell apoptosis) of Hela cells and A549 cells.

1) Dispersed Hela cells and A549 cells in logarithmic growth phase onto 6cm-plate, separately, the number of cells being no less than 2×10⁵.
2) 24 hours later, added 5µM Rapamycin. DMSO of same amount was used as negative control.
3) After 4 h treatment, digested and collected cells into EP tube. Rested the cells for a moment and centrifuged the cells for 5 minutes at 800g.
4) Removed supernatant at best, and then added slowly along the wall of tube new prepared 2.5% glutaraldehyde, and stored at 4 degree.
5) Repeatedly washed 3 times with phosphate buffer.
6) Fixed for 1 hour with 1% osmic acid (OsO₄) solution.
7) Washed 3 times with ddH₂O.
8) Dehydrated with gradient acetone (50%, 70%, 80%, 90% and 100% once, 10min for each time, treated with 100% acetone twice, 1 hour for each time).
9) Embedded and soaked for 2 hours with 812 resin and acetone embedding agent at 1:1, and then soaked for 4 hours with 100% 812 resin.
10) Polymerized for 12 hours at 370, for 12 hours at 45°C, and for 24 hours at 60°C.
11) Repaired the block, located and then sliced sections.
12) Staiend: stained for 5-10min with sodium ethoxide acetate, and stained for 10min with lead citrate,
13) Observed and photographed.
14) Randomly selected at least 100 cells for observing and counting, if there was no or only one typical autophgysome in cells, it was defined as autophagy negative; if there were more than two autophgysome in cells, it was defined as autophagy positive.

Experimental results are shown in Figures 8A, 8B, 8C, 11A, 11B and 11C.

Figure 8 displays the electron micrograph (x20000) of Hela cells treated in different ways and the percentage of autophagy cells (type II cell apoptosis) observed under electron microscope in bar graph. Fig. A indicates the electron micrograph of Hela cells treated with DMSO for 4 hours. Fig. B indicates the electron micrograph of Hela cells treated with 5µM Rapamycin for 4 hours, the arrow indicates autophagosome. Fig. C indicates the percentage of autophagy cells (type II cell apoptosis) of Hela cells under the above two conditions in bar graph. It can be known from literatures that Rapamycin results in the occurrence of cell autophagy. Thus, the method of the experiment is proven to be accurate and effective, considering Rapamycin as a positive control of the experiment.

Figure 11 displays the electron micrograph (×20000) of A549 cells treated in different ways and the percentage of autophagy cells (type II cell apoptosis) observed under electron microscope in bar graph. Fig. A indicates the electron micrograph of A549 cells treated with DMSO for 4 hours. Fig. B indicates the electron micrograph of A549 cells treated with 5uM Rapamycin for 4 hours, the arrow indicates autophagosome. Fig. C indicates the percentage of cell autophagy (type II cell apoptosis) of A549 cells under the above two conditions in the bar graph. It can be known from literatures that Rapamycin results in the occurrence of cell autophagy. Thus, the method of the experiment is proven to be accurate and effective, considering Rapamycin as a positive control of the experiment.

### 4. Detection of preventing and treating effect of drug on cell autophagy (type II cell apoptosis) induced by avian influenza virus.

1) Dispersed A549 cells in logarithmic growth phase to 6cm-plate, separately, the number of cells are not less than 2×10⁵.
2) 24 hours later, added drug SB203580 and treated for 1 hour.
3) Added inactivated H5N1 avian influenza virus with Virus titer MOI of 10, chicken chorioallantoic fluid with the same volume was used as negative control.
4) 4 hours after affected by virus, digested and collected cells into EP tube. Rested the cells for a moment and then centrifuged for 5 minutes at 800g.
5) Removed supernatant at best, and then added slowly along the wall of tube the new prepared 2.5% glutaraldehyde, stored at 4 degree.
6) Repeatedly washed 3 times with phosphate buffer.
7) Fixed for 1 hour with 1% osmic acid (OsO₄) solution
8) Washed 3 times with ddH₂O.
9) Dehydrated with gradient acetone (50%, 70%, 80%, 90% and 100% once, 10min for each time, treated with 100% acetone twice, 1 hour for each time).
10) Embedded and soaked for 2 hours with 812 resin and acetone embedding agent at 1:1, and then soaked for 4 hours with 100% of 812 resin.
11) Polymerized for 12 hours at 37□, for 12 hours at 45°C, and for 24 hours at 60°C .
12) Repaired the block, located and then sliced sections.
13) Stained: stained for 5-10min with sodium ethoxide acetate, and stained for 10min with lead citrate,
14) Observed and photographed.
15) Randomly selected at least 100 cells for observing and counting, if there was no or only one typical autophgysome in cells, it was defined as autophagy negative; if there were more than two autophgysome in cells, it was defined as autophagy positive.

Experimental results are shown in Figures 52A, 52B, 52C and 53.

Figure 52 displays electron micrograph (×20000) of A549 cells treated in different ways. The first photograph of Fig. A indicates the electron micrograph of A549 cells treated with adjuvant for 4 hours. Fig.B indicates the electron micrograph of A549 cells treated with Inactivated H5N1 avian influenza virus for 4 hours, and Fig. C indicates the electron micrograph of A549 cells pretreated with SB203580, a specific inhibitor of P38 pathway for 1 hour firstly, and then treated with inactivated H5N1 avian influenza virus for 4 hours. It demonstrates that autophagy of A549 cells occurred under the effect of inactivated H5N1 avian influenza virus, whereas SB203580, a specific inhibitor of P38 pathway reduced cell autophagy.

Figure 53 displays percentage of A549 cells with autophagy (type II cell apoptosis) after various treatments in bar graph under electron microscope. The first graph on the left indicates percentage of A549 cells with autophagy (type II cell apoptosis) after treatment with adjuvant for 4 hours, the second graph indicates the percentage of A549 cells with autophagy (type II cell apoptosis) after treatment with inactivated H5N1 avian influenza virus for 4 hours, the third graph indicates the percentage of A549 cells with autophagy (type II cell apoptosis) after pretreatment with SB203580, a specific inhibitor of P38 pathway for 1 hour, and then with inactivated H5N1 avian influenza virus for 4 hours. Results show that the ratio of A549 cells with autophagy after the treatment only with inactivated H5N1 avian influenza virus is 24.3%. By pretreated with SB203580, an inhibitor of P38 pathway prior to the treatment with inactivated H5N1 avian influenza virus, the ratio of A549 cells with autophagy is 7.73%. There is significant difference between the two cases. The result proves that SB203580, the inhibitor of P38 pathway can effectively inhibit the occurrence of autophagy induced by inactivated H5N1 avian influenza virus.

### 4. Detection of preventing and treating effect of drug on cell autophagy (type II cell apoptosis) induced by nanometer materials.

1) Dispersed A549 cells in logarithmic growth phase to 6cm-plate, separately, the number of cells are not less than 2×10⁵.
2) 24 hours later, added drug 3-MA and treated for 1 hour.
3) Added nanometer materials PAMAM G3 (100µg/mL), PAMAM G5.5 (100µg/mL) and solvent PBS serving as negative control.
4) After being affected for 24 hours, digested and collected cells into EP tube. Rested the cells for a moment and centrifuged for 5 minutes at 800g.
5) Removed supernatant at best, and then added slowly along the wall of tube new prepared 2.5% glutaraldehyde, stored at 4 degree.
6) Repeatedly washed 3 times with phosphate buffer.
7) Fixed for 1 hour with 1% osmic acid (OsO₄) solution
8) Washed 3 times with ddH₂O.
9) Dehydrated with gradient acetone (50%, 70%, 80%, 90% and 100% once, 10min for each time, treated with 100% acetone twice, 1 hour for each time).
10) Embedded and soaked for 2 hours with 812 resin and acetone embedding agent at 1:1, and then soaked for 4 hours with 100% of 812 resin.
11) Polymerized for 12 hours at 37□, for 12 hours at 45°C, and for 24 hours at 60 °C .
12) Repaired the block, located and then sliced sections.
13) Stained: staiend for 5-10min with sodium ethoxide acetate, and stained for 10min with lead citrate,
14) Observed and photographed.
15) Randomly selected at least 100 cells for observing and counting, if there was no or only one typical autophgysome in cells, it wass defined as autophagy negative; if there were more than two autophgysome in cells, wat is defined as autophagy positive.

Experimental results are shown in Figures 64A, 64B, 64C, 64D and 65.

Figure 64 displays electron micrograph (×20000) of A549 cells treated in different ways. Fig. A indicates the electron micrograph of A549 cells treated with control for 24 hours. Fig.B indicates the electron micrograph of A549 cells treated with nanometer material PAMAM G5.5 (100µg/mL) for 24 hours. Fig. C indicates the electron micrograph of A549 cells treated with nanometer material PAMAM G3 (100µg/mL) for 24 hours. Fig. D indicates the electron micrograph of A549 cells firstly treated with 3-MA (10mM) for 1 hour, and then treated with nanometer material PAMAM G3 (100µg/mL) for 24 hours. It demonstrates that autophagy of A549 cells occurred under the effect of nanometer material PAMAM G5, and the cell death induced by cell autophagy can be relieved by 3-MA.

Figure 65 displays the percentage of A549 cells with autophagy (type II cell apoptosis) in bar graph. Typical characterization after occurrence of cell autophagy is the appearance of autophagosome. The percentage of cells having more than or equal to 2 autophagosomes per hundred random cells was calculated. It is obvious that 3-MA reduced the percentage of cells with autophagy (type II cell apoptosis) induced by nanometer material PAMAM G3. Namely 3-MA can treat or relieve cell autophagy (type II cell apoptosis).

### 5. Effect of inactivated avian influenza virus on inducing cell autophagy (type II cell apoptosis) of lung epithelial cells of mice.

1) Selected Balb/c mice of 4-6 weeks old, randomly grouped. Anesthetized mice with 1% sodium pentobarbital.
2) Performed the operation of trachea cannula of mice.
3) Perfused mice with chicken chorioallantoic fluid or inactivated H5N1 avian influenza virus via trachea.
4) After affected by virus for 2 hours, killed the mice and dissected lung.
5) Washed with PBS. Added the new prepared 2.5% glutaraldehyde immediately into lung and stored the lung.
6) Repeatedly washed 3 times with phosphate buffer.
7) Fixed for 1 hour with 1% osmic acid (OsO₄) solution
8) Washed 3 times with ddH₂O.
9) Dehydrated with gradient acetone (50%, 70%, 80%, 90% and 100% once, 10 min for each time, treated with 100% acetone twice, 1 hour for each time).
10)Embedded and soaked for 2 hours with 812 resin and acetone embedding agent at 1:1, and then soaked for 4 hours with 100% 812 resin.
11) Polymerized for 12 hours at 37□, for 12 hours at 45°C, and for 24 hours at 60°C.
12) Repaired the block, located and then sliced sectioning.
13) Stained: stained for 5-10 min with sodium ethoxide acetate, and stained for 10 min with lead citrate,
14) Observed and photographed.
15) Randomly selected at least 100 cells for observing and counting, if there was no or only one typical autophgysome in cells, it was defined as autophagy negative; if there were more than two autophgysome in cells, it was defined as autophagy positive.

Experimental results are shown in Figures 36A, 36B, 36C and 36D.

Figure 36 displays the electron micrographs of lung tissue of mice perfused with chicken chorioallantoic fluid or inactivated H5N1 avian influenza virus. Fig. A indicates electron micrograph of lung tissue perfused with chicken chorioallantoic fluid; Fig. B indicates partially enlarged view of the position shown in white box of A. A more complete cell was observed, however, no autophagosome was observed; Fig. C indicates electron micrograph of lung tissue perfused with inactivated H5N1 avian influenza virus; Figure D indicates partially enlarged view of the position shown in white box of A. A more complete cell was observed. An autophagosome in the cell was observed (represented by arrow). It demonstrates that inactivated H5N1 avian influenza virus can induce cell autophagy (type II cell apoptosis) of lung tissue.

### Example 11 Preparation and observation of laser scanning confocal microscope specimens

### 1. LC3 aggregation induced by Avian influenza virus

Hela cells or A549 cells were treated with inactivated H5N1, H5N2, H9N2 avian influenza virus, H5 protein and positive drug Rapamycin for 4 hours. The change of LC3 aggregation was observed.
1) Placed a disinfected cover-glass to 24-well cell culture plate, and then implanted Hela cells or A549 cells in logarithmic growth phase.
2) 24 hours later, transfected EGFP-LC3 plasmid with liposome.
3) 72 hours later, added inactivated H5N1, H5N2, H9N2 avian influenza virus, H5 protein and the corresponding negative control and positive control (5µM Rapamycin).
4) After incubated for 4 hours at 37°C, fixed cells with 4% paraformaldehyde, taked out the cover-glass and put it on the glass slide dropped with sealing agent. The cells were observed under laser scanning confocal microscope (Leica TCS PS2).
5) Randomly selected 100 cells to observe. The cells with more than or equal to 20 green bright spots were defined as positive, and those with less than 20 green bright spots were defined as negative.

Experimental results are shown in Figures 12, 13, 14, 16, 17, 18 and 19.

Figure 12 displays photographs of Hela cells transfected with EGFP-LC3 plasmid and treated in different ways under laser scanning confocal microscope (×1000) and the percentage of cells with EGFP-LC3 agglutination in the bar graph. Fig. A indicates the photograph of Hela cells treated with adjuvant for 4 hours under the laser scanning confocal microscope. Fig. B indicates the photograph of Hela cells treated with inactivated H5N1 avian influenza virus for 4 hours under laser scanning confocal microscope. Fig. C indicates the percentage of Hela cells with EGFP-LC3 agglutination under the above two conditions in the bar graph. Typical characterization after the occurrence of cell autophagy is the agglutination of LC3 (ATG8) molecular so as to form autophagysome. If the autophagy happens in cells, LC3 molecular labeled with EGFP will aggregate, and strongly emitted green fluorescence can be observed under confocal microscope; While for the cells without autophagy, the green fluorescence dispersed or only a little agglutinated. It demonstrates that autophagy of Hela cells occurred under the effect of inactivated H5N1 avian influenza virus.

Figure 13 displays photographs of Hela cells transfected with EGFP-LC3 plasmid and treated in different ways under laser scanning confocal microscope (×1000) and the percentage of cells with EGFP-LC3 agglutination in bar graph. Fig. A indicates photograph of Hela cells treated with adjuvant for 4 hours under the laser scanning confocal microscope. Fig. B indicates the photographs of Hela cells treated with inactivated H5N2 avian influenza virus for 4 hours under laser scanning confocal microscope, and Fig. C indicates the percentage of Hela cells with EGFP-LC3 agglutination under the above two conditions in the bar graph. It demonstrated that autophagy of Hela cells occurred under the effect of inactivated H5N2 avian influenza virus.

Figure 14 displays the photographs (×1000) of Hela cells transfected with EGFP-LC3 plasmid and treated in different ways under laser scanning confocal microscope and the percentage of cells with EGFP-LC3 agglutination in bar graph. Fig. A indicates photograph of Hela cells treated with adjuvant for 4 hours under the laser scanning confocal microscope. Fig.B indicates photographs of Hela cells treated with inactivated H9N2 avian influenza virus for 4 hours under laser scanning confocal microscope. Fig. C indicates the percentage of cells with EGFP-LC3 agglutination under the above two conditions in the bar graph. It demonstrates that autophagy of Hela cells occurred under the effect of inactivated H9N2 avian influenza virus.

Figure 16 displays photographs (×1000) of Hela cells transfected with EGFP-LC3 plasmid and then treated in different ways under laser scanning confocal microscope and the percentage of the cells with EGFP-LC3 agglutination in bar graph. Fig. A indicates photograph of Hela cells treated with DMSO for 4 hours provided by laser confocal microscope. Fig.B indicates photographs of Hela cells treated with Rapamycin for 4 hours provided by laser confocal microscope. Fig. C indicates the percentage of Hela cells with EGFP-LC3 agglutination under the above two conditions in bar graph. It is known from literatures that Rapamycin induces the occurrence of cell autophagy. Thus, the method of the experiment is proven to be accurate and effective, considering Rapamycin as a positive control of the experiment.

Figure 17 displays photographs (×1000) of A549 cells transfected with EGFP-LC3 plasmid and then treated in different ways under laser scanning confocal microscope and the percentage of the Hela cells with EGFP-LC3 agglutination in bar graph. Fig. A indicates photograph of A549 cells treated with negative control, chicken chorioallantoic fluid for 4 hours provided by laser confocal microscope. Fig. B indicates photographs of A549 cells treated with inactivated H5N1 avian influenza virus for 4 hours provided by laser confocal microscope. Fig. C indicates the percentage of A549 cells with EGFP-LC3 agglutination under the above two conditions in bar graph. It demonstrates that autophagy of A549 cells occurred under the effect of inactivated H5N1 avian influenza virus.

Figure 18 displays photographs (×1000) of A549 cells transfected with EGFP-LC3 plasmid and then treated in different ways under laser scanning confocal microscope and the percentage of Hela cells with EGFP-LC3 agglutination in bar graph. Fig. A indicates photograph of A549 cells treated with BSA for 4 hours provided by laser confocal microscope. Fig. B indicates photograph of A549 cells treated with H5 protein for 4 hours provided by laser confocal microscope. Fig. C indicates the percentage of A549 cells with EGFP-LC3 agglutination under the above two conditions in bar graph. It demonstrates that autophagy of A549 cells occurred under the effect of H5 protein, and it also demonstrated that the expressed H5 protein has biological activity.

Figure 19 displays photographs (×1000) of A549 cells transfected with EGFP-LC3 plasmid and then treated in different ways under laser scanning confocal microscope and the percentage of the Hela cells with EGFP-LC3 agglutination in bar graph. Fig. A indicates photograph of A549 cells treated with DMSO for 4 hours provided by laser confocal microscope. Fig.B indicates photographs of A549 cells treated with Rapamycin for 4 hours provided by laser confocal microscope. Fig. C indicates the percentage of A549 cells with EGFP-LC3 agglutination under the above two conditions in bar graph. It is known from literatures that Rapamycin induces the occurrence of cell autophagy. Thus, the method of the experiment is proven to be accurate and effective, considering Rapamycin as a positive control of the experiment.

### 2. Effect of TSC2 siRNA on inhibiting LC3 aggregation induced by avian influenza virus or nanometer material PAMAM.

1) Placed a disinfected cover-glass to 24-well cell culture plate, and then implanted Hela cells or A549 cells in logarithmic growth phase.
2) 24 hours later, transfected EGFP-LC3 plasmid, TSC2 siRNA and control siRNA with liposome.
3) 48 hours later, added inactivated H5N1 avian influenza virus or nanometer material PAMAM G3 (100µg/mL).
4) After incubated for 4 hours (inactivated avian influenza virus) or for 24 hours (material PAMAM G3) at 37°C, fixed the cells with 4% paraformaldehyde, take out the cover-glass and put it on the glass slide dropped with sealing agent. The cells were observed with laser scanning confocal microscope (Leica TCS PS2).
5) Randomly selected 100 cells for observing. The cells with more than or equal to 20 green bright spots were defined as positive, and those with less than 20 green bright spots were defined as negative.

Experimental results are shown in Figures 29A, 29B, 30, 79A, 79B and 80.

Figure 29 displays photographs (×1000) of A549 cells transfected with EGFP-LC3 plasmid and then treated in different ways under laser scanning confocal microscope. Fig. A indicates photograph of A549 cells transfected with negative control siRNA and then treated with inactivated H5N1 avian influenza virus provided by laser confocal microscope. Fig. B indicates photographs of A549 cells transfected with TSC2 siRNA and then treated with inactivated H5N1 avian influenza virus provided by laser confocal microscope. After transfection of TSC2 siRNA, cells with EGFP-LC3 agglutination was decreased, suggesting that TSC2 siRNA inhibits cell autophagy (type II cell apoptosis) induced by inactivated H5N1 avian influenza virus.

Figure 30 displays the percentage of cells with EGFP-LC3 agglutination among the A549 cells transfected with EGFP-LC3 plasmid and then treated in different ways in bar graph. The left one indicates percentage of the cells with EGFP-LC3 agglutination among the A549 cells transfected with negative control siRNA and then treated with inactivated H5N1 avian influenza virus, and the right one indicates the percentage of cells with EGFP-LC3 agglutination among A549 cells transfected with TSC2 siRNA and then treated with inactivated H5N1 avian influenza virus. It demonstrates that TSC2 siRNA inhibits cell autophagy (type II cell apoptosis) induced by inactivated H5N1 avian influenza virus.

Figure 79 displays photograph (×1000) of A549 cells transfected with EGFP-LC3 plasmid and then treated in different ways provided by laser scanning confocal microscope. Fig. A indicates photograph of cells treated with Control siRNA for 24 hours, and then treated with nanometer material PAMAM G3 (100µg/mL) provided by laser confocal microscope. Fig.B indicates photograph of cells treated with TSC2 siRNA for 24 hours, and then treated with nanometer material PAMAM G3 (100µg/mL) provided by laser confocal microscope.

Figure 80 displays the percentage of cells with EGFP-LC3 agglutination after the A549 cells transfected with EGFP-LC3 plasmid were treated in different ways. Fig. A indicates the percentage of cells with EGFP-LC3 agglutination after A549 cells transfected with negative control siRNA and then treated with PAMAM G3 (100µg/mL), and Fig.B indicates the percentage of cells with EGFP-LC3 agglutination after A549 cells transfected with TSC2 siRNA and then treated with PAMAM G3 (100µg/mL). It demonstrates that TSC2 siRNA can inhibit cell autophagy (type II cell apoptosis) induced by PAMAM G3 (100µg/mL).

### 3. Effect of 3-MA on inhibiting LC3 aggregation induced by nanometer material PAMAM.

1) Placed a disinfected cover-glass to 24-well cell culture plate, and then implanted Hela cells or A549 cells in logarithmic growth phase.
2) 24 hours later, transfected EGFP-LC3 plasmid, TSC2 siRNA and control siRNA with liposome.
3) 48 hours later, added negative control, nanometer material PAMAM G3 (100µg/mL), nanometer material PAMAM G5.5 (100µg/mL) and added nanometer material PAMAM G3 (100µg/mL) after pretreated with 3-MA (10mM) for 1 hour.
4) After incubated for 4 hours at 37°C, fixed cells with 4% paraformaldehyde, took out the cover-glass and put it on the glass slide dropped with sealing agent. The cells were observed with laser scanning confocal microscope (Leica TCS PS2).
5) Randomly selected 100 cells to observe. The cells with more than or equal to 20 green bright spots were defined as positive, and those with less than 20 green bright spots were defined as negative.

Experimental results are shown in Figures 66A, 66B, 66C, 66D and 67.

Figure 66 displays photographs (×1000) of A549 cells transfected with EGFP-LC3 plasmid and then treated in different ways under laser scanning confocal microscope. Fig. A indicates photographs of A549 cells transfected with EGFP-LC3 plasmid and then treated with control for 24 hours provided by laser confocal microscope. Fig.B indicates photograph of A549 cells transfected with EGFP-LC3 plasmid and then treated with nanometer material PAMAM G5.5 (100µg/mL) for 24 hours provided by laser confocal microscope. Fig. C indicates photograph of A549 cells transfected with EGFP-LC3 plasmid and then treated with nanometer material PAMAM G3 (100µg/mL) for 24 hours provided by laser confocal microscope. Fig. D indicates photograph of A549 cells transfected with EGFP-LC3 plasmid and treated with 3-MA (10mM) firstly for 1 hour, and then treated with nanometer material PAMAM G3 (100µg/mL) for 24 hours provided by laser confocal microscope.

Figure 67 displays the percentage of cells with EGFP-LC3 agglutination under the above conditions in bar graph. Typical characterization for the occurrence of cell autophagy is the agglutination of LC3 (ATG8) molecular so as to form autophagysome. LC3 molecular was labeled with EGFP. If the cell autophagy occurs, LC3 molecular will aggregate, and strongly emitting green fluorescence can be observed under confocal microscope. However, if the cells autophagy does not occur, green fluorescence disperses or only a little agglutinates. It demonstrates that of the autophagy of A549 cells occurred under the effect of nanometer material PAMAM G3, and such cell death induced by cell autophagy can be relieved by 3-MA.

### Example 12 Synthesis of siRNA

SiRNAs used in the experimental results were synthesized by RiBo Biotechnology.

Sequences of siRNA used in the experimental results as indicated in Figures 29, 30, 31,32 and 33 are as follows:
TSC2 siRNA: 5' GGGACAUUCUGCUGAACAU dTdT 3'/3' dTdT CCCUGUAAGACGACUUGUA 5' (SEQ ID NO.: 2, 3)

Sequences of siRNAs used in the experimental results as indicted in Figures 46, 47 and 48 are as follows:
Atg5 siRNA: 5' ACCGGAAACUCAUGGAAUA dTdT 3'/3' dTdT UGGCCUUUGAGUACCUUAU 5' (SEQ ID NO.: 4, 5)

Sequences of siRNA used in the experimental results as indicated in Figures 70 and 71 are as follows:
Atg6 siRNA: 5' CAGUUUGGCACAAUCAAUA dTdT 3'/3' dTdT GUCAAACCGUGUUAGUUAU 5' (SEQ ID NO.: 6, 7)

Sequences of siRNA used in the experimental results as indicated in Figures 22, 23 and 24 are as follows:
ATG12 siRNA: 5' GCAGUAGAGCGAACACGAA dTdT 3'/3' dTdT CGUCAUCUCGCUUGUGCUU 5'(SEQ ID NO.: 8,9)

Sequences of siRNA used in the experimental results as indicated in Figures 81 and 82 are as follows:
TSC2 siRNA: 5' CCAUCAAGGGCCAGUUCAA dTdT 3'/3' dTdT GGUAGUUCCCGGUCAAGUU 5'(SEQ ID NO.: 10, 11)

SiRNAs used in the experimental results of Figures 81 and 82 were purchased from Santa Cruz Biotechnology.

### Example 13 Disturbance of siRNA on gene expression of cells

A549 cells were transfected with siRNA targeted on different genes to disturb the expression of the corresponding genes.
1) Dispersed A549 cells in logarithmic growth phase onto 24-well plate.
2) 24 hours later, transfected A549 cells with siRNA (50nM).
3) 48 hours later, treated A549 cells with inactivated H5N1 avian influenza virus or nanometer materials.
4) 2 to 8 hours later, lysed cells and detected the lysis by Western Blotting.

Experimental results are shown in Figures 23, 31, 70 and 81.

Figure 23 displays the result of Western Blotting experiments of A549 cells treated in different ways. The left lane was a lysis sample of A549 cells transfected with control siRNA and then treated with inactivated H5N1 avian influenza virus, and the right lane was a lysis sample of A549 cells transfected with control Atg12 siRNA and then treated with inactivated H5N1 avian influenza virus. The antibody used was the antibody agonist Atg12 and actin, respectively (from top to bottom). As Atg5 and Atg12 form complex inside the cell so as to induce cell autophagy (type II cell apoptosis), the detecting result was the relative amount of the complex of Atg5 and Atg12 to actin. It demonstrates that Atg12 siRNA effectively reduces the relative amount of complex of Atg5 and Atg12.

Figure 31 displays the result of A549 cells treated in different ways in Western Blotting experiments. The samples of A549 cells transfected with control siRNA and TSC2 siRNA , respectively, were lysed 48h after the transfection; The antibodies used are antibodies agonist TSC2 and actin, respectively (from top to bottom). It demonstrates that TSC2 siRNA can significantly reduce the expression level of TSC2.

Figure 70 displays the results of Western Blot experiments of A549 cells after different treatments. The samples were the lysis samples of A549 cells transfected with Control siRNA or with ATG6 siRNA, respectively, for 48 hours. The antibodies were antibodies agonist ATG6 and actin, respectively (from top to bottom). The results of Western Blotting experiments show that ATG6 siRNA effectively inhibits the expression of ATG6 gene.

Figure 81 displays the results of Western Blotting experiments of A549 cells treated in different ways. A549 cells were transfected with control siRNA and TSC 2 siRNA firstly, and then treated with nanometer material PAMAM G3 (100µg/mL) for 24 hours, and the survival rate of cells were detected by MTT method. It shows that TSC2 siRNA effectively inhibits the expression of TSC2 gene.

### Example 14 Real Time-PCR

1) Extracted total RNA with Trizol (vitrogen) according to the instructions, and determined concentration of RNA and ratio of OD280/OD260.
2) After identifying that RNA was complete by electrophoresis, took 10µg RNA for reverse transcription (ABI reverse transcription kit) so as to generate single-stranded DNA.
3) Amplified with BIO-RAD IQ5 Real Time-PCR
4) Amplification conditions were as follows: pre-denaturing for 10 min at 95□, denaturing for 15s at 95□, annealing and extending for 1min at 60□, totally 40 recycles.
5) After the reaction, obtained Ct values of Atg5 and β-actin of each specimen automatically from the computer, β-actin was used as reference. □Ct=Ct(Atg5)-Ct(β-actin), □□Ct=□Ct(experimental group) - □Ct (blank group), 2^{-ΔΔCt} represents the relative amount of Atg5 gene.

### Primers:

Forward primer of Atg5 is: 5'- CAGATGGACAGCTGCACACACT -3', (SEQ ID NO. :12)
Reverse primer is : 5'- GGCTCTATCCCGTGAATCATCA -3', (SEQ ID NO.: 13)
β-actin forward primer is: 5'- AGTGTGACGTTGACATCCGTA -3', (SEQ ID NO.: 14)
Reverse primer is: 5'- GCCAGAGCAGTAATCTCCTTCT -3'. (SEQ ID NO.: 15)

The above primers were synthesized by Shanghai Sangon Biological Engineering Technology and Service Co., Ltd.

Experimental result is shown in Figure 46.

Figure 46 displays the real-time PCR results of lung tissue of mice. The mice were injected with control siRNA and Atg5 siRNA, respectively, 24 hours later, lung tissues were homogenized, RNAs were extracted, and real-time PCRs were performed. It demonstrates that the injection of Atg5 siRNA via trachea effectively leads to the decrease of Atg5 mRNA level.

### Example 15 Detection of survival rate of cells by MTT experiment

### 1. Detecting the effect of 3-MA on survival rate of 3-MA-treated cells by MTT experiment.

1) Digested A549 cells in logarithmic growth phase into single cell suspension, and dispersed to 96-well plate, 200µl volume for each well.
2) Set into CO2 incubator to culture for 24 hours at 37°C.
3) 24 hours later, added 3mM 3MA (or control solvent) to pretreat cells.
4) 1 hour later, added inactivated H5N1 avian influenza virus with Virus titer MOI of 2, or added chicken chorioallantoic fluid of the same volume; or added nanometer material PAMAM.
5) After affected for 4 hours (avian influenza inactivating virus) or 24 hours (nanometer materials), added 20µl MTS agent (CellTiter 96 Aqueous One Solution Cell Proliferation Assay, Promega) to each well for culturing 1-2h at 37°C.
6) Detected by ELISA Reader 490 nm
7) Analysed the results. Calculated values of average and standard deviations in the group, and drew graphs.

Experimental results are shown in Figures 38, 69 and 74.

Figure 38 displays survival percentage of A549 cells treated in different ways in bar graph. After being treated with negative control, 3MA, or inactivated H5N1 avian influenza virus, the survival rates of A549 cells were detected through MTT kit. It demonstrates that inactivated H5N1 avian influenza virus greatly reduces the survival rate of the cells, whereas 3MA decreases the cell death induced by inactivated H5N1 avian influenza virus.

Figure 69 displays the percentage of survival A549 cells after different treatments. A549 cells were pretreated with control, nanometer material PAMAM G5.5, PAMAM G3, 3-MA for 1 hour, respectively, and then treated with nanometer material PAMAM G3 and drug control 3-MA for 24 hours. The result of cell survival rate was detected by MTT method. It shows that 3-MA can significantly reduce cell death induced by nanometer material PAMAM G3.

Figure 74 displays the result of Western Blotting of A549 cells treated in different ways. A549 cells were treated with different generations of nanometer materials (PAMAM) for 24 hours, and the result of cell survival rate were detected by MTT method. It demonstrates that nanometer materials PAMAM G4, G5, G6, G7 and G8 all can induce cell death.

### 2. Detection of the survival rate of cells treated with siRNA by MTT experiment.

1) Digested A549 cells in logarithmic growth phase into single cell suspension, and dispersed to 96-well plate, 200µl volume for each well.
2) Set into CO2 incubator to culture for 24 hours at 37°C.
3) 24 hours later, the corresponding siRNA was transfected.
4) 48 hours later, added inactivated H5N1 avian influenza virus or nanometer material PAMAM.
5) After affected for 4 hours (avian influenza inactivating virus) or 24 hours (nanometer materials), added 20µl MTS agent (CellTiter 96 Aqueous One Solution Cell Proliferation Assay, Promega) to each well for culturing 1-2h at 37°C.
6) Detected by ELISA Reader 490 nm
7) Analysed the results, calculated the values of averageand standard deviations in the group, and drew graph.

Experimental results are shown in Figures 22, 33, 71 and 82.

Figure 22 displays the survival percentages of A549 cells treated in different ways in bar graph. A549 cells were transfected with control siRNA and Atg12 siRNA respectively, then treated with control agent or inactivated H5N1 avian influenza virus. The results of cell survival rate were detected through MTT kit. It demonstrates that inactivated H5N1 avian influenza virus greatly reduces of survival rate of the cells, whereas Atg12 siRNA attenuates the effects of inactivated H5N1 avian influenza virus. Namely, inhibitory effect on cell autophagy (type II cell apoptosis) relieves the cell death induced by inactivated H5N1 avian influenza virus.

Figure 33 displays survival percentage of A549 cells treated in different ways in bar graph. A549 cells were transfected with control siRNA and TSC2 siRNA, respectively, and then treated with inactivated H5N1 avian influenza virus. The the result of the survival rate of the cells was detected by MTT kit. The survival rates of the cells were increased via the transfection of TSC2 siRNA. It demonstrates that TSC2 siRNA can delay the cell death induced by inactivated H5N1 avian influenza virus.

Figure 71 displays the percentage of survival A549 cells after various treatments. A549 cells were transfected with Control siRNA and ATG6 siRNA , respectively. Then treated with nanometer material PAMAM G3 (100µg/mL) for 24 hours. The cell survival rate was detected by MTT method. It demonstrates that nanometer material PAMAM G3 induces the cell death by regulating expression of ATG6 gene so as to form autophagy.

Figure 82 displays percentage of survival A549 cells after various treatments in bar graph. A549 cells were transfected with control siRNA and TSC 2 siRNA, respectively, then treated with nanometer material PAMAM G3 (100µg/mL) for 24 hours. The survival rates of the cells were detected by MTT method. It demonstrates that nanometer material PAMAM G3 induces the cell death by regulating the expression of TSC 2 gene to form autophagy.

### 3. Detection of the survival rate of cells treated with nanometer materials by MTT experiment.

1) Digested A549 cells in logarithmic growth phase into single cell suspension, and dispersed onto 96-well plate, 200µl volume for each well.
2) Set into CO2 incubator to culture for 24 hours at 37°C.
3) 24 hours later, added nanometer material PAMAM.
4) After affected for 4 hours, added 20µl MTS agent (CellTiter 96 Aqueous One Solution Cell Proliferation Assay, Promega) to each well for culturing 1-2h at 37°C.
5) Detected by ELISA Reader 490 nm.
6) Analyzed the results, calculated values of average and standard deviations in the group, and drew a graph.

Experimental result is shown in Figure 61.

Figure 61 displays percentage of survival A549 cells after various treatments. A549 cells were treated with various generations of nanometer materials (PAMAM). 24h after the treatment, percentage of survival A549 cells was detected through MTT agent.

### Example 16 Preparation of pathological section

### 1. Preparing pathological section

1) Detected the protein concentration of H5N1 inactivated virus by Bradford method.
2) Randomly divided mice into groups, 4-6 for each group.
3) One group was injected intraperitoneally with 3MA (30mg/kg) or SB203580 (16mg/kg) one hour earlier.
4) Isolated trachea of mice. Chicken chorioallantoic fluid was dripped into control group via trachea, whereas inactivated H5N1 avian influenza virus (10µg/g) or nanometer materials PAMAM G3 (50µg/g), G5.5 (50µg/g) was dripped into experimental group via trachea.
5) After dripping, mice were mechanically ventilated with respirator (HX-200 animal respirator, Chengdu Taimeng Science and Technology Co., LTD) for 5min and stopped mechanical ventilation. Killed the mice and removed lung after spontaneous breath for 6h.
6) Dissected left lung and fixed with 13% formalin fixed liquid.
7) 48 hours later, repaired the block, dehydrated, waxed, embeded, sliced and stained the pathological tissue sections.

Experimental results are shown in Figures 39, 55 and 86.

Figure 39 displays the photographs (×200) of pathological sections from lung of Balb/c mice. Fig. A indicates the photograph of mice injected with control (chicken chorioallantoic fluid) via trachea. 6 hours after injection, the lung was dissected and sliced to obtain pathological sections. Fig. B indicates the photographs of mice injected with inactivated H5N1 avian influenza virus via trachea. 6 hours after the injection, the lung was dissected and sliced to obtain pathological sections. Fig. C indicates the photograph of mice injected with 3-MA (30mg/kg) and inactivated H5N1 avian influenza virus. 30 minutes after the 3-MA injection, the mice were injected with inactivated H5N1 avian influenza virus via trachea. 6 hours later, the lung was dissected and sliced to obtained pathological sections. It demonstrates that inactivated H5N1 avian influenza virus can lead to severe lung injury, whereas 3-MA has soem effect on ameliorating the lung injury induced by inactivated H5N1 avian influenza virus.

Figure 55 displays photograph (×200) of pathological sections of Balb/c mice lung. Fig. A indicates the photograph of pathological sections from mice injected with control (chicken chorioallantoic fluid) via trachea. The lung was dissected 6 hours after the injection. Fig. B indicates the photograph of pathological sections from mice injected with inactivated H5N1 avian influenza virus via trachea. The lung was dissected 6 hours after the injection. Fig. C indicates the photograph of pathological sections from mice injected with SB203580 (16mg/kg) via intraperitoneal injection firstly, and 30 min later injected with inactivated H5N1 avian influenza virus via trachea. The lung was dissected 6 hours after the injection. It demonstrates that inactivated H5N1 avian influenza virus leaded to severe lung injury, whereas SB203580 has some effect on ameliorating the lung injury induced by inactivated H5N1 avian influenza virus. It shows that inactivated H5N1 avian influenza virus induces lung injury through p38 signal pathway.

Figure 86 displays photograph (×200) of lung pathological sections of Balb/c mice. Fig. A indicates the photograph of pathological sections. The mice were injected with control via trachea, and 4 hours later, the lung was dissected and sliced into pathological sections. Fig.B indicates the photograph of pathological sections. The mice were injected with nanometer material PAMAM G5.5 (100µg/mL) via trachea, and 4 hours later, the lung was dissected and sliced. Fig. C indicates the photograph of pathological sections. The mice were injected with nanometer material PAMAM G3 (100µg/mL) via trachea, 4 hours later, the lung was dissected and sliced. It demonstrates that nanometer material PAMAM G3 leaded to the severe lung injury.

### 2. Preparation of pathological sections

1) Randomly divided mice into 4 groups, 4-6 for each group.
2) 30min after injection of 1M HCl (0.5uL/g) to each group via trachea, constructed model of mice lung injury.
3) Selected two groups and injected intraperitoneally and dripped via trachea 15mg/kg 3MA and 0.25mg/kg SB203580, respectively 30min before administering of HCl.
4)Isolated trachea of mice. The mice in control group were dripped with Fc (4×10-12mol/g) via trachea, whereas the mice in the other three groups were dripped with H5Fc (4×10-12mol/g) to, respectively.
5) Dissected lung after spontaneous breath for 6h.
6) Dissected left lung and fixed with 13% formalin fixed liquid
7) 48 hours later, repaired the block, dehydrated, waxed, embedded, sliced and stained the pathological tissue sections.

Experimental results are shown in Figures 49 and 58.

Figure 49 displays photographs (×200) of Balb/c mice lung in pathological sections. Fig. A indicates the photographs of mice injected with Fc protein via trachea after acid aspiration. The lung was dissected 6h later to obtain pathological sections. Fig.B indicates photographs of mice injected with H5Fc protein via trachea after acid aspiration. The lung was dissected 6h later to obtain pathological sections. Fig. C indicates photographs of mice injected with 3-MA (30mg/kg) via intraperitoneal injection firstly, and 30 min later injected with H5Fc protein via trachea after acid aspiration. The lung was dissected 6h later to obtain pathological sections. It indicates that H5Fc protein after acid aspiration aggravates the lung injury, whereas 3-MA has effect on ameliorating the lung injury induced by H5Fc protein after acid aspiration.

Figure 58 displays photograph (×200) of lung pathological sections of Balb/c mice. The left one is the photograph of pathological sections from mice injected with Fc protein via trachea after acid aspiration. The lung was dissected 6h after the injection. The middle one is the photograph of pathological sections from mice injected with H5Fc protein via trachea after acid aspiration. The lung was dissected 6h after the injection. The right one is the photograph of pathological sections from mice injected with SB203580 (16mg/kg) via intraperitoneal injection firstly, and 30min later injected with H5Fc protein via trachea after acid aspiration. The lung was dissected 6h after the last injection. It proves that injection of H5Fc protein after acid aspiration aggravated the lung injury, whereas SB203580 has some effect on ameliorating the lung injury induced by H5Fc protein after acid aspiration.

### Example 17 Counts of inflammatory cells

The lung pathological sections of mice obtain from Example 16 were observed under microscope. Fields of view amplified by 1000 times were randomly selected and the multinucleated cells and macrophages infiltrated in the lung tissue within field of view were counted.

100 fields of view were randomly counted from each experimental group.

Calculated values of average and standard deviations of multinucleated cells and macrophages infiltrated in each field of view of each group, and drew bar graph.

Experimental results are shown in Figures 40, 50, 56 and 59.

Figure 40 displays the counts of inflammatory cells located in pathological section of lung tissue under oil immersion lens (×1000) in bar graph. From left to right are the counting results in lung pathological sections from the mice injected with control (chicken chorioallantoic fluid) via trachea 6 hours before the dissection, from the mice injected with inactivated H5N1 avian influenza virus via trachea 6 hours before the dissection, from the mice firstly injected with 3-MA (30mg/kg) via intraperitoneal injection, and 30 min later, injected with inactivated H5N1 avian influenza virus via trachea 6 hours before the dissection, respectively. Inflammatory cell infiltration is one of the most important indicators. The injection of inactivated H5N1 avian influenza virus leaded to abundantly infiltrated inflammatory cells, whereas 3-MA reduced the counts of inflammatory cells induced by inactivated H5N1 avian influenza virus. The results indicate that inactivated H5N1 avian influenza virus leads to severe lung injury, whereas 3-MA has some effect on ameliorating the lung injury induced by inactivated H5N1 avian influenza virus.

Figure 50 displays the counts of inflammatory cell of lung tissue in pathological sections under oil immersion lens (×1000) in bar graph. From left to right, indicates the counting results in lung pathological sections from the mice injected with Fc protein via trachea after acid aspiration, from the mice injected with H5Fc protein via trachea after acid aspiration, from the mice injected with 3-MA (30mg/kg) via intraperitoneal injection firstly, and 3min later injected with H5Fc protein via trachea after acid aspiration, respectively. The lung was dissected 6h later. Inflammatory cell infiltration is one of important indicators. The injection of H5Fc protein increased the counts of infiltrated inflammatory cells, whereas 3-MA reduced the augmentation of the counts inflammatory cells induced by H5Fc protein. The result proves that H5Fc protein after acid aspiration can aggravate lung injury, whereas 3-MA has some effect on ameliorating the lung injury induced by H5Fc protein after acid aspiration.

Figure 56 displays the counts of inflammatory cells in lung tissue pathological sections under oil immersion lens (×1000). From left to right were the counting results in lung pathological sections of mice injected with control (chicken chorioallantoic fluid) via trachea, the counting results in lung pathological sections of mice injected with inactivated H5N1 avian influenza virus via trachea, the counting results in lung pathological sections of mice injected with SB203580 (16mg/kg) via intraperitoneal injection firstly, and 30min later injected with inactivated H5N1 avian influenza virus via trachea, respectively. The lung was dissected 6 hours after the injection. Inflammatory cell infiltration is one of important indicators of lung injury. Injection of inactivated H5N1 avian influenza virus leaded to abundantly infiltrated inflammatory cells, whereas SB203580 reduced the counts of infiltrated inflammatory cells induced by inactivated H5N1 avian influenza virus. The results prove that inactivated H5N1 avian influenza virus leads to severe lung injury, whereas SB203580 has some effect on ameliorating the lung injury induced by inactivated H5N1 avian influenza virus.

Figure 59 displays the counts of inflammatory cells in lung tissue pathological sections under oil immersion lens (×1000). Fig. A indicates the counting results in lung pathological sections from mice injected with Fc protein via trachea after acid aspiration. Fig.B indicates counting results in lung pathological sections from mice injected with H5Fc protein via trachea after acid aspiration. Fig. C indicates counting results in lung pathological sections from mice injected with SB203580 (16mg/kg) via intraperitoneal injection firstly, and 30 min later injected with H5Fc protein via trachea after acid aspiration, respectively. The lung was dissected 6h after the last injection. Inflammatory cell infiltration is one of important indicators of lung injury. The injection of H5Fc protein increased the counts of infiltrated inflammatory cells, whereas SB203580 reduced the augmentation of infiltrated inflammatory cells induced by H5Fc protein. The result proves that H5Fc protein after acid aspiration aggravates lung injury, whereas SB203580 has some effect on ameliorating the lung injury induced by H5Fc protein after acid aspiration.

### Example 18 Measurement of lung wet/dry ratio

### 1. Detected the effect of drugs on the change of lung wet/dry ratio induced by avian influenza.

1) Detemined the concentration of H5N1 inactivated virus protein by Bradford method.
2) Randomly divided mice into 5 groups, 4-6 of each group.
3) Selected two groups, 30min and 1h earlier, the mice were injected intraperitoneally with 3MA (30mg/kg) and SB203580 (16mg/kg) and Wortmannin (1.5mg/kg), respectively.
4) Isolated trachea of mice, the mice in control group were dripped with chicken chorioallantoic fluid via trachea, and the mice in other three groups were dripped with inactivated H5N1 avian influenza virus (10µg/g) via trachea, respectively.
5) After dripping, mice were mechanically ventilated with respirator (HX-200 animal respirator, Chengdu Taimeng Science and Technology Co., LTD) and 5min later stopped mechanical ventilation. The lung was removed after spontaneous breath for 6h.
6) Dissected right lung and measured the wet weigh thereof.
7) Measured dry weight after baking the lung for 24 hours at 68□.
8) Obtained wet/dry ratio from wet weight divided by dry weight. The wet/dry ratio can be used to evaluate the degree of pulmonary edema.

Experimental results are shown in Figures 44 and 57.

Figure 44 displays wet/dry ratio of Balb/c mice lung of in bar graph. It shows, from left to right, the wet/dry ratio of mice lung from the mice injected with control (chicken chorioallantoic fluid) 6 hours before the dissection via trachea, from the mice injected with inactivated H5N1 avian influenza virus 6 hours before the dissection via trachea, and from the mice injected with 3-MA (30mg/kg) via intraperitoneal injection firstly, and 30 min later injected with inactivated H5N1 avian influenza virus via trachea, from the mice injected with Wortmannin (1.5mg/kg) via intraperitoneal injection firstly, and 30 min later injected with inactivated H5N1 avian influenza virus via trachea. Lung wet/dry ratio is one of important indicators of lung injury. Injection of inactivated H5N1 avian influenza virus greatly increased the wet/dry ratio, whereas 3-MA and Wortmannin decreased the augmentation of wet/dry ratio induced by inactivated H5N1 avian influenza virus. The result indicates that inactivated H5N1 avian influenza virus leads to the severe lung injury, whereas 3-MA and Wortmannin have some effect on ameliorating the lung injury induced by inactivated H5N1 avian influenza virus.

Figure 57 displays wet/dry ratio of Balb/c mice lung in bar graph. They indicate, from left to right, lung wet/dry ratio of mice 6 hours after the injection of control (chicken chorioallantoic fluid) via trachea, lung wet/dry ratio of mice 6 hours after injection of inactivated H5N1 avian influenza virus via trachea, lung wet/dry ratio of mice 6 hours after the injection with SB203580 (16mg/kg) via intraperitoneal injection firstly, and 1h later injected with inactivated H5N1 avian influenza virus via trachea. Lung wet/dry ratio is one of important indicators of lung injury. The injection of inactivated H5N1 avian influenza virus greatly increased wet/dry ratio, whereas SB203580 decreased augmentation of wet/dry ratio induced by inactivated H5N1 avian influenza virus. The result proves that inactivated H5N1 avian influenza virus leads to the severe lung injury, whereas SB203580 has some effect on ameliorating the lung injury induced by inactivated H5N1 avian influenza virus.

### 2. Detecting the effect of drugs on the change of lung wet/dry ratio induced by nanometer materials.

1) Randomly divided mice into 5 groups, 4-6 of each group.
2) Selected two groups and injected the mice intraperitoneally with 3MA (30mg/kg) 1h earlier.
3) Isolated trachea of mice, the mice in control group and one group of those injected intraperitoneally with 3MA were dripped with PBS via trachea. The mice in the other group which were injected intraperitoneally with 3M were dripped with nanometer material PAMAM G3(50µg/g) via trachea. The mice in the other two groups were dripped with nanometer material PAMAM G3 (50µg/g) and G5.5 (50µg/g) via trachea.
4) After dripping, the mice were mechanically ventilated with respirator (HX-200 animal respirator, Chengdu Taimeng Science and Technology Co., LTD) and 5min later stopped the mechanical ventilation. The lung was removed after spontaneous breath for 6h.
5) Measured the wet weight of right lung.
6) Measured dry weight after baking 24 hours at 68°C.
8) Obtained wet/dry ratio from wet weight divided by dry weight. The wet/dry ratio can used to evaluate the degree of pulmonary edema.

Experimental result is shown in Figure 87.

Figure 87 displays lung wet/dry ratio of Balb/c mice in bar graph. They indicate, from left to right, lung wet/dry ratio of mice 16 hours after the injection of control via trachea for, lung wet/dry ratio of mice 16 hours after the injection of nanometer material PAMAM G5.5 (100µg/mL) via trachea, lung wet/dry ratio of mice 16 hour after the injection of nanometer material PAMAM G3 (100µg/mL) via trachea, lung wet/dry ratio of mice 16 hour after the injection of nanometer material PAMAM G3 via trachea, and 1 hour before the injection of PAMAM G3 via trachea, mice were injected with 3-MA via intraperitoneal, and lung wet/dry ratio of mice 17 hours after the injection of 3-MA (15mg/kg) via intraperitoneal. Lung wet/dry ratio is one of important indicators of lung injury. The injection of nanometer material PAMAM G3 via trachea increased wet/dry ratio of lung tissue, whereas 3-MA relieved the the increased lung wet/dry ratio of mice induced by nanometer material PAMAM G3. The result proves that nanometer material PAMAM G3 leads to the severe lung injury, whereas 3-MA has some effect on ameliorating the lung injury induced by nanometer material PAMAM G3.

### 3. Detecting the effect of drugs on the change of lung wet/dry ratio induced by avian influenza surface protein.

1) Randomly divided mice into 5 groups, 4-6 of each group.
2) 30min after injection of 1M HCl (0.5uL/g) to each group via trachea, constructed lung injury model of mice.
3) Selected three groups, 30min before administering HCl, injected intraperitoneally and dripped via trachea with 15mg/kg 3MA, 0.25mg/kg SB203580 and 0.25mg/kg LY294002, respectively.
4) Isolated tracheas of mice, the mice in control group were dripped with Fc (4×10-12mol/g) via trachea, the mice in the other three groups were dripped with H5Fc (4×10-12mol/g), respectively.
5) Removed lung after spontaneous breath for 6h.
6) Measured wet weigh of right lung.
7) Measured dry weight after baking 24 hours at 68°C.
8) Obtained wet/dry ratio from wet weight divided by dry weight. The wet/dry ratio can be used to evaluate the degree of pulmonary edema.

Experimental results are shown in Figures 51 and 60.

Figure 51 displays wet/dry ratio of Balb/c mice lung in bar graph. From left to right are lung wet/dry ratio of mice injected with Fc protein via trachea after acid aspiration, lung wet/dry ratio of mice injected with H5Fc protein via trachea after acid aspiration, lung wet/dry ratio of mice injected with 3-MA (30mg/kg) via intraperitoneal injection firstly, and 30 min later injected with H5Fc protein via trachea after acid aspiration, respectively, lung wet/dry ratio of mice injected with LY294002 (0.25mg/kg) via intraperitoneal injection firstly, and 30 min later injected with H5Fc protein via trachea for 6 hours after acid aspiration. Lung wet/dry ratio is one of important indicators of lung injury. The injection of H5Fc protein via trachea after acid aspiration greatly increased the wet/dry ratio, whereas 3-MA and LY294002 decreased the augmentation of wet/dry ratio induced by H5Fc protein after acid aspiration. The result proves that H5Fc protein after acid aspiration leads to the severe lung injury, whereas 3-MA and LY294002 have some effect on ameliorating the lung injury induced by H5Fc protein after acid aspiration.

Figure 60 displays lung wet/dry ratio of Balb/c mice in bar graph. From left to right, they indicate lung wet/dry ratio of mice injected with Fc protein via trachea after acid aspiration, lung wet/dry ratio of mice injected with H5Fc protein via trachea after acid aspiration, lungwet/dry ratio of mice injected with SB203580 (16mg/kg) via intraperitoneal injection firstly, and then 30min later injected with H5Fc protein via trachea after acid aspiration, respectively. The lung was dissected 6h after the last injection. Lung wet/dry ratio is one of important indicators of lung injury, the injection of H5Fc protein via trachea after acid aspiration greatly increased wet/dry ratio, whereas SB203580 decreased the augmentation of wet/dry ratio induced by H5Fc protein after acid aspiration. The result proves that H5Fc protein after acid aspiration leads to the severe lung injury, whereas SB203580 has some effect on ameliorating the lung injury induced by H5Fc protein after acid aspiration.

### 4. Detecting the effect of drugs on the change of lung wet/dry ratio induced by avian influenza.

1) Determined the concentration of H5N1 inactivated virus protein by Bradford method.
2) Randomly divided the mice into 4 groups, 4-6 of each group.
3) Injected control siRNA (100µg) and Atg5siRNA (100µg) to each two groups, respectively.
4) 24 hours later, the mice in the control siRNA groups were dripped with chicken chorioallantoic fluid and inactivated H5N1 avian influenza virus (10µg/g) via trachea, respectively, and the mice in Atg5siRNA groups were dripped with chicken chorioallantoic fluid and inactivated H5N1 avian influenza virus (10µg/g) via trachea, respectively.
5) After dripping, the mice were mechanically ventilated with respirator (HX-200 animal respirator, Chengdu Taimeng Science and Technology Co., LTD), 5min later stopped mechanical ventilation. The lung was removed after spontaneous breath for 6h.
6) Measured wet weigh of right lung.
7) Measured dry weight after baking 24 hours at 68°C.
8) Obtained wet/dry ratio from wet weight divided by dry weight. The wet/dry ratio can be used to evaluate the degree of pulmonary edema.

Experimental result is shown in Figure 48.

Figure 48 displays lung wet/dry ratio of Balb/c mice in bar graph. Mice were injected with control siRNA and Atg5 siRNA, respectively. 24 hours later, mice were injected with control (chicken chorioallantoic fluid) and inactivated H5N1 avian influenza virus, respectively. The wet/dry ratio of mice lung tissue of was shown 4 hours later. Lung wet/dry ratio is one of important indicators of lung injury. Injection of inactivated H5N1 avian influenza virus greatly increased wet/dry ratio, whereas Atg5 siRNA has some effect on relieving lung injury through inhibiting the expression of Atg5 protein, suggesting that inactivated H5N1 avian influenza virus induces the occurrence of lung injury through activating cell autophagy (type II cell apoptosis).

### Example 19 Method of lung elasticity

### 1 Effect of drugs on the change of lung elasticity induced by avian influenza virus.

1) Selected Balb/c mice of 8-10 weeks old and randomly divided the mice into groups. 3MA (30mg/kg) and PBS was injected via intraperitoneally 30min earlier, respectively.
2) Anesthetized mice with 1% sodium pentobarbital.
3) Performed operation of trachea cannula of mice.
4) After the mice were deeply anesthetized, detected Cchord (0-10cmH2O lung tissue compliance) of lung tissue of mice at Oh by BUXCO PFT, and calculated E (lung elasticity)=1/Cchord.
5) Injected chorioallantoic fluid or H5N1 inactivating virus (200µg) accurately to trachea cannula with pipette, and recorded the time of injection.
6) Ventilated the mice with animal respirator, f (frequency)= 150 times/min, p (pressure)= 30cmH2O 3sec, p=25cmH2O 2min.
7) Maintained the spontaneous breath of the mice.
8) Half hour after administrating, detected Cchord of lung tissue of mice by BUXCO PFT to obtain E=1/Cchord at each half hour.
9) Calculated the rate of changed elasticity of lung tissue of each half hour, and drew the curve.

Experimental result is shown in Figure 43.

Figure 43 displays the result of elasticity of Balb/c mice lung tissue. It shows the changes of elasticity of Balb/c mice lung tissue from the mice group injected with control (chicken chorioallantoic fluid) via trachea, from the group injected with inactivated H5N1 avian influenza virus via trachea, and from the group injected with 3-MA (30mg/kg) via intraperitoneal injection firstly, and 30 min later injected with inactivated H5N1 avian influenza virus via trachea, respectively. The changes of lung elasticity of mice with spontaneous breath were detected every 30min for 4 hours. Lung elasticity is an important indicator to measure lung function. The injection of inactivated avian influenza virus via trachea greatly decreased the compliance of mice lung, whereas 3-MA has some effect on relieving the induced injury and on protecting function of lung.

### 2. Effect of siRNA on the change of lung elasticity induced by avian influenza virus.

1) Selected Balb/c mice of 8-10 weeks old, randomly divided the mice into groups. Mice were anesthetized with 1% sodium pentobarbital.
2) Injected siRNA Control 100uL (100µg) and siRNA Atg5 100uL (100µg) via trachea with microsyringe.
3) 24 hours later, anesthetized mice with 1% sodium pentobarbital.
4) Performed operation of trachea cannula of mice.
5) After the mice were deeply anesthetized, detected Cchord (0-10cmH2O lung tissue compliance) of lung tissue of mice at Oh by BUXCO PFT, and calculated E (lung elasticity)=1/Cchord.
6) Injected chorioallantoic fluid or H5N1 inactivating virus (200µg) accurately to trachea cannula with pipette, and recorded the time of injection.
7) Ventilated the mice with animal respirator, f (frequency) =150 times/min, p (pressure) = 30cmH2O 3sec, p=25cmH2O 2min.
8) Maintained spontaneous breath of the mice.
9) Half hour after administrating, detected Cchord of lung of mice by BUXCO PFT to obtain E=1/Cchord at each half hour.
10) Calculated the rate of changed elasticity of lung tissue of each half hour, and drew the curve.

Experimental result is shown in Figure 47.

Figure 47 displays the result of elasticity of Balb/c mice lung tissue. The mice were injected with control siRNA and Atg5 siRNA, respectively. 24 hours later, the mice were injected with control (chicken chorioallantoic fluid) and inactivated H5N1 avian influenza virus, respectively. The elasticity of mice lung tissue was detected every 30min. Detections of the changed lung elasticity of the mice with spontaneous breath were performed within 4 hours. The injection of inactivated avian influenza virus via trachea greatly decreased the compliance of mice lung, whereas Atg5 siRNA has some effect on relieving the lung injury via inhibiting the expression of Atg5 protein, suggesting that inactivated H5N1 avian influenza virus induces the occurrence of lung injury via activating cell autophagy (type II cell apoptosis).

### 3. The effect of drugs on the change of lung elasticity induced by nanometer materials.

1) Selected Balb/c mice of 8-10 weeks old and randomly divided the mice into groups. Injected intraperitoneally 3MA (30mg/kg) and PBS 30min earlier, respectively.
2) Anesthetized mice with 1% sodium pentobarbital.
3) Performed operation of trachea cannula of mice.
4) After the mice were deeply anesthetized, detected Cchord (0-10cmH2O lung tissue compliance) of lung tissue of mice at Oh by BUXCO PFT, and calculated E (lung elasticity)= 1/Cchord.
5) Injected PBS or nanometer material PAMAM G3 (12.5µg/g) and G5.5 (12.5µg/g) inactivated virus accurately to trachea cannula with pipette, and recorded the time of injection.
6) Ventilating the mice with animal respirator, f (frequency)= 150 times/min, p (pressure)= 30cmH2O 3sec, p=25cmH2O 2min.
7) Maintained the spontaneous breath of the mice.
8) Half hour after administration, detected Cchord of lung tissue of mice by BUXCO PFT to obtain E=1/Cchord at each half hour.
9) Calculated the rate of changed elasticity of lung tissue of each half hour, and drew the curve.

Experimental result is shown in Figure 88.

Figure 88 displays diagram of the changed lung elasticity of Balb/c mice. From left to right, it indicates successively the changed lung elasticity of Balb/c mice injected with control via trachea, the changed lung elasticity of mice injected with nanometer material PAMAM G5.5 (50mg/kg) via trachea, lung wet/dry ratio of mice injected with nanometer material PAMAM G3 (50mg/kg), the changed lung elasticity of mice injected with 3-MA (15mg/kg) via intraperitoneal, and 1h later injected with nanometer material PAMAM G3. Lung elasticity is one of important indicators of lung injury. The nanometer material PAMAM G3 leads to the changes of lung elasticity of mice, whereas 3-MA relieves the changes of lung elasticity of mice induced by nanometer material PAMAM G3. The result proves that nanometer material PAMAM G3 leads to the severe lung injury, whereas 3-MA has some effects on ameliorating the lung injury induced by nanometer material PAMAM G3.

### Example 20 Experiment of survival rate

### 1. Effect of drugs on the survival ratio induced by avian influenza virus

1) Detected the concentration of H5N1 inactivated virus protein by Bradford method.
2) Randomly divided mice into 4 groups, 6-8 of each group.
3) Selected two groups, 8h and 2h earlier and 0.5 hour after injection of H5N1 inactivated virus, injected intraperitoneal 3MA (15mg/kg), respectively.
4) Isolated tracheas of mice, the mice in control group and in one group of 3MA were dripped with chicken chorioallantoic fluid via trachea, and the mice in the other two groups were dripped with inactivated H5N1 avian influenza virus (10µg/g) via trachea, respectively.
5) After dripping, the mice were mechanically ventilating with respirator (HX-200 animal respirator, Chengdu Taimeng Science and Technology Co., LTD) and 5min later stopped mechanical ventilation. Maintained the spontaneous breath of the mice.
6) Recorded the surviving condition of mice every half hour.
7) Statistically analyzed the final results by SPSS and drew the curve of survival rate.

Experimental result is shown in Figure 45.

Figure 45 displays the survival curve of Balb/c mice. The mice were injected with 3-MA (15mg/kg) via intraperitoneal injection. 30 min, 2h and 8h after the injection, the mice were injected with inactivated H5N1 avian influenza virus via trachea. The survival situations were observed every 15 minutes. It demonstrates that 3-MA has effect on delaying the death of mice.

### 2. Effect of drugs on the survival ratio induced by nanometer material PAMAM

1) Detected the concentration of H5N1 inactivated virus protein by Bradford method.
2) Randomly divided mice into 4 groups, 6-8 of each group.
3) Selected two groups, 12h and 3h earlier and 0.5 hour after injection of nanometer material PAMAM G3 (50µg/g) via trachea, the mice were injected intraperitoneal with 3MA (15mg/kg), respectively.
4) Isolated trachea of mice, the mice in control group and one group of 3MA via trachea were dripped with PBS, and the mice in the other two groups were dripped with nanometer material PAMAM G3 (50µg/g) via trachea, respectively.
5) Recorded the surviving condition of mice every half hour.
6) Statistically analyzed the final results by SPSS and drew the curve of survival rate.

Experimental result is shown in Figure 89.

Figure 89 displays survival curve of Balb/c mice. Balb/c mice were treated as follows: injected with control via trachea; injected with nanometer material PAMAM G5.5 (50mg/kg) via trachea; injected with nanometer material PAMAM G3 (50mg/kg) via trachea; injected with 3-MA (15mg/kg) via intraperitoneal injection firstly, and 1h later injected with nanometer material PAMAM G3 (50mg/kg) via trachea; and injected with 3-MA (15mg/kg) via intraperitoneal injection. The mice were observed for 24 hours continuously. Counted up the surviving conditions of mice every other hour, and conducted statistical analysis. Results: nanometer material PAMAM G3 increased the death rate of mice, whereas 3-MA can significantly relieve the death of mice induced by nanometer material PAMAM G3.

Figure 90 displays the structural formula of wortmannin. CAS No. is 19545-26-7, molecular formula is C23H24O8, and molecular weight is 428.43.

### Example 21 Drawing cellular signal pathway

Cellular signal pathway were drawn by software of photoshop, certain shapes were used to represent different molecules or organelles or tissues in signal pathway. Arrow represents that upstream signal molecules activates downstream signal molecules or causes certain effect, and "T" shape represents inhibiting effect.

Experimental results are shown in Figures 37 and 85.

Figure 37 displays diagram of cell signal pathway. From the above experimental result, we can get the conclusion as shown in the diagram: avian influenza virus can induce cell autophagy (type II cell apoptosis) via the pathway of from AKT to TSC1/2 to mTOR and autophagy. Avian influenza virus inhibits AKT, AKT inhibits TSC1/2, TSC1/2 inhibits mTOR pathway, mTOR pathway inhibits cell autophagy (type II cell apoptosis); The autophagy pathway works through AtgS-Atg12 to LC3 pathway so as to induce cell autophagy (type II cell apoptosis).

Figure 85 displays diagram of cell signal pathway. From the above experimental results, we conclude as shown in the diagram: nanometer material PAMAM G3 can activate the pathway of Akt-TSC 1/2-mTOR-autophagy. The activation of mTOR can inhibit the occurrence of Autophagy; and the activation of TSC1/2 can inhibit the activation of mTOR so as to enhance cell autophagy (type II cell apoptosis).

### Example 22 DNA Ladder detection

1) Digested A549 cells in logarithmic growth phase into single cell suspension, and dispersed to 6cm-dish.
2) Set into CO2 incubator to culture for 24 hours at 37°C.
3) Added PBS to each dish as negative control, added 6% DMSO as positive control and added nanometer material PAMAM G3 to each dish, separately.
4) 24 hours later, collected all cells.
5) Extracted genomic DNA of cells by Qiagen complete genome extracting kit.
6) Performed agarose gel electrophoresis, 60V, 2h.
7) Observed and photographed under UV.

Experimental result is shown in Figure 62.

Figure 62 displays genomic electrophoretogram of A549 cells treated in different ways. The A549 cells were collected after treated with control, dimethyl sulfoxide (DMSO, 6% v/v) and nanometer material PAMAM G3 (100µg/mL). The genomic DNA of cells was isolated with Genome Extraction Kit and agarose gel electrophoresis was performed. Dimethyl sulfoxide was used as inducer for apoptosis.

### Example 23 Fluorescent detection of Caspase-3 activity

1) Digested A549 cells in logarithmic growth phase into single cell suspension, and dispersed to 6cm-dish.
2) Set into CO2 incubator to culture for 24 hours at 370.
3) Added PBS to each dish as negative control, added 6% DMSO as positive control, and added nanometer material PAMAM G3 and G5.5to each dish, separately.
4) 24 hours later, lysed cells.
5) Centrifuged, removed supernatant and detected by caspase-3 activity fluorescence detection kit (CEPREI).
6) Detected the fluorescence intensity.

Experimental result is shown in Figure 63.

Figure 63 displays the result of Caspase-3 activity in A549 cells treated in different ways. A549 cells were treated with control, dimethyl sulfoxide (DMSO, 6% v/v), nanometer material PAMAM G5.5 and PAMAM G3 generation for 24 hours, respetively. The Caspase-3 activity of cells was detected with Caspase-3 activity test kit.

### Example 24 Formula of compounds

The structures of compounds are drawn according to standard rules commonly used in the field of chemistry.

Experimental results are shown in Figures 90, 91, 92 and 93.

Figure 90 displays the structural formula of wortmannin. CAS No. is 19545-26-7, molecular formula is C23H24O8, and molecular weight is 428.43.

Figure 91 displays structural formula of LY-294,002. CAS No. is 934389-88-5, molecular formula is C19H17NO3·HCl, and molecular weight is 343.80.

Figure 92 displays structure of 3-Methyladenine. CAS No. is 15142-23-4, molecular formula is C6H7N5, and molecular weight is 149.15.

Figure 93 displays the structure of SB 203580. CAS No. is 152121-47-6, molecular formula is C21H16FN3OS, and molecular weight is 377.43.

## Claims

1. Inhibitor of autophagy (type II programmed cell death) for use in preventing and/or treating avian influenza of mammals, the said inhibitor is selected from 3-methyladenine.

2. Inhibitor of autophagy for use according to claim 1, wherein the mammal is human.

3. Inhibitor of autophagy for use according to claim 1 or 2, wherein the said avian influenza of mammals includes lung injury of mammals induced by avian influenza virus.

4. Inhibitor of autophagy for use according to claim 3, wherein the said lung injury includes acute respiratory distress syndrome.

5. Inhibitor of autophagy for use according to claim 3, wherein the said avian influenza virus is H5N1, H5N2, H9N2 virus.

6. Inhibitor of autophagy for use according to claim 1 or 2, wherein the 3-methyladenine is an inhibitor of autophagy (type II PCD), which is an inhibitor of signal transduction pathway of P13K Class III cell.

## Patentansprüche

1. Autophagie (programmierter Zelltod Typ II) Inhibitor zur Verwendung bei der Verhinderung und/oder Behandlung von Vogelgrippe in Säugern, worin der Inhibitor ausgewählt ist unter 3-Methyladenin.

2. Autophagie-Inhibitor zur Verwendung nach Anspruch 1, worin der Säuger ein Mensch ist.

3. Autophagie-Inhibitor zur Verwendung nach Anspruch 1 oder 2, worin die Vogelgrippe in Säugern Lungenverletzungen der Säuger umfasst, die durch das Vogel-Influenzavirus hervorgerufen wurden.

4. Autophagie-Inhibitor zur Verwendung nach Anspruch 3, worin die Lungenverletzungen akutes Atemnotsyndrom umfassen.

5. Autophagie-Inhibitor zur Verwendung nach Anspruch 3, worin das Vogel-Influenzavirus das H5N1-, H5N2-, H9N2-Virus ist.

6. Autophagie-Inhibitor zur Verwendung nach Anspruch 1 oder 2, worin das 3-Methyladenin ein Autophagie (PCD Typ II) Inhibitor ist, welcher ein Inhibitor des Signal-übertragungsweges von P13K Klasse III Zellen ist.

## Revendications

1. Inhibiteur de l'autophagie (mort cellulaire programmée de type II) destiné à être utilisé dans la prévention et/ou le traitement de la grippe aviaire des mammifères, ledit inhibiteur étant sélectionné parmi la 3-méthyladénine.

2. Inhibiteur de l'autophagie destiné à être utilisé selon la revendication 1, où le mammifère est humain.

3. Inhibiteur de l'autophagie destiné à être utilisé selon la revendication 1 ou 2, où ladite grippe aviaire des mammifères comprend une lésion pulmonaire de mammifères induite par un virus de la grippe aviaire.

4. Inhibiteur de l'autophagie destiné à être utilisé selon la revendication 3, où ladite lésion pulmonaire comprend le syndrome de détresse respiratoire aiguë.

5. Inhibiteur de l'autophagie destiné à être utilisé selon la revendication 3, où ledit virus de la grippe aviaire est le virus H5N1, H5N2, H9N2.

6. Inhibiteur de l'autophagie destiné à être utilisé selon la revendication 1 ou 2, où la 3-méthyladénine est un inhibiteur de l'autophagie (MCP de type II), qui est un inhibiteur de la voie de transduction du signal d'une cellule PI3K de classe III.


### Sequence Listing

<110> Institute of Basic Medical Sciences, Chinese Academy of Medical Sciences
<120> Use of Cell Autophage (type II Cell Apoptosis) Inhibitors
<130> 880162CG
<160> 15
<170> PatentIn version 3.4
<210> 1
   <211> 1563
   <212> DNA
   <213> artificial
<400> 1
   gcgctagccg accagatctg catcgggtac cacgccaaca acagcaccga gcaggtcgac      60
   accatcatgg agaagaacgt gaccgtgacc cacgcccagg acatcctcga gaagacccac     120
   aacggcaagc tgtgcgacct ggacggcgtg aagcccctga tcctgcgcga ctgcagcgtg     180
   gccggctggc tgctgggcaa ccccatgtgt gacgagttca tcaacgtgcc cgagtggagc     240
   tacatcgtgg agaaggccaa ccccgtgaac gacctgtgct accccggcga cttcaacgac     300
   tacgaggagc tgaagcacct gctgagccgc atcaaccact tcgagaagat ccagatcatc     360
   cccaagagca gctggagcag ccacgaggcc agcctgggcg tcagcagcgc ctgtccctac     420
   cagggcaaga gcagcttctt ccgcaacgtg gtgtggctga tcaagaagaa cagcacctac     480
   cccaccatca agcgcagcta caacaacacc aaccaggagg acctgctggt gctgtggggc     540
   atccaccacc ccaacgacgc cgccgagcag accaagctgt accagaaccc caccacctac     600
   atcagcgtgg gcaccagcac cctcaaccag cgcctcgtgc cccgcatcgc cacccgcagc     660
   aaggtcaacg ggcagtccgg ccgcatggag ttcttctgga ccatcctcaa gcccaacgac     720
   gccatcaact tcgagagcaa cggcaacttc atcgcccccg agtacgccta caagatcgtg     780
   aagaagggcg acagcaccat tatgaagagc gagctggagt acggcaactg caacaccaag     840
   tgccagaccc ctatgggcgc catcaacagc agcatgccct tccacaacat ccaccccctg      900
   accatcggcg agtgccccaa atacgtgaag agcaaccgcc tggtgctggc caccggcctg     960
   cgcaacagcc cccagcgcga gcgccgccgg aagaagcgcg gcctgttcgg cgccatcgcc    1020
   ggcttcatcg agggcggctg gcagggcatg gtggacgggt ggtatgggta ccaccacagc    1080
   aacgagcagg gctccggcta cgccgccgac aaggagagca cccagaaggc catcgacggc    1140
   gtgaccaaca aggtcaacag catcatcgac aagatgaaca cccagttcga ggccgtgggg    1200
   cgcgagttca acaacctgga gcgccgcatc gagaacctga acaagaagat ggaggacggc    1260
   ttcctggacg tgtggaccta caacgccgag ctgctggtgc tgatggagaa cgagcgcacc    1320
   ctggacttcc acgacagcaa cgtgaagaac ctgtacgaca aggtccgcct gcagctgcgc    1380
   gacaacgcca aggagctggg caacggctgc ttcgagttct accacaagtg cgacaacgag    1440
   tgcatggaga gcgtgcgcaa cggcacctac gactaccccc agtacagcga ggaggcccgc    1500
   ctgaagcgcg aggagatcag cggcgtgaag ctggagagca tcggcattta ccagaaggat     1560
   cca                                                                  1563
<210> 2
   <211> 21
   <212> RNA
   <213> artificial
<220>
   <221> modified_base
   <222> (20)..(21)
   <223> n=dT
<400> 2
   gggacauucu gcugaacaun n 21
<210> 3
   <211> 21
   <212> RNA
   <213> artificial
<220>
   <221> modified_base
   <222> (20)..(21)
   <223> n=dT
<400> 3
   auguucagca gaaugucccn n 21
<210> 4
   <211> 21
   <212> RNA
   <213> artificial
<220>
   <221> modified_base
   <222> (20)..(21)
   <223> n=dT
<400> 4
   accggaaacu cauggaauan n 21
<210> 5
   <211> 21
   <212> RNA
   <213> artificial
<220>
   <221> modified_base
   <222> (20)..(21)
   <223> n=dT
<400> 5
   uauuccauga guuuccggun n 21
<210> 6
   <211> 21
   <212> RNA
   <213> artificial
<220>
   <221> modified_base
   <222> (20).. (21)
   <223> n=dT
<400> 6
   caguuuggca caaucaauan n 21
<210> 7
   <211> 21
   <212> RNA
   <213> artificial
<220>
   <221> modified_base
   <222> (20)..(21)
   <223> n=dT
<400> 7
   uauugauugu gccaaacugn n 21
<210> 8
   <211> 21
   <212> RNA
   <213> artificial
<220>
   <221> modified_base
   <222> (20)..(21)
   <223> n=dT
<400> 8
   gcaguagagc gaacacgaan n 21
<210> 9
   <211> 21
   <212> RNA
   <213> artificial
<220>
   <221> modified_base
   <222> (20)..(21)
   <223> n=dT
<400> 9
   uucguguucg cucuacugcn n 21
<210> 10
   <211> 21
   <212> RNA
   <213> artificial
<220>
   <221> modified_base
   <222> (20)..(21)
   <223> n=dT
<400> 10
   ccaucaaggg ccaguucaan n 21
<210> 11
   <211> 21
   <212> RNA
   <213> artificial
<220>
   <221> modified_base
   <222> (20).. (21)
   <223> n=dT
<400> 11
   uugaacuggc ccuugauggn n 21
<210> 12
   <211> 22
   <212> DNA
   <213> artificial
<400> 12
   cagatggaca gctgcacaca ct 22
<210> 13
   <211> 22
   <212> DNA
   <213> artificial
<400> 13
   ggctctatcc cgtgaatcat ca 22
<210> 14
   <211> 21
   <212> DNA
   <213> artificial
<400> 14
   agtgtgacgt tgacatccgt a 21
<210> 15
   <211> 22
   <212> DNA
   <213> artificial
<400> 15

### Sequence Listing

<110> Institute of Basic Medical Sciences, Chinese Academy of Medical Sciences
<120> Use of Cell Autophage (type II Cell Apoptosis) Inhibitors
<130> 880162CG
<160> 15
<170> PatentIn version 3.4
<210> 1
   <211> 1563
   <212> DNA
   <213> artificial
<400> 1
   gcgctagccg accagatctg catcgggtac cacgccaaca acagcaccga gcaggtcgac      60
   accatcatgg agaagaacgt gaccgtgacc cacgcccagg acatcctcga gaagacccac     120
   aacggcaagc tgtgcgacct ggacggcgtg aagcccctga tcctgcgcga ctgcagcgtg     180
   gccggctggc tgctgggcaa ccccatgtgt gacgagttca tcaacgtgcc cgagtggagc     240
   tacatcgtgg agaaggccaa ccccgtgaac gacctgtgct accccggcga cttcaacgac     300
   tacgaggagc tgaagcacct gctgagccgc atcaaccact tcgagaagat ccagatcatc     360
   cccaagagca gctggagcag ccacgaggcc agcctgggcg tcagcagcgc ctgtccctac     420
   cagggcaaga gcagcttctt ccgcaacgtg gtgtggctga tcaagaagaa cagcacctac     480
   cccaccatca agcgcagcta caacaacacc aaccaggagg acctgctggt gctgtggggc     540
   atccaccacc ccaacgacgc cgccgagcag accaagctgt accagaaccc caccacctac     600
   atcagcgtgg gcaccagcac cctcaaccag cgcctcgtgc cccgcatcgc cacccgcagc     660
   aaggtcaacg ggcagtccgg ccgcatggag ttcttctgga ccatcctcaa gcccaacgac     720
   gccatcaact tcgagagcaa cggcaacttc atcgcccccg agtacgccta caagatcgtg     780
   aagaagggcg acagcaccat tatgaagagc gagctggagt acggcaactg caacaccaag     840
   tgccagaccc ctatgggcgc catcaacagc agcatgccct tccacaacat ccaccccctg    900
   accatcggcg agtgccccaa atacgtgaag agcaaccgcc tggtgctggc caccggcctg     960
   cgcaacagcc cccagcgcga gcgccgccgg aagaagcgcg gcctgttcgg cgccatcgcc    1020
   ggcttcatcg agggcggctg gcagggcatg gtggacgggt ggtatgggta ccaccacagc    1080
   aacgagcagg gctccggcta cgccgccgac aaggagagca cccagaaggc catcgacggc    1140
   gtgaccaaca aggtcaacag catcatcgac aagatgaaca cccagttcga ggccgtgggg    1200
   cgcgagttca acaacctgga gcgccgcatc gagaacctga acaagaagat ggaggacggc    1260
   ttcctggacg tgtggaccta caacgccgag ctgctggtgc tgatggagaa cgagcgcacc    1320
   ctggacttcc acgacagcaa cgtgaagaac ctgtacgaca aggtccgcct gcagctgcgc    1380
   gacaacgcca aggagctggg caacggctgc ttcgagttct accacaagtg cgacaacgag    1440
   tgcatggaga gcgtgcgcaa cggcacctac gactaccccc agtacagcga ggaggcccgc    1500
   ctgaagcgcg aggagatcag cggcgtgaag ctggagagca tcggcattta ccagaaggat    1560
   cca                                                                  1563
<210> 2
   <211> 21
   <212> RNA
   <213> artificial
<220>
   <221> modified_base
   <222> (20)..(21)
   <223> n=dT
<400> 2
   gggacauucu gcugaacaun n 21
<210> 3
   <211> 21
   <212> RNA
   <213> artificial
<220>
   <221> modified_base
   <222> (20)..(21)
   <223> n=dT
<400> 3
   auguucagca gaaugucccn n 21
<210> 4
   <211> 21
   <212> RNA
   <213> artificial
<220>
   <221> modified_base
   <222> (20)..(21)
   <223> n=dT
<400> 4
   accggaaacu cauggaauan n 21
<210> 5
   <211> 21
   <212> RNA
   <213> artificial
<220>
   <221> modified_base
   <222> (20)..(21)
   <223> n=dT
<400> 5
   uauuccauga guuuccggun n 21
<210> 6
   <211> 21
   <212> RNA
   <213> artificial
<220>
   <221> modified_base
   <222> (20)..(21)
   <223> n=dT
<400> 6
   caguuuggca caaucaauan n 21
<210> 7
   <211> 21
   <212> RNA
   <213> artificial
<220>
   <221> modified_base
   <222> (20)..(21)
   <223> n=dT
<400> 7
   uauugauugu gccaaacugn n 21
<210> 8
   <211> 21
   <212> RNA
   <213> artificial
<220>
   <221> modified_base
   <222> (20)..(21)
   <223> n=dT
<400> 8
   gcaguagagc gaacacgaan n 21
<210> 9
   <211> 21
   <212> RNA
   <213> artificial
<220>
   <221> modified_base
   <222> (20)..(21)
   <223> n=dT
<400> 9
   uucguguucg cucuacugcn n 21
<210> 10
   <211> 21
   <212> RNA
   <213> artificial
<220>
   <221> modified_base
   <222> (20)..(21)
   <223> n=dT
<400> 10
   ccaucaaggg ccaguucaan n 21
<210> 11
   <211> 21
   <212> RNA
   <213> artificial
<220>
   <221> modified_base
   <222> (20)..(21)
   <223> n=dT
<400> 11
   uugaacuggc ccuugauggn n 21
<210> 12
   <211> 22
   <212> DNA
   <213> artificial
<400> 12
   cagatggaca gctgcacaca ct 22
<210> 13
   <211> 22
   <212> DNA
   <213> artificial
<400> 13
   ggctctatcc cgtgaatcat ca 22
<210> 14
   <211> 21
   <212> DNA
   <213> artificial
<400> 14
   agtgtgacgt tgacatccgt a 21
<210> 15
   <211> 22
   <212> DNA
   <213> artificial
<400> 15